# EUROPEAN PATENT APPLICATION

(11) **EP 4 715 057 A1**
(43) Date of publication of application: **25.03.2026**
(21) Application number: 24806163.2
(22) Date of filing: 12.03.2024
(51) Int. Cl.: C12N 15/861, C12N 7/00

(54) **ADENO-ASSOCIATED VIRUS CAPSID PROTEIN, ADENO-ASSOCIATED VIRUS COMPRISING SAME AND USE**

(30) Priority: 15.05.2023 CN 202310560310
(71) Applicant: Hangzhou Xinchang Gene Therapeutics Inc., Hangzhou, Zhejiang 310018 (CN)
(72) Inventor: WU, Guofeng, Hangzhou, Zhejiang 310018 (CN)
(74) Representative: Weickmann & Weickmann PartmbB
(86) International application number: PCT/CN2024/081199
(87) International publication number: WO 2024/234803

(57) **Abstract**

Provided are an adeno-associated virus capsid protein, an adeno-associated virus comprising same and the use. Specifically, provided is an adeno-associated virus capsid protein comprising a targeting peptide, wherein the targeting peptide comprises one or more of the following sequences: an amino acid sequence as shown in SEQ ID NO: 23; an amino acid sequence having at least 80% identity to the amino acid sequence as shown in SEQ ID NO: 23, etc. The adeno-associated virus capsid protein and the adeno-associated virus comprising same have good producibility, have improved cell infection efficiency both *in vivo* and *in vitro* compared with a wild-type adeno-associated virus, have good targeting of brain tissue, and can be used as delivery carriers for treating brain tumors and other brain-associated diseases.

## Description

### PRIORITY AND RELATED APPLICATIONS

The present disclosure claims the benefit of a priority of Chinese Patent Application No. 202310560310.8, filed on May 15, 2023, entitled "ADENO-ASSOCIATED VIRUS CAPSID PROTEIN, ADENO-ASSOCIATED VIRUS COMPRISING SAME AND USE", the entire contents of which including the appendices are incorporated herein by reference.

### TECHNICAL FIELD

The present disclosure pertains to the technical field of viral vectors, specifically relates to an adeno-associated virus capsid protein, an adeno-associated virus comprising the same, and use thereof.

### BACKGROUND

Malignant brain tumor is often accompanied by very poor prognosis, and drugs for clinical treatment are severely limited. Gene therapy holds great promise for treatment of malignant brain tumors ¹. Adeno-associated viruses (AAV) are widely used for *in vivo* gene therapy, but none of them has yet been approved for clinical treatment of tumors ^{2, 3}. The tumor infection efficiency and targeting ability of AAVs are major bottlenecks in their application.

The functions of AAV are largely hinged on its capsid. The capsid of AAV determines its tissue targeting ability, transduction efficiency, *in vivo* immunoreactivity, etc. Capsid modification is currently a crucial means to improve the functions of AAV ⁴.

Available routes of administration for treatment of brain tumors include local intratumoral injection, intravascular administration, and intracranial administration into cerebrospinal fluid. The AAV serotype that is commonly used in research of brain tumors at present is wild-type AAV2. The drawbacks of AAV2 include poor infection efficiency in *in vivo* brain tumor tissue and inability to cross the blood-brain barrier (BBB), thereby precluding intravascular administration for targeting brain tumors, and causing poor infection efficiency and targeting ability in brain tissue after administration into cerebrospinal fluid ^{3, 5}. Other AAV serotypes available for treatment of brain tumors include AAV6 and AAV9, but both of them have the similar drawbacks of AAV2 ⁶.

Inserting a functionalized peptide into the AAV capsid is an important means of current AAV capsid modifications ⁴. Since a majority of tumor cells highly express integrin and the Arg-Gly-Asp domain (hereinafter referred to as RGD domain, RGD structural domain or RGD short peptide) is the key site in many ligand proteins capable of binding to integrin, there have been studies on insertion of an RGD domain-containing short peptide into a specific site in the AAV capsid to enhance the affinity of AAV for tumor cells. For example, the RGD short peptide (QAGTFALRGDNPQG) is inserted at site 587 of AAV2 ⁷, or the RGD short peptide (CDCRGDCFC) is inserted at site 588 of AAV2 ⁸.

While AAVs containing an RGD short peptide may theoretically exhibit an enhanced infection efficiency in tumor cells, this depends on a variety of factors in practice, such as the specific sequence of the inserted RGD short peptide, the specific serotype of AAV, the insertion site in the capsid, and the protein conformation after insertion. The practicability of such RGD-modified AAVs, if used for developing antitumor drugs, depends further on the tumor cell type, the microenvironment where the tumor cells exist, and the specific mode of administration. For example, when the RGD short peptide CDCRGDCFC is inserted at site 588 in the AAV2 capsid, the affinity of new AAV for the tumor cell will be enhanced, but when the same short peptide is inserted at site 453 in the AAV2 capsid, the new AAV completely loses its infectivity to cells ⁹.

### SUMMARY

### Technical Problem

To modify and screen out novel AAVs suitable for treatment of brain tumors, the present disclosure selects the RGD short peptide GRGDSP derived from human fibronectin, and inserts it at sites G453 and R588 in AAV2, at sites G454 and S588 in AAV6, and at sites G455 and G588 in AAV9 by the genetic engineering technology. The present disclosure has made comprehensive analysis and evaluations of the virus producibility, the *in vivo* and *in vitro* cell infection efficiency, and the targeting ability to brain tissues on the six newly generated AAV variants, and finally found a plurality of novel AAV capsids available for future development of gene-based drugs.

### Solution to Problem

According to the first aspect of the present disclosure, there is provided an adeno-associated virus capsid protein, comprising a targeting peptide, wherein the targeting peptide comprises one or more of the following sequences:
(i) an amino acid sequence as set forth in SEQ ID NO: 23;
(ii) an amino acid sequence having at least 80%, 82%, 85%, 87%, 90%, 92%, 95%, 96%, 97%, 98% or 99% identity to the amino acid sequence as set forth in SEQ ID NO: 23, and having or partially having a function of the amino acid sequence as set forth in SEQ ID NO: 23;
(iii) an amino acid sequence obtained by adding, substituting, deleting or inserting one or more amino acid residues in the amino acid sequence as set forth in SEQ ID NO: 23, and having or partially having a function of the amino acid sequence as set forth in SEQ ID NO: 23; or
(iv) an amino acid sequence encoded by a nucleotide sequence that hybridizes with a nucleotide sequence encoding the amino acid sequence as set forth in SEQ ID NO: 23 under stringency conditions, and the amino acid sequence having or partially having a function of the amino acid sequence as set forth in SEQ ID NO: 23, wherein the stringency conditions are moderate stringency conditions, moderate-high stringency conditions, high stringency conditions, or very high stringency conditions.

In some embodiments, the adeno-associated virus is selected from one or more of AAV1, AAV2, AAV3, AAV4, AAV5, AAV6, AAV7, AAV8, AAV9, rh.10, rh.39, rh.43, and rh.74.

In some preferred embodiments, the adeno-associated virus is selected from one or more of AAV2, AAV6, and AAV9.

In some embodiments, AAV2 comprises AAV2 VP1, AAV2 VP2 and/or AAV2 VP3; and/or AAV6 comprises AAV6 VP1, AAV6 VP2 and/or AAV6 VP3; and/or AAV9 comprises AAV9 VP1, AAV9 VP2 and/or AAV9 VP3.

In some specific embodiments, the targeting peptide is inserted at a position corresponding to amino acid 453 and/or a position corresponding to amino acid 588 in AAV2 VP1, AAV2 VP2 and/or AAV2 VP3; and/or the targeting peptide is inserted at a position corresponding to amino acid 454 and/or a position corresponding to amino acid 588 in AAV6 VP1, AAV6 VP2 and/or AAV6 VP3; and/or the targeting peptide is inserted at a position corresponding to amino acid 455 and/or a position corresponding to amino acid 588 in AAV9 VP1, AAV9 VP2 and/or AAV9 VP3.

In some specific embodiments, the targeting peptide is inserted at a position corresponding to amino acid 453 in AAV2 VP1, AAV2 VP2 and/or AAV2 VP3; and/or the targeting peptide is inserted at a position corresponding to amino acid 454 in AAV6 VP1, AAV6 VP2 and/or AAV6 VP3; and/or the targeting peptide is inserted at a position corresponding to amino acid 588 in AAV9 VP1, AAV9 VP2 and/or AAV9 VP3.

According to the second aspect of the present disclosure, there is provided a polynucleotide encoding the adeno-associated virus capsid protein according to the first aspect of the present disclosure.

According to the third aspect of the present disclosure, there is provided an adeno-associated virus, comprising the adeno-associated virus capsid protein according to the first aspect of the present disclosure.

In some embodiments, the adeno-associated virus further comprises a transgene.

In some optional embodiments, the transgene is a therapeutic transgene, a prophylactic transgene or a diagnostic transgene.

According to the fourth aspect of the present disclosure, there is provided a transgene delivery vector, comprising the adeno-associated virus according to the third aspect of the present disclosure.

According to the fifth aspect of the present disclosure, there is provided a pharmaceutical composition, comprising:
(a) the adeno-associated virus according to the third aspect of the present disclosure, or the transgene delivery vector according to the fourth aspect of the present disclosure; and optionally
(b) a pharmaceutically acceptable carrier.

According to the sixth aspect of the present disclosure, there is provided use of the adeno-associated virus according to the third aspect of the present disclosure, the transgene delivery vector according to the fourth aspect of the present disclosure, or the pharmaceutical composition according to the fifth aspect of the present disclosure in preparation of a reagent for delivering a transgene to a cell.

In some embodiments, the cell is derived from a subject.

In some preferred embodiments, the subject is a mammalian subject.

In some more preferred embodiments, the subject is a human.

In some embodiments, the cell is derived from a brain tissue.

In some embodiments, the subject suffers from a brain disease.

In some embodiments, the adeno-associated virus, the transgene delivery vector, or the pharmaceutical composition is administered to a subject via one or more of intravenous, intraarterial, subcutaneous, intraperitoneal, intramuscular, intracerebrospinal fluid, intraparenchymal, or intratumoral administration.

In some preferred embodiments, the adeno-associated virus, the transgene delivery vector, or the pharmaceutical composition is administered to a subject via one or more of intravenous, arterial, intracerebrospinal fluid, intraparenchymal or intratumoral administration.

According to the seventh aspect of the present disclosure, there is provided use of the adeno-associated virus according to the third aspect of the present disclosure, the transgene delivery vector according to the fourth aspect of the present disclosure, or the pharmaceutical composition according to the fifth aspect of the present disclosure in preparation of a medicament for treating a disease.

In some preferred embodiments, the disease comprises a brain disease.

### Advantageous Effects of the Invention

The adeno-associated virus capsid protein and the adeno-associated virus comprising the same as provided herein have good producibility, exhibit an improved *in vivo* and *in vitro* cell infection efficiency as compared to the wild-type adeno-associated virus, have a good targeting ability to the brain tissue, and are useful as a delivery vector for treatment of brain tumors and other brain diseases.

### BRIEF DESCRIPTION OF THE DRAWINGS

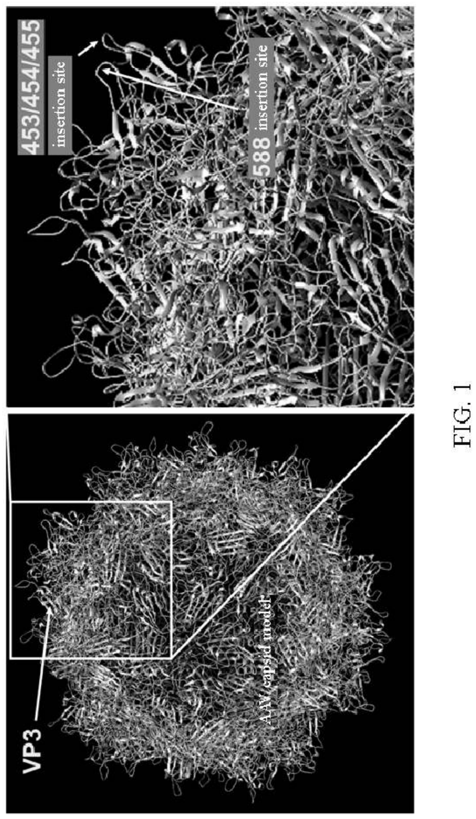

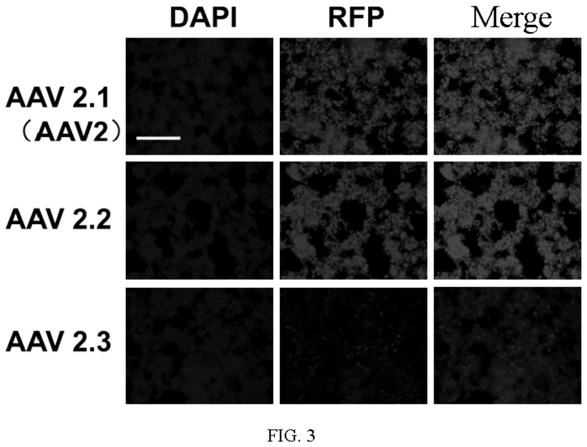

FIG. 1: AAV capsid model and modification sites.
   The left panel shows a three-dimensional model of an AAV capsid and one of the capsid protein monomers (VP3). The right panel is a partial enlarged view of the left panel showing the corresponding positions of the modification sites in the VP3 monomer. One AAV particle is composed of 60 capsid protein monomers. The approach of the present disclosure causes each of the monomers to be modified.
FIG. 2: maps of plasmids RC2 (A), RC6 (B), and RC9 (C) required for packaging wild-type AAV2, AAV6, and AAV9.
FIG. 3: comparison between infection efficiencies of AAV2 and its modified AAVs in the *in vitro* HEK293 cells.
   The leftmost panels show images of nuclei in the HEK293 cells stained with DAPI. The middle panels show images of the red fluorescent proteins RFPs in the same field of view. The rightmost panels each show the merged image of the corresponding two images on the left thereof. The higher the red fluorescence intensity, the better the expression efficiency of the corresponding AAV.

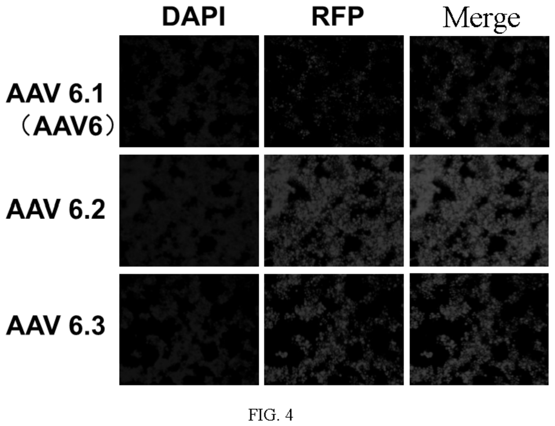

FIG. 4: comparison between infection efficiencies of AAV6 and its modified AAVs in the *in vitro* HEK293 cells.
   The leftmost panels show images of nuclei in the HEK293 cells stained with DAPI. The middle panels show images of the red fluorescent proteins RFPs in the same field of view. The rightmost panels each show the merged image of the corresponding two images on the left thereof. The higher the red fluorescence intensity, the better the expression efficiency of the corresponding AAV.

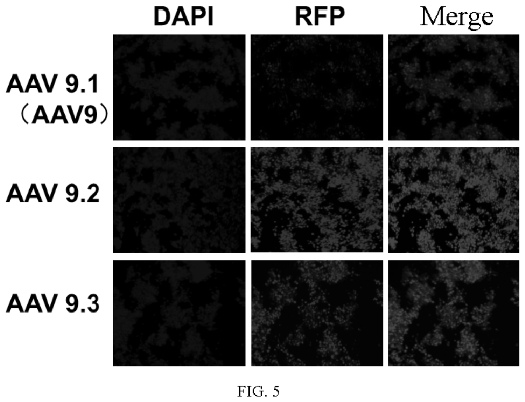

FIG. 5: comparison between infection efficiencies of AAV9 and its modified AAVs in the *in vitro* HEK293 cells.
   The leftmost panels show images of nuclei in the HEK293 cells stained with DAPI. The middle panels show images of the red fluorescent proteins RFPs in the same field of view. The rightmost panels each show the merged image of the corresponding two images on the left thereof. The higher the red fluorescence intensity, the better the expression efficiency of the corresponding AAV.

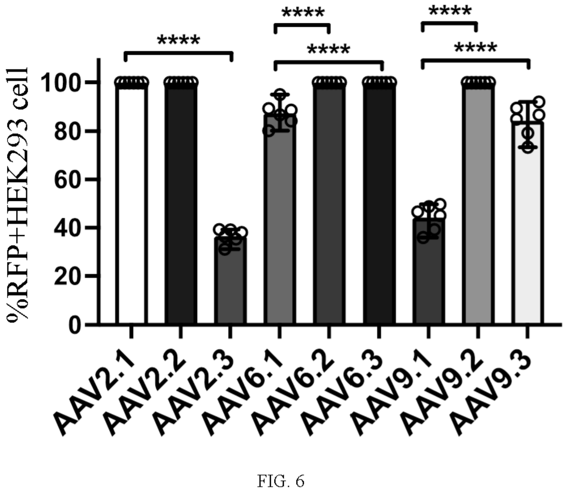

FIG. 6: quantitative analysis of the infection efficiencies of AAVs in the *in vitro* HEK293 cells.
   The infection efficiency of AAV is defined as the percentage of RFP-positive cells to all DAPI-positive HEK293 cells. The representative images are as shown in FIG. 3 to FIG. 5. AAV2.1, AAV6.1, and AAV9.1 are wild-type AAV2, AAV6, and AAV9, respectively. AAV2.2 and AAV2.3 are modified AAV2. AAV6.2 and AAV6.3 are modified AAV6. AAV9.2 and AAV9.3 are modified AAV9.

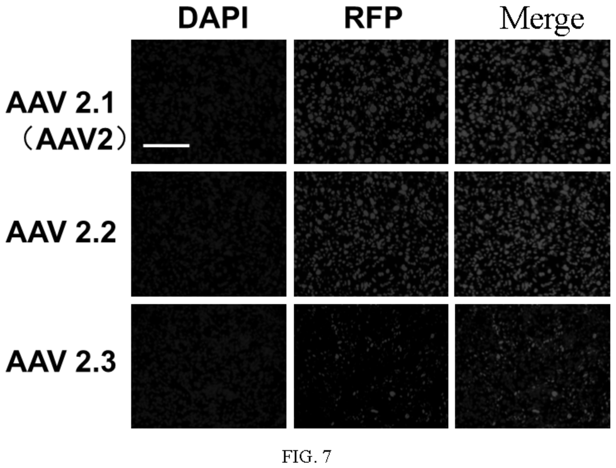

FIG. 7: comparison between infection efficiencies of AAV2 and its modified AAVs in the *in vitro* U251 human glioma cells.
   The leftmost panels show images of nuclei in the U251 cells stained with DAPI. The middle panels show images of the red fluorescent proteins RFPs in the same field of view. The rightmost panels each show the merged image of the corresponding two images on the left thereof. The higher the red fluorescence intensity, the better the expression efficiency of the corresponding AAV.

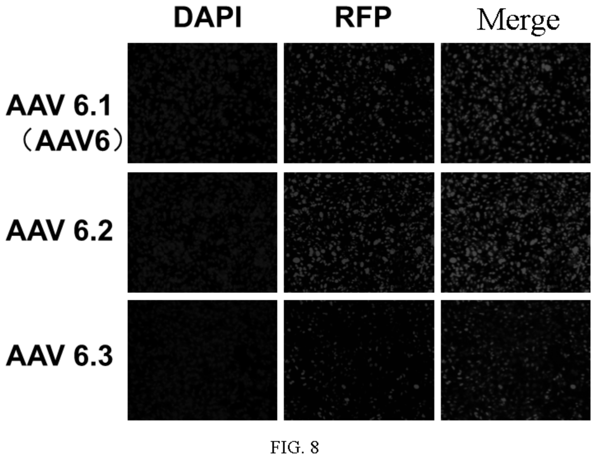

FIG. 8: comparison between infection efficiencies of AAV6 and its modified AAVs in the *in vitro* U251 human glioma cells.
   The leftmost panels show images of nuclei in the U251 cells stained with DAPI. The middle panels show images of the red fluorescent proteins RFPs in the same field of view. The rightmost panels each show the merged image of the corresponding two images on the left thereof. The higher the red fluorescence intensity, the better the expression efficiency of the corresponding AAV.

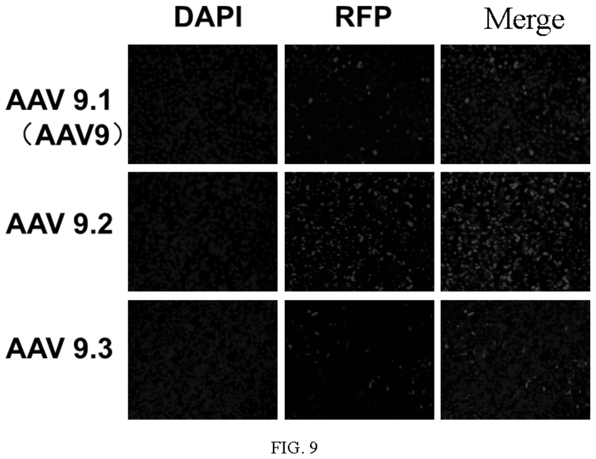

FIG. 9: comparison between infection efficiencies AAV9 and its modified AAVs in the *in vitro* U251 human glioma cells.
   The leftmost panels show images of nuclei in the U251 cells stained with DAPI. The middle panels show images of the red fluorescent proteins RFPs in the same field of view. The rightmost panels each show the merged image of the corresponding two images on the left thereof. The higher the red fluorescence intensity, the better the expression efficiency of the corresponding AAV.

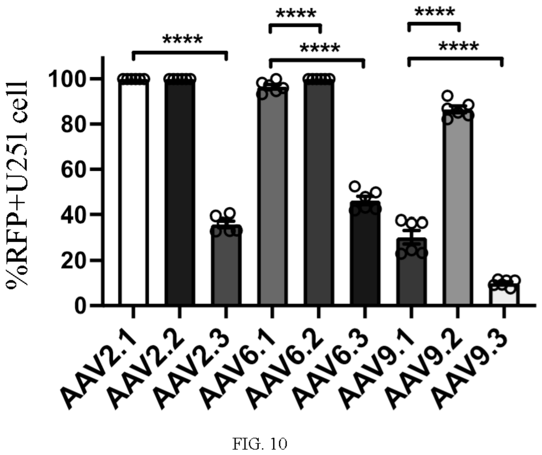

FIG. 10: quantitative analysis of the infection efficiencies of AAVs in the *in vitro* U251 human glioma cells.
   The infection efficiency of AAV is defined as the percentage of RFP-positive cells to all DAPI-positive U251 cells. The representative images are as shown in FIG. 7 to FIG. 9. AAV2.1, AAV6.1, and AAV9.1 are wild-type AAV2, AAV6, and AAV9, respectively. AAV2.2 and AAV2.3 are modified AAV2. AAV6.2 and AAV6.3 are modified AAV6. AAV9.2 and AAV9.3 are modified AAV9.

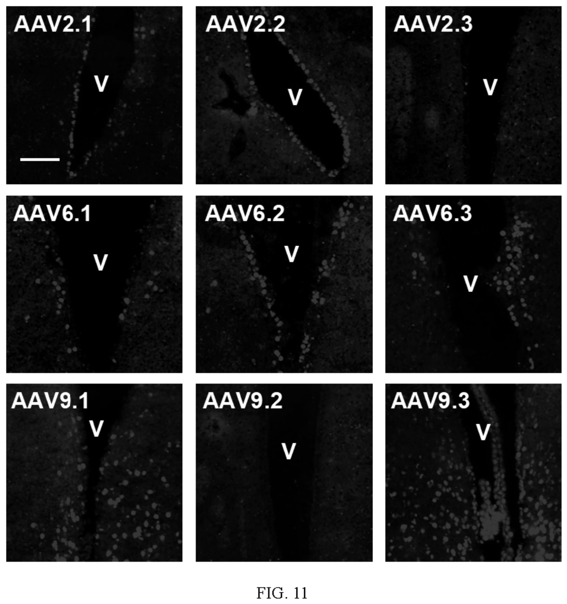

FIG. 11: distribution diagram for AAV-mediated expression of red fluorescent proteins near the lateral ventricles of mice.
   5 µl of AAV-CAG-RFP viruses are slowly injected via a microsyringe into the unilateral lateral ventricles of adult mice (3 mice per AAV, 8.3 × 10⁹ vg per mouse). After localized injection, the mice are fed for 4 weeks. The brain tissue is cut into coronal sections and then scanned with red fluorescence. The sign "V" in the images marks the location of the lateral ventricle. AAV2.1, AAV6.1, and AAV9.1 are wild-type AAV2, AAV6, and AAV9, respectively. AAV2.2 and AAV2.3 are modified AAV2. AAV6.2 and AAV6.3 are modified AAV6. AAV9.2 and AAV9.3 are modified AAV9.

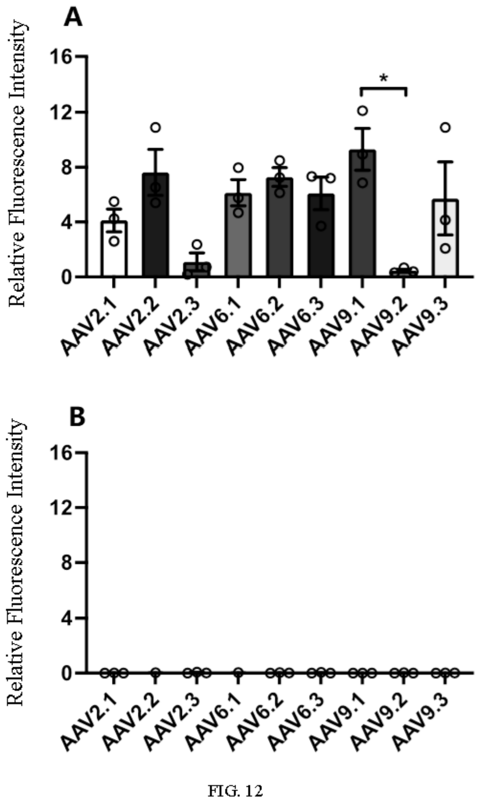

FIG. 12: quantification of AAV-mediated intensity of red fluorescent proteins near the lateral ventricle (A) and in hippocampus (B) of mice.
   The relative fluorescence intensity is measured by the ImageJ software. No fluorescent protein signals are found in other brain areas than the vicinity of lateral ventricle. AAV2.1, AAV6.1, and AAV9.1 are wild-type AAV2, AAV6, and AAV9, respectively. AAV2.2 and AAV2.3 are modified AAV2. AAV6.2 and AAV6.3 are modified AAV6. AAV9.2 and AAV9.3 are modified AAV9.

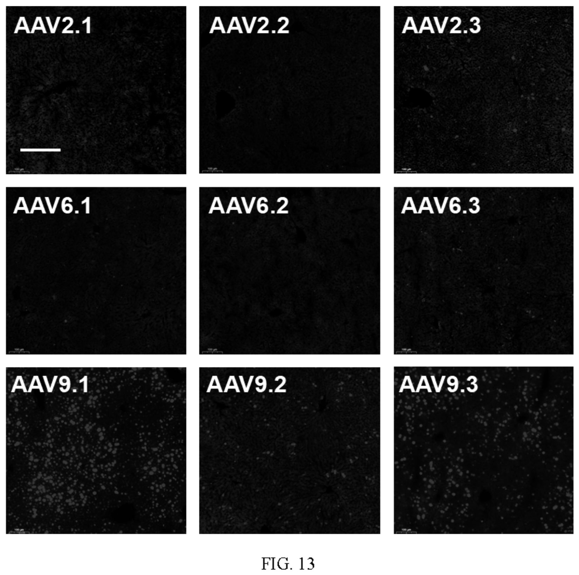

FIG. 13: distribution diagram for AAV-mediated expression of red fluorescent proteins in mouse liver.
   5 µl of AAV-CAG-RFP viruses are slowly injected via a microsyringe into the unilateral lateral ventricles of adult mice (3 mice per AAV, 8.3 × 10⁹ vg per mouse). After localized injection, the mice are fed for 4 weeks. The liver tissue is cut into coronal sections and then scanned with red fluorescence. The stronger the red fluorescence signals, the more "leaked" AAVs are expressed. AAV2.1, AAV6.1, and AAV9.1 are wild-type AAV2, AAV6, and AAV9, respectively.

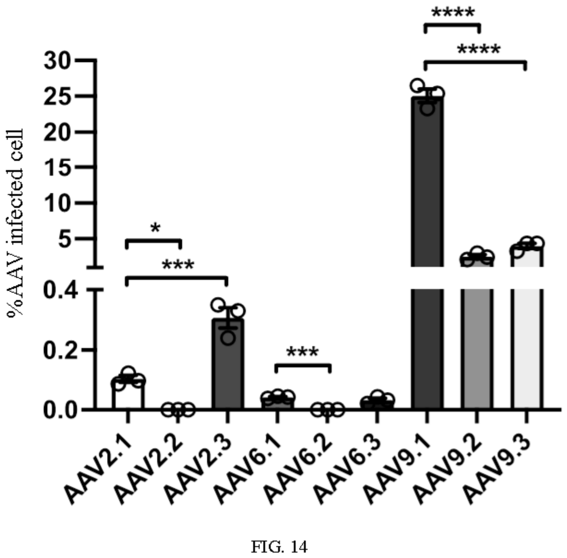

FIG. 14: AAV expression intensity in mouse liver.
   The AAV expression intensity is defined as the percentage of RFP-positive cells to all DAPI-positive hepatocytes. AAV2.1, AAV6.1, and AAV9.1 are wild-type AAV2, AAV6, and AAV9, respectively. AAV2.2 and AAV2.3 are modified AAV2. AAV6.2 and AAV6.3 are modified AAV6. AAV9.2 and AAV9.3 are modified AAV9.

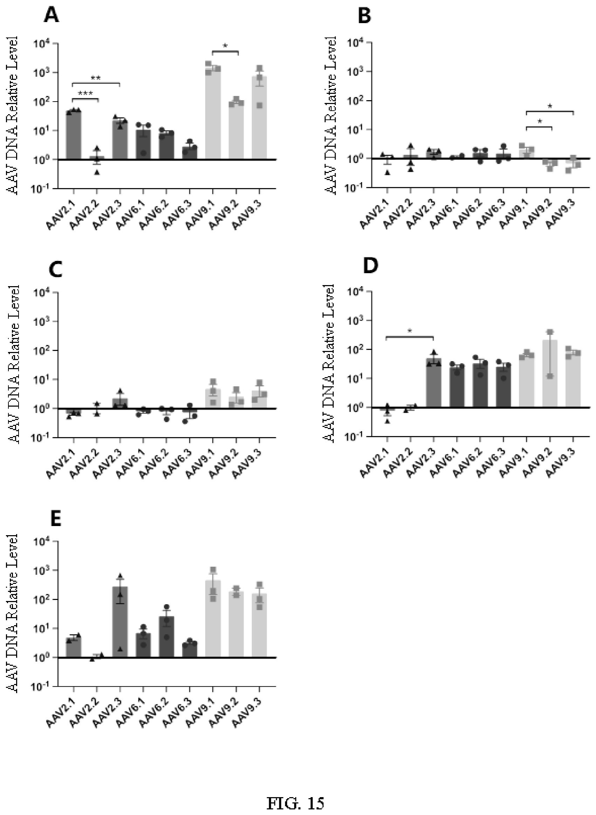

FIG. 15: DNA quantification of AAVs "leaked" into the peripheral tissues of mice; A: liver; B: skeletal muscle; C: heart; D: kidney; E: spleen.

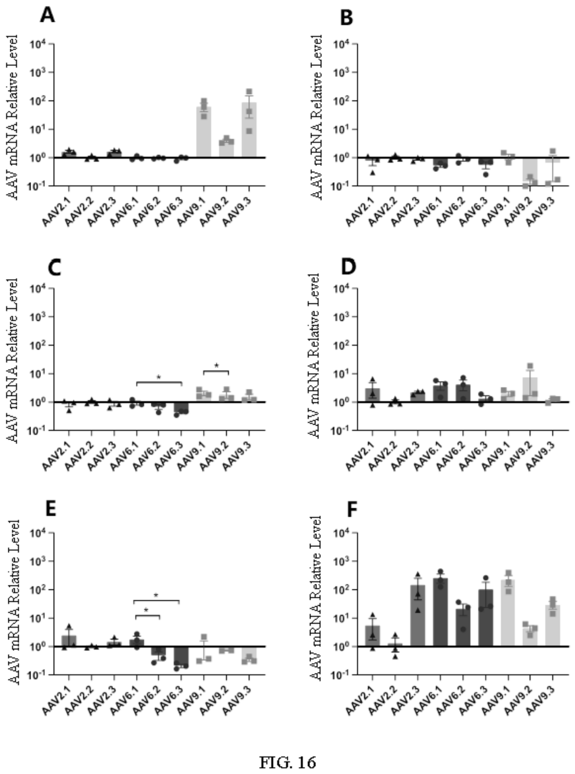

FIG. 16: RNA quantification of AAVs "leaked" into the peripheral tissues of mice; A: liver; B: skeletal muscle; C: heart; D: kidney; E: spleen; F: dorsal ganglion.

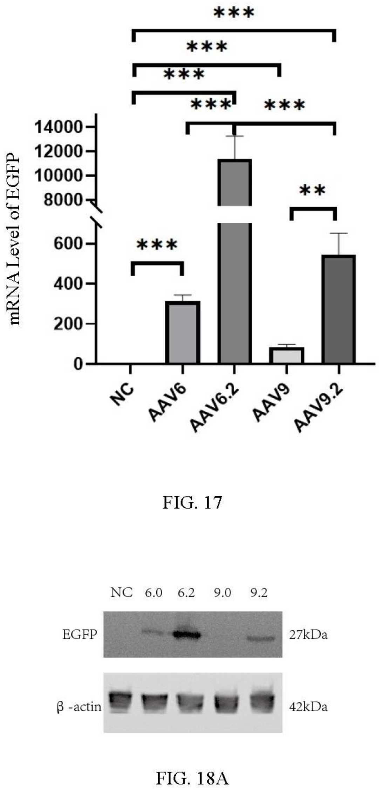

FIG. 17: qPCR detection of the mRNA level of EGFP after AAV infection in HEK293 cells for 72 h, where NC group (mean = 1), AAV6 group (mean = 311.7), AAV6.2 group (mean = 11390), AAV9 group (mean = 84.57), AAV9.2 group (mean = 545.2).

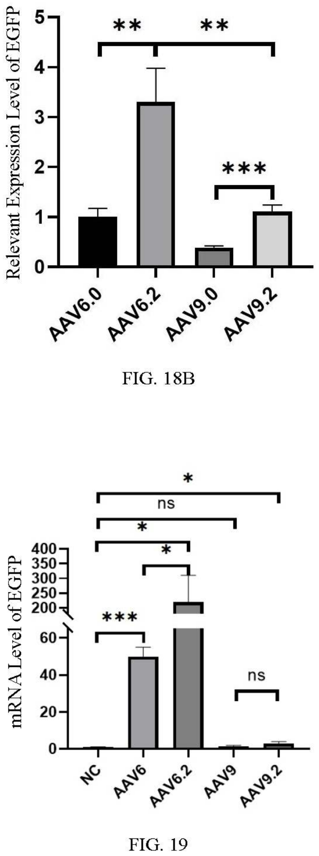

FIG. 18A and FIG. 18B: WB detection of the protein level of EGFP after AAV infection in HEK293 cells for 72 h, where AAV6 group (mean = 1), AAV6.2 group (mean = 3.3), AAV9 group (mean = 0.4), AAV9.2 group (mean = 1.1).
FIG. 19: qPCR detection of the mRNA level of EGFP after AAV infection in BT474 cells for 72 h, where NC group (mean = 1), AAV6 group (mean = 50.02), AAV6.2 group (mean = 219.4), AAV9 group (mean = 1.423), AAV9.2 group (mean = 2.878).

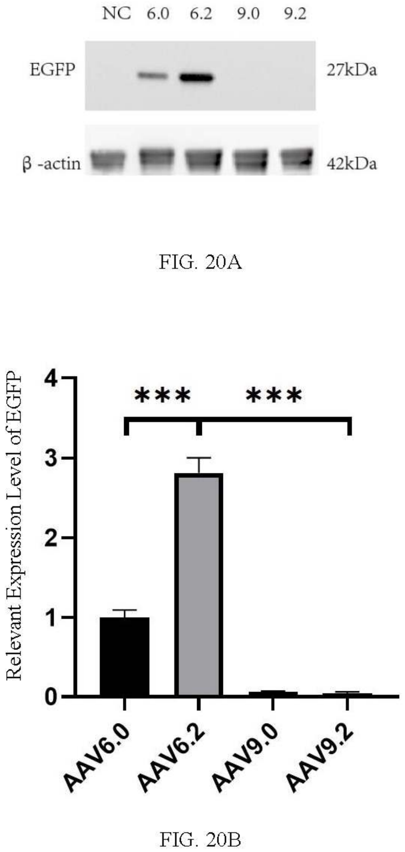

FIG. 20A and FIG. 20B: WB detection of the protein level of EGFP after AAV infection in BT474 cells for 72 h, where AAV6 group (mean = 1), AAV6.2 group (mean = 2.8), AAV9 group (mean = 0.06), AAV9.2 group (mean = 0.05).

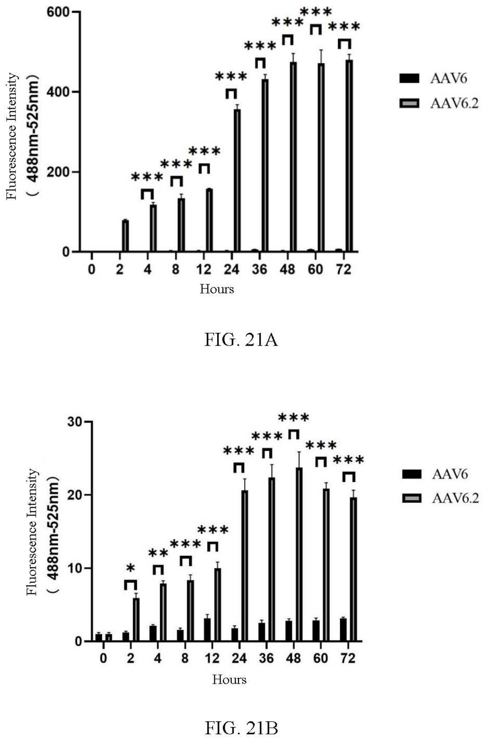

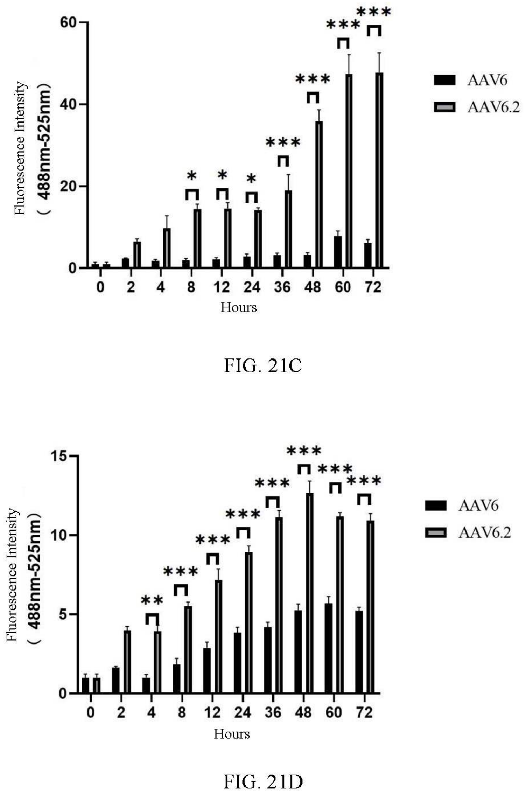

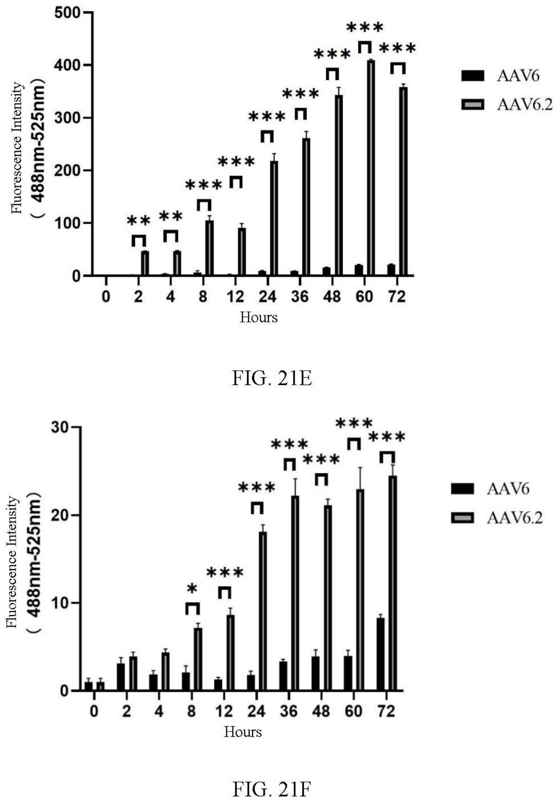

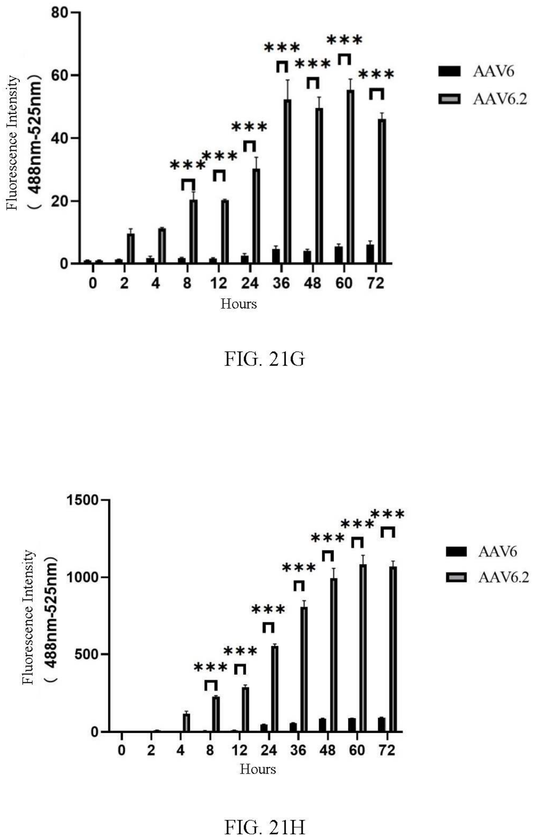

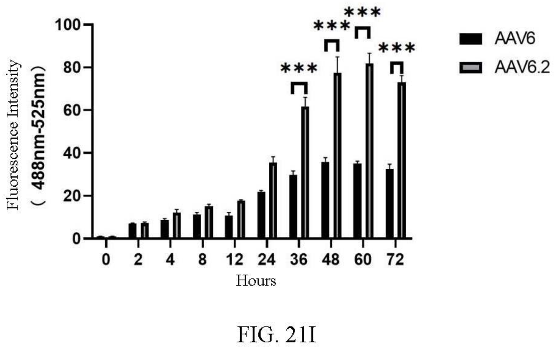

FIG. 21A to FIG. 21I: schematic diagrams for fluorescence intensity of EGFP detected by a microplate reader after AAV6 and AAV6.2 infect cell lines of different sources. Among them, FIG. 21A shows NCI-H196 (human small cell lung cancer cells); FIG. 21B shows SK-N-SH (human neuroblastoma cells); FIG. 21C shows T98G (human glioma cells); FIG. 21D shows A172 (human glioblastoma cells); FIG. 21E shows U-87 MG (human brain astroblastoma cells); FIG. 21F shows SK-BR-3 (human breast cancer cells); FIG. 21G shows PC-3 (human prostate cancer cells); FIG. 21H shows A-375 (human malignant melanoma cells); and FIG. 21I shows HCT116 (human colon cancer cells).

### DETAILED DESCRIPTION

To render the present disclosure more understandable, some technical and scientific terms are specifically defined below. Unless otherwise explicitly defined herein, all of the other technical and scientific terms used herein have the same meanings as typically understood by one of ordinary skill in the art to which the present disclosure belongs.

In the present specification, the numerical range represented by "numerical value A to numerical value B" refers to the range including the endpoint values A and B.

In the present specification, the term "basically" or "substantially" means that the standard deviation from the theoretical model or theoretical data is within a range of 5%, preferably 3%, more preferably 1%.

In the present specification, the term "may" involves both the meaning of doing something and the meaning of not doing something.

In the present specification, the term "optional" or "optionally" means that the event or case described below may or may not occur, and this description includes the case where this event occurs and the case where this event does not occur.

Phrases such as "some specific/preferred embodiments", "other specific/preferred embodiments", and "embodiments" referred to in the present specification mean that particular elements (for example, features, structures, properties and/or characteristics) described in relation to this embodiment are included in at least one of the embodiments described herein, and may or may not exist in other embodiments. Additionally, it should be appreciated that the elements may be combined in any suitable manner into various embodiments.

According to the present disclosure, the terms "polypeptide", "protein", and "peptide" are used interchangeably herein to refer to a polymeric form of amino acids of any length, which may include coded and non-coded amino acids, chemically or biochemically modified or derived amino acids, and polypeptides having similar peptide backbones.

According to the present disclosure, the terms "nucleic acid molecule", "polynucleotide", "polynucleic acid", and "nucleic acid" are used interchangeably to refer to a polymeric form of nucleotides of any length, regardless of deoxyribonucleotides or ribonucleotides, or analogs thereof. Polynucleotide may have any three-dimensional structure and may implement any known or unknown function. Non-limiting examples of the polynucleotide include genes, gene fragments, exons, introns, messenger RNA (mRNA), transfer RNA, ribosomal RNA, ribozymes, cDNA, recombinant polynucleotides, branched polynucleotides, plasmids, vectors, isolated DNA of any sequence, control regions, isolated RNA of any sequence, and nucleic acid probes and primers. The nucleic acid molecule may be linear or circular.

According to the present disclosure, the three-letter and single-letter codes for amino acids used herein are as described in J. biol. chem, 243, p3558 (1968).

According to the present disclosure, the "addition" of an amino acid means that an amino acid is added at the C-terminus or N-terminus of an amino acid sequence. According to the present disclosure, the "deletion" of an amino acid means that 1, 2 or 3 or more amino acids can be deleted from an amino acid sequence. According to the present disclosure, the "insertion" of an amino acid means that an amino acid residue is inserted at an appropriate position in an amino acid sequence, and the inserted amino acid residues may also be all or partially adjacent to one another, or the inserted amino acids are not adjacent to one another. According to the present disclosure, the "substitution" of an amino acid means that an amino acid residue at a position in an amino acid sequence is substituted with an additional amino acid residue, where the "substitution" may be a conservative substitution of an amino acid.

According to the present disclosure, the term "conservative modification", "conservative substitution" or "conservative replacement" refers to replacement of an amino acid in a protein with an additional amino acid having similar characteristics (*e*.*g*. charge, size of side chain, hydrophobicity/hydrophilicity, backbone conformation, and rigidity), such that the protein can be changed frequently without altering the biological activity of the protein. A person skilled in the art knows that in general, replacement of a single amino acid in the non-essential region of a polypeptide does not substantially alter the biological activity (see, for example, Watson et al. (1987) Molecular Biology of the Gene, The Benjamin/Cummings Pub. Co., page 224, (4th ed.)). Furthermore, replacement with structurally or functionally similar amino acids is unlikely to disrupt the biological activity. Exemplary conservative substitutions are set forth in the following "Exemplary Amino Acid Conservative Substitutions":

### Exemplary Amino Acid Conservative Substitutions

| Original Residue | Conservative Substitution |
|---|---|
| Ala (A) | Gly; Ser |
| Arg (R) | Lys; His |
| Asn (N) | Gln; His; Asp |
| Asp (D) | Glu; Asn |
| Cys (C) | Ser; Ala; Val |
| Gln (Q) | Asn; Glu |
| Glu (E) | Asp; Gln |
| Gly (G) | Ala |
| His (H) | Asn; Gln |
| Ile (I) | Leu; Val |
| Leu (L) | Ile; Val |
| Lys (K) | Arg; His |
| Met (M) | Leu; Ile; Tyr |
| Phe (F) | Tyr; Met; Leu |
| Pro (P) | Ala |
| Ser (S) | Thr |
| Thr (T) | Ser |
| Trp (W) | Tyr; Phe |
| Tyr (Y) | Trp; Phe |
| Val (V) | Ile; Leu |

According to the present disclosure, "moderate to very high stringency conditions" include "moderate stringency conditions", "moderate-high stringency conditions", "high stringency conditions" or "very high stringency conditions", which describe the conditions for hybridization and washing of nucleic acids. Guidance for conducting hybridization reactions can be found in Current Protocols in Molecular Biology, John Wiley & Sons, N.Y. (1989), 6.3.1-6.3.6, which is incorporated herein by reference. This literature describes both aqueous and non-aqueous processes and either can be used. For example, specific hybridization conditions are as follows: (1) the low stringency hybridization conditions refer to hybridizing in 6× sodium chloride/sodium citrate (SSC) at about 45°C, and then washing twice in 0.2× SSC and 0.1% SDS at 50°C or higher (the washing temperature can be raised to 55°C in the case of the low stringency conditions); (2) the moderate stringency hybridization conditions refer to hybridizing in 6× SSC at about 45°C, and then washing one or more times in 0.2× SSC and 0.1% SDS at 60°C; (3) the high stringency hybridization conditions refer to hybridizing in 6× SSC at about 45°C, and then washing one or more times in 0.2× SSC and 0.1% SDS at 65°C, which is preferred; and (4) the very high stringency hybridization conditions refer to hybridizing in 0.5 M sodium phosphate and 7% SDS at 65°C, and then washing one or more times in 0.2× SSC and 1% SDS at 65°C.

The term "identity" refers to the sequence similarity between two polynucleotide sequences or between two polypeptides. When the positions in two compared sequences are all occupied by the same base or amino acid monomer subunit, for example, if each of the positions in two DNA molecules is occupied by adenine, the molecules are homologous at this position. The percent identity between two sequences is a function of the number of matching or homologous positions shared by the two sequences / the number of positions to be compared × 100%. For example, in the case of optimal alignment of sequences, if six out of ten positions in two sequences are matched or homologous, the two sequences are 60% homologous. In general, comparison is made between two sequences when aligned to obtain the maximum percent identity.

When applied to an animal, human, test subject, cell, tissue, organ or biological fluid, the terms "administration", "giving", and "treating" refer to contact of an exogenous drug, therapeutic agent, diagnostic agent, or composition with the animal, human, subject, cell, tissue, organ or biological fluid. The terms "administration", "giving", and "treating" may refer to, e.g., therapeutic, pharmacokinetics, diagnostic, research, and experimental methods. Treating of cells includes contact of a reagent with the cells and contact of a reagent with a fluid, where the fluid is in contact with the cells. The terms "administration", "giving", and "treating" also means *in vitro* and *ex vivo* treatment of, for example, a cell, by a reagent, a diagnostic agent, a binding composition or an additional cell. When applied to humans, veterinary, or research subjects, "treating" refers to therapeutic, prophylactic or precautionary measures, research and diagnostic applications.

The term "treatment" means administering a therapeutic agent for internal or external use, such as a therapeutic agent comprising any of the antibodies described herein, to a subject having one or more symptoms of a disease, on which the therapeutic agent is known to have a therapeutic effect. Generally, the therapeutic agent is administered in an amount effective to alleviate one or more symptoms of the disease in the subject or population being treated by either inducing regression of such symptoms or suppressing progression of such symptoms to any clinically measurable degree. The amount of a therapeutic agent effective to alleviate any specific disease symptom (also referred to as a "therapeutically effective amount") may vary depending upon multiple factors such as the disease state, age, and body weight of the patient, and the ability of the drug to produce the desired therapeutic effect in the patient. Any clinical test method typically used by a physician or other health care professional to assess the severity or progression of a disease symptom may be adopted to evaluate whether this symptom has been alleviated.

In the present specification, the term "prevention" refers to prophylactic treatment on a subject who does not have a disease now or in the past but is at risk of developing a disease or who had a disease in the past and does not have a disease now but is at risk of recurrence of the disease. In some embodiments, the subject runs a higher risk of developing a disease or a higher risk of recurrence of the disease as compared to the members at the average healthy level in the subject population.

An "effective amount" includes an amount sufficient to ameliorate or prevent the symptoms of a medical condition or the condition. An effective amount also means an amount sufficient to allow for or facilitate diagnosis. An effective amount for a particular patient or veterinary subject may vary depending on factors such as the condition to be treated, the patient's overall health, the method, route, and dose of administration, and the severity of side effects. An effective amount may be the maximum dose or dosage regimen that avoids significant side effects or toxic effects.

In the present specification, the "therapeutically effective amount" is an amount sufficient to provide a therapeutic benefit in the course of treating a condition or sufficient to delay or minimize one or more symptoms associated with the condition. A therapeutically effective amount refers to an amount of a therapeutic agent alone or in combination with other therapies, which provides a therapeutic benefit in the course of treating a condition. The term "therapeutically effective amount" may include an amount that improves overall therapy; reduces or avoids symptoms, signs, or causes of a condition; and/or enhances the therapeutic efficacy of an additional therapeutic agent.

In the present specification, the "prophylactically effective amount" is an amount sufficient to prevent a condition or one or more symptoms associated with the condition or to prevent its recurrence. A prophylactically effective amount refers to an amount of a therapeutic agent alone or in combination with other drugs, which provides a therapeutic benefit in the course of preventing a condition. The term "prophylactically effective amount" may include an amount that improves overall prevention or enhances the prophylactic efficacy of an additional prophylactic agent.

In the present specification, the term "subject" refers to a human (*i.e*., a male or female of any age, *e.g.,* a pediatric subject (*e.g.,* an infant, child, or adolescent) or an adult subject (*e.g.,* a young, middle-aged, or elderly person)) or a non-human animal. In some embodiments, the non-human animal is a mammal (*e.g*., a primate (such as *Macaca fascicularis* or *Macaca mulatta*), a commercially relevant mammal (such as cow, pig, horse, sheep, goat, cat, or dog), or a bird. The non-human animal may be male or female at any stage of development. The non-human animal may be a transgenic animal or a genetically engineered animal.

### Detailed Description of the Invention

The present disclosure selects the RGD short peptide GRGDSP (SEQ ID NO: 23) derived from human fibronectin as a targeting peptide, inserts it into the capsid proteins of AAV2, AAV6, and AAV9 by the genetic engineering technology, and evaluates its producibility, *in vivo* and *in vitro* cell infection efficiency, and targeting ability to brain tissue.

### AAV Capsid Protein

In some embodiments of the present disclosure, there is provided an AAV capsid protein, comprising a targeting peptide, wherein the targeting peptide comprises one or more of the following sequences:
(i) an amino acid sequence as set forth in SEQ ID NO: 23;
(ii) an amino acid sequence having at least 80%, 82%, 85%, 87%, 90%, 92%, 95%, 96%, 97%, 98% or 99% identity to the amino acid sequence as set forth in SEQ ID NO: 23, and having or partially having a function of the amino acid sequence as set forth in SEQ ID NO: 23;
(iii) an amino acid sequence obtained by adding, substituting, deleting or inserting one or more amino acid residues in the amino acid sequence as set forth in SEQ ID NO: 23, and having or partially having a function of the amino acid sequence as set forth in SEQ ID NO: 23; or
(iv) an amino acid sequence encoded by a nucleotide sequence that hybridizes with a nucleotide sequence encoding the amino acid sequence as set forth in SEQ ID NO: 23 under stringency conditions, and the amino acid sequence having or partially having a function of the amino acid sequence as set forth in SEQ ID NO: 23, wherein the stringency conditions are moderate stringency conditions, moderate-high stringency conditions, high stringency conditions, or very high stringency conditions.

In the present disclosure, AAV is a tiny non-enveloped virus having a 25 nm capsid. No disease is known or has been shown to be associated with the wild type virus. AAV has a single-stranded DNA (ssDNA) genome. AAV has been shown to exhibit long-term episomal transgene expression. Vectors containing as few as 300 base pairs of AAV can be packaged and can integrate. Space for exogenous DNA is limited to about 4.7 kb. An AAV vector such as that described in Tratschin et al., Mol. Cell. Biol. 5:3251-3260 (1985) can be used to introduce DNA into cells. A variety of nucleic acids have been introduced into different cell types using AAV vectors (see for example Hermonat et al., Proc. Natl. Acad. Sci. USA 81:6466-6470 (1984); Tratschin et al., Mol. Cell. Biol. 4:2072-2081 (1985); Wondisford et al., Mol. Endocrinol. 2:32-39 (1988); Tratschin et al., J. Virol. 51:611-619 (1984); and Flotte et al., J. Biol. Chem. 268:3781-3790 (1993)). There are numerous alternative AAV variants (over 100 have been cloned), and AAV variants have been identified based on desirable characteristics.

In some embodiments, AAV is selected from one or more of AAV1, AAV2, AAV3, AAV4, AAV5, AAV6, AAV7, AAV8, AAV9, rh.10, rh.39, rh.43, and rh.74.

In some preferred embodiments, AAV is selected from one or more of AAV2, AAV6, and AAV9.

AAV capsid is an icosahedron composed of 60 VP capsid protein monomers including 5 VP1 monomers, 5 VP2 monomers, and 50 VP3 monomers. VP1, VP2, and VP3 monomers are all transcribed and translated by the *Cap* gene of AAV. VP1 is the longest and includes approximate 735 amino acids. VP2 and VP3 are "truncated forms" of VP1 and do not contain part of amino acids at the N-terminus of the VP1 protein. Conventionally, the modification sites in the capsid protein are designated based on the amino acid sequence of the VP1 protein. For instance, insertion site 453 in AAV2 means that a short peptide is inserted after the 453^{rd} amino acid of the VP1 monomer (containing 735 amino acids) in AAV2, so that all of AAV2 VP1, VP2, and VP3 contain this short peptide; insertion site 454 in AAV6 means that a short peptide is inserted after the 454^{th} amino acid of the VP1 monomer (containing 736 amino acids) in AAV6, so that all of AAV6 VP1, VP2, and VP3 contain this short peptide.

In some embodiments, AAV2 includes AAV2 VP1, AAV2 VP2 and/or AAV2 VP3. In some embodiments, AAV6 includes AAV6 VP1, AAV6 VP2 and/or AAV6 VP3. In some embodiments, AAV9 includes AAV9 VP1, AAV9 VP2 and/or AAV9 VP3.

In the present specification, the insertion sites of the targeting peptide in VP2 and VP3 are described based on their corresponding amino acid positions in VP1. For example, the amino acid sequence (SEQ ID NO: 15) of AAV2 VP2 consists of a total of 598 amino acids corresponding to amino acids 138-735 in AAV2 VP1.

Exemplarily, the amino acid sequence of AAV2 VP1 is as shown below (SEQ ID NO: 14): (A total of 735 amino acids)

The amino acid sequence of AAV2 VP2 is as shown below (SEQ ID NO: 15): 
TAPGKKRPVEHSPVEPDSSSGTGKAGQQPARKRLNFGQTGDADSVPDPQPLGQPP
AAPSGLGTNTMATGSGAPMADNNEGADGVGNSSGNWHCDSTWMGDRVITTSTRTWA
LPTYNNHLYKQISSQSGASNDNHYFGYSTPWGYFDFNRFHCHFSPRDWQRLINNNWG
FRPKRLNFKLFNIQVKEVTQNDGTTTIANNLTSTVQVFTDSEYQLPYVLGSAHQGCLPP
FPADVFMVPQYGYLTLNNGSQAVGRSSFYCLEYFPSQMLRTGNNFTFSYTFEDVPFHSS
YAHSQSLDRLMNPLIDQYLYYLSRTNTPSGTTTQSRLQFSQAGASDIRDQSRNWLPGPC
YRQQRVSKTSADNNNSEYSWTGATKYHLNGRDSLVNPGPAMASHKDDEEKFFPQSGV
LIFGKQGSEKTNVDIEKVMITDEEEIRTTNPVATEQYGSVSTNLQRGNRQAATADVNTQ
GVLPGMVWQDRDVYLQGPIWAKIPHTDGHFHPSPLMGGFGLKHPPPQILIKNTPVPAN
PSTTFSAAKFASFITQYSTGQVSVEIEWELQKENSKRWNPEIQYTSNYNKSVNVDFTVD
TNGVYSEPRPIGTRYLTRNL
 (Amino acids 138-735 in AAV2 VP1, a total of 598 amino acids)

The amino acid sequence of AAV2 VP3 is as shown below (SEQ ID NO: 16): 
MATGSGAPMADNNEGADGVGNSSGNWHCDSTWMGDRVITTSTRTWALPTYNN
HLYKQISSQSGASNDNHYFGYSTPWGYFDFNRFHCHFSPRDWQRLINNNWGFRPKRL
NFKLFNIQVKEVTQNDGTTTIANNLTSTVQVFTDSEYQLPYVLGSAHQGCLPPFPADVF
MVPQYGYLTLNNGSQAVGRSSFYCLEYFPSQMLRTGNNFTFSYTFEDVPFHSSYAHSQ
SLDRLMNPLIDQYLYYLSRTNTPSGTTTQSRLQFSQAGASDIRDQSRNWLPGPCYRQQR
VSKTSADNNNSEYSWTGATKYHLNGRDSLVNPGPAMASHKDDEEKFFPQSGVLIFGK
QGSEKTNVDIEKVMITDEEEIRTTNPVATEQYGSVSTNLQRGNRQAATADVNTQGVLP
 
GMVWQDRDVYLQGPIWAKIPHTDGHFHPSPLMGGFGLKHPPPQILIKNTPVPANPSTTF
SAAKFASFITQYSTGQVSVEIEWELQKENSKRWNPEIQYTSNYNKSVNVDFTVDTNGV
YSEPRPIGTRYLTRNL
 (Amino acids 203-735 in AAV2 VP1, a total of 533 amino acids)

The amino acid sequence of AAV6 VP1 is as shown below (SEQ ID NO: 17): 
MAADGYLPDWLEDNLSEGIREWWDLKPGAPKPKANQQKQDDGRGLVLPGYKY
LGPFNGLDKGEPVNAADAAALEHDKAYDQQLKAGDNPYLRYNHADAEFQERLQEDT
SFGGNLGRAVFQAKKRVLEPLGLVEEGAKTAPGKKRPVEQSPQEPDSSSGIGKTGQQPA
KKRLNFGQTGDSESVPDPQPLGEPPATPAAVGPTTMASGGGAPMADNNEGADGVGNA
SGNWHCDSTWLGDRVITTSTRTWALPTYNNHLYKQISSASTGASNDNHYFGYSTPWG
YFDFNRFHCHFSPRDWQRLINNNWGFRPKRLNFKLFNIQVKEVTTNDGVTTIANNLTS
TVQVFSDSEYQLPYVLGSAHQGCLPPFPADVFMIPQYGYLTLNNGSQAVGRSSFYCLE
YFPSQMLRTGNNFTFSYTFEDVPFHSSYAHSQSLDRLMNPLIDQYLYYLNRTQNQSGSA
QNKDLLFSRGSPAGMSVQPKNWLPGPCYRQQRVSKTKTDNNNSNFTWTGASKYNLN
GRESIINPGTAMASHKDDKDKFFPMSGVMIFGKESAGASNTALDNVMITDEEEIKATNP
VATERFGTVAVNLQSSSTDPATGDVHVMGALPGMVWQDRDVYLQGPIWAKIPHTDGH
FHPSPLMGGFGLKHPPPQILIKNTPVPANPPAEFSATKFASFITQYSTGQVSVEIEWELQK
ENSKRWNPEVQYTSNYAKSANVDFTVDNNGLYTEPRPIGTRYLTRPL
 (A total of 736 amino acids)

The amino acid sequence of AAV6 VP2 is as shown below (SEQ ID NO: 18): 
TAPGKKRPVEQSPQEPDSSSGIGKTGQQPAKKRLNFGQTGDSESVPDPQPLGEPPA
TPAAVGPTTMASGGGAPMADNNEGADGVGNASGNWHCDSTWLGDRVITTSTRTWAL
PTYNNHLYKQISSASTGASNDNHYFGYSTPWGYFDFNRFHCHFSPRDWQRLINNNWG
FRPKRLNFKLFNIQVKEVTTNDGVTTIANNLTSTVQVFSDSEYQLPYVLGSAHQGCLPP
FPADVFMIPQYGYLTLNNGSQAVGRSSFYCLEYFPSQMLRTGNNFTFSYTFEDVPFHSS
YAHSQSLDRLMNPLIDQYLYYLNRTQNQSGSAQNKDLLFSRGSPAGMSVQPKNWLPG
PCYRQQRVSKTKTDNNNSNFTWTGASKYNLNGRESIINPGTAMASHKDDKDKFFPMS
GVMIFGKESAGASNTALDNVMITDEEEIKATNPVATERFGTVAVNLQSSSTDPATGDVH
VMGALPGMVWQDRDVYLQGPIWAKIPHTDGHFHPSPLMGGFGLKHPPPQILIKNTPVP
ANPPAEFSATKFASFITQYSTGQVSVEIEWELQKENSKRWNPEVQYTSNYAKSANVDFT
VDNNGLYTEPRPIGTRYLTRPL
 (Amino acids 138-736 in AAV6 VP1, a total of 599 amino acids)

The amino acid sequence of AAV6 VP3 is as shown below (SEQ ID NO: 19): 
MASGGGAPMADNNEGADGVGNASGNWHCDSTWLGDRVITTSTRTWALPTYNN
HLYKQISSASTGASNDNHYFGYSTPWGYFDFNRFHCHFSPRDWQRLINNNWGFRPKR
 
LNFKLFNIQVKEVTTNDGVTTIANNLTSTVQVFSDSEYQLPYVLGSAHQGCLPPFPADV
FMIPQYGYLTLNNGSQAVGRSSFYCLEYFPSQMLRTGNNFTFSYTFEDVPFHSSYAHSQ
SLDRLMNPLIDQYLYYLNRTQNQSGSAQNKDLLFSRGSPAGMSVQPKNWLPGPCYRQ
QRVSKTKTDNNNSNFTWTGASKYNLNGRESIINPGTAMASHKDDKDKFFPMSGVMIF
GKESAGASNTALDNVMITDEEEIKATNPVATERFGTVAVNLQSSSTDPATGDVHVMGAL
PGMVWQDRDVYLQGPIWAKIPHTDGHFHPSPLMGGFGLKHPPPQILIKNTPVPANPPAE
FSATKFASFITQYSTGQVSVEIEWELQKENSKRWNPEVQYTSNYAKSANVDFTVDNNG
LYTEPRPIGTRYLTRPL
 (Amino acids 203-736 in AAV6 VP1, a total of 534 amino acids)

The amino acid sequence of AAV9 VP1 is as shown below (SEQ ID NO: 20): 
MAADGYLPDWLEDNLSEGIREWWALKPGAPQPKANQQHQDNARGLVLPGYKY
LGPGNGLDKGEPVNAADAAALEHDKAYDQQLKAGDNPYLKYNHADAEFQERLKED
TSFGGNLGRAVFQAKKRLLEPLGLVEEAAKTAPGKKRPVEQSPQEPDSSAGIGKSGAQP
AKKRLNFGQTGDTESVPDPQPIGEPPAAPSGVGSLTMASGGGAPVADNNEGADGVGSS
SGNWHCDSQWLGDRVITTSTRTWALPTYNNHLYKQISNSTSGGSSNDNAYFGYSTPW
GYFDFNRFHCHFSPRDWQRLINNNWGFRPKRLNFKLFNIQVKEVTDNNGVKTIANNLT
STVQVFTDSDYQLPYVLGSAHEGCLPPFPADVFMIPQYGYLTLNDGSQAVGRSSFYCLE
YFPSQMLRTGNNFQFSYEFENVPFHSSYAHSQSLDRLMNPLIDQYLYYLSKTINGSGQN
QQTLKFSVAGPSNMAVQGRNYIPGPSYRQQRVSTTVTQNNNSEFAWPGASSWALNGR
NSLMNPGPAMASHKEGEDRFFPLSGSLIFGKQGTGRDNVDADKVMITNEEEIKTTNPV
ATESYGQVATNHQSAQAQAQTGWVQNQGILPGMVWQDRDVYLQGPIWAKIPHTDGN
FHPSPLMGGFGMKHPPPQILIKNTPVPADPPTAFNKDKLNSFITQYSTGQVSVEIEWELQ
KENSKRWNPEIQYTSNYYKSNNVEFAVNTEGVYSEPRPIGTRYLTRNL
 (A total of 736 amino acids)

The amino acid sequence of AAV9 VP2 is as shown below (SEQ ID NO: 21): 
TAPGKKRPVEQSPQEPDSSAGIGKSGAQPAKKRLNFGQTGDTESVPDPQPIGEPPA
APSGVGSLTMASGGGAPVADNNEGADGVGSSSGNWHCDSQWLGDRVITTSTRTWALP
TYNNHLYKQISNSTSGGSSNDNAYFGYSTPWGYFDFNRFHCHFSPRDWQRLINNNWG
FRPKRLNFKLFNIQVKEVTDNNGVKTIANNLTSTVQVFTDSDYQLPYVLGSAHEGCLP
PFPADVFMIPQYGYLTLNDGSQAVGRSSFYCLEYFPSQMLRTGNNFQFSYEFENVPFHS
SYAHSQSLDRLMNPLIDQYLYYLSKTINGSGQNQQTLKFSVAGPSNMAVQGRNYIPGPS
YRQQRVSTTVTQNNNSEFAWPGASSWALNGRNSLMNPGPAMASHKEGEDRFFPLSGS
LIFGKQGTGRDNVDADKVMITNEEEIKTTNPVATESYGQVATNHQSAQAQAQTGWVQ
NQGILPGMVWQDRDVYLQGPIWAKIPHTDGNFHPSPLMGGFGMKHPPPQILIKNTPVP
ADPPTAFNKDKLNSFITQYSTGQVSVEIEWELQKENSKRWNPEIQYTSNYYKSNNVEFA
 VNTEGVYSEPRPIGTRYLTRNL (Amino acids 138-736 in AAV9 VP1, a total of 599 amino acids)

The amino acid sequence of AAV9 VP3 is as shown below (SEQ ID NO: 22): 
MASGGGAPVADNNEGADGVGSSSGNWHCDSQWLGDRVITTSTRTWALPTYNNH
LYKQISNSTSGGSSNDNAYFGYSTPWGYFDFNRFHCHFSPRDWQRLINNNWGFRPKRL
NFKLFNIQVKEVTDNNGVKTIANNLTSTVQVFTDSDYQLPYVLGSAHEGCLPPFPADVF
MIPQYGYLTLNDGSQAVGRSSFYCLEYFPSQMLRTGNNFQFSYEFENVPFHSSYAHSQS
LDRLMNPLIDQYLYYLSKTINGSGQNQQTLKFSVAGPSNMAVQGRNYIPGPSYRQQRV
STTVTQNNNSEFAWPGASSWALNGRNSLMNPGPAMASHKEGEDRFFPLSGSLIFGKQG
TGRDNVDADKVMITNEEEIKTTNPVATESYGQVATNHQSAQAQAQTGWVQNQGILPG
MVWQDRDVYLQGPIWAKIPHTDGNFHPSPLMGGFGMKHPPPQILIKNTPVPADPPTAF
NKDKLNSFITQYSTGQVSVEIEWELQKENSKRWNPEIQYTSNYYKSNNVEFAVNTEGV
YSEPRPIGTRYLTRNL
 (Amino acids 203-736 in AAV9 VP1, a total of 534 amino acids)

In some embodiments, the targeting peptide is inserted at a position corresponding to amino acid 453 and/or a position corresponding to amino acid 588 in AAV2 VP1, AAV2 VP2 and/or AAV2 VP3. Specifically, the targeting peptide is inserted at a position corresponding to amino acid 453 and/or amino acid 588 in AAV2 VP1, such that the targeting peptide is inserted at both the position(s) in AAV2 VP2 corresponding to amino acid 453 and/or amino acid 588 in AAV2 VP1 and the position(s) in AAV2 VP3 corresponding to amino acid 453 and/or amino acid 588 in AAV2 VP1.

In some embodiments, the targeting peptide is inserted at a position corresponding to amino acid 454 and/or a position corresponding to amino acid 588 in AAV6 VP1, AAV6 VP2 and/or AAV6 VP3. Specifically, the targeting peptide is inserted at the position corresponding to amino acid 454 or 588 in AAV6 VP1, such that the targeting peptide is inserted at both the position(s) in AAV6 VP2 corresponding to amino acid 454 and/or amino acid 588 in AAV6 VP1 and the position(s) in AAV6 VP3 corresponding to amino acid 454 and/or amino acid 588 in AAV6 VP1.

In some embodiments, the targeting peptide is inserted at a position corresponding to amino acid 455 and/or a position corresponding to amino acid 588 in AAV9 VP1, AAV9 VP2 and/or AAV9 VP3. Specifically, the targeting peptide is inserted at the position corresponding to amino acid 455 or 588 in AAV9 VP1, such that the targeting peptide is inserted at both the position(s) in AAV9 VP2 corresponding to amino acid 455 and/or amino acids 588 in AAV9 VP1 and the position(s) in AAV9 VP3 corresponding to amino acid 455 and/or amino acids 588 in AAV9 VP1.

In some preferred embodiments, the targeting peptide is inserted at the position corresponding to amino acid 453 in AAV2 VP1, such that the targeting peptide is inserted at both the position in AAV2 VP2 corresponding to amino acid 453 in AAV2 VP1 and the position in AAV2 VP3 corresponding to amino acid 453 in AAV2 VP1. In some preferred embodiments, the targeting peptide is inserted at the position corresponding to amino acid 454 in AAV6 VP1, such that the targeting peptide is inserted at both the position in AAV6 VP2 corresponding to amino acid 454 of AAV6 VP1 and the position in AAV6 VP3 corresponding to amino acid 454 in AAV6 VP1. In some preferred embodiments, the targeting peptide is inserted at the position corresponding to amino acid 588 in AAV9 VP1, such that the targeting peptide is inserted at both the position in AAV9 VP2 corresponding to amino acid 588 in AAV9 VP1 and the position in AAV9 VP3 corresponding to amino acid 588 in AAV9 VP1.

Targeting peptides disclosed herein can be modified according to the methods known for producing peptidomimetics in the art. See, *e.g.,* Qvit et al., Drug Discov Today. 2017 Feb.; 22(2): 454-462; Farhadi and Hashemian, Drug Des Devel Ther. 2018; 12: 1239-1254; Avan et al., Chem. Soc. Rev., 2014, 43, 3575-3594; Pathak et al., Indo American Journal of Pharmaceutical Research, 2015. 8; Kazmierski, W.M., ed., Peptidomimetics Protocols, Human Press (Totowa NJ 1998); Goodman et al., ed., Houben-Weyl Methods of Organic Chemistry: Synthesis of Peptides and Peptidomimetics, Thiele Verlag (New York 2003); and Mayo et al., J. Biol. Chem., 278: 45746(2003). In some cases, these modified peptidomimetic versions of the peptides and fragments disclosed herein exhibit enhanced stability *in vivo,* relative to the non-peptidomimetic peptides.

Methods for creating a peptidomimetic include substituting one or more, *e.g.,* all, of the amino acids in a peptide sequence with D-amino acid enantiomers. Such sequences are referred to herein as "retro" sequences. In another method, the N-terminal to C-terminal order of the amino acid residues is reversed, such that the order of amino acid residues from the N terminus to the C terminus of the original peptide becomes the order of amino acid residues from the C-terminus to the N-terminus in the modified peptidomimetic. Such sequences can be referred to as "inverso" sequences.

Peptidomimetics can be both the retro and inverso versions, *i.e.,* the "retro-inverso" version of a peptide disclosed herein. The new peptidomimetics can be composed of D-amino acids arranged so that the order of amino acid residues from the N-terminus to the C-terminus in the peptidomimetic corresponds to the order of amino acid residues from the C-terminus to the N-terminus in the original peptide.

Other methods for making a peptidomimetic include replacing one or more amino acid residues in a peptide with a chemically distinct but recognized functional analog of the amino acid, i.e., an artificial amino acid analog. Artificial amino acid analogs include β-amino acids, β-substituted β-amino acids ("β³-amino acids"), phosphorous analogs of amino acids, such as ∀-amino phosphonic acids and ∀-amino phosphinic acids, and amino acids having non-peptide linkages. Artificial amino acids can be used to create peptidomimetics, such as peptoid oligomers (*e*.*g*., peptoid amide or ester analogues), β-peptides, cyclic peptides, oligourea or oligocarbamate peptides; or heterocyclic ring molecules.

### Polynucleotide

In some embodiments of the present disclosure, there is provided a polynucleotide encoding the AAV capsid protein according to the present disclosure.

The polynucleotide of the present disclosure may be in a DNA or RNA form. The DNA form includes cDNA, genomic DNA or synthetic DNA. DNA may be single-stranded or double-stranded. DNA may be a coding strand or a non-coding strand.

The polynucleotide encoding the AAV capsid protein according to the present disclosure includes: a coding sequence encoding only the AAV capsid protein/targeting peptide; a coding sequence of the AAV capsid protein/targeting peptide and various additional coding sequences; and a coding sequence (and optionally additional coding sequences) of the AAV capsid protein/targeting peptide and a non-coding sequence.

The "polynucleotide encoding the AAV capsid protein/targeting peptide" may be a polynucleotide containing the coding sequence encoding the AAV capsid protein/targeting peptide, or may be a polynucleotide further containing an additional coding sequence and/or a non-coding sequence.

The present disclosure further relates to a polynucleotide whose sequence hybridizes with and having at least 50%, preferably at least 70%, more preferably at least 80% identity to the above-mentioned sequence. The present disclosure relates particularly to a polynucleotide hybridizable with the polynucleotide according to the present disclosure under stringency conditions. The stringency conditions are moderate stringency conditions, moderate-high stringency conditions, high stringency conditions or very high stringency conditions.

### AAV and AAV Vectors

In some embodiments of the present disclosure, there is provided an AAV, comprising the AAV capsid protein according to the present disclosure.

In some embodiments, the AAV further comprises a transgene.

In some specific embodiments, the transgene comprises a nucleotide sequence encoding a gene product. "Gene" refers to a polynucleotide containing at least one open reading frame capable of encoding a specific protein after transcription and translation. "Gene product" is a molecule resulting from expression of a particular gene. The gene product includes, *e*.*g*., polypeptides, aptamers, interfering RNAs, and mRNAs.

In some specific embodiments, the transgene is a therapeutic transgene, a prophylactic transgene, or a diagnostic transgene.

In some exemplary embodiments, the transgene is a therapeutic transgene encoding the gene sequence of a therapeutic agent, *e*.*g*., encoding the gene sequence of an anti-cancer (brain tumor) agent or encoding the gene sequence of a therapeutic agent for an additional brain disease.

According to some aspects of the present disclosure, there is provided a transgene delivery vector, comprising the AAV according to the present disclosure.

### Pharmaceutical Composition and Methods of Administration

In some embodiments of the present disclosure, there is provided a pharmaceutical composition, comprising:
(a) the AAV or the transgene delivery vector as described above; and optionally
(b) a pharmaceutically acceptable carrier.

In some embodiments, the pharmaceutical composition according to the present disclosure contains the aforementioned AAV or the aforementioned transgene delivery vector as an active ingredient, the AAV or the transgene delivery vector comprising (i) a targeting peptide and (ii) a transgene, for example, a therapeutic transgene, exemplarily, therapeutic agents for treating brain tumors or other brain diseases.

As used herein, the term "pharmaceutically acceptable carrier" includes saline, solvents, dispersion media, coatings, antibacterial and antifungal agents, isotonic and absorption delaying agents, and the like, compatible with pharmaceutical administration.

Pharmaceutical compositions are typically formulated to be compatible with its intended route of administration. Examples of routes of administration include parenteral, *e*.*g*., intravenous, intraarterial, subcutaneous, intraperitoneal, intramuscular, intracerebrospinal fluid, intraparenchymal, or intratumoral injection or infusion administration. Delivery can thus be systemic or localized.

Methods of formulating suitable pharmaceutical compositions are known in the art, see, *e.g.,* Remington: The Science and Practice of Pharmacy, 21st ed., 2005; and the books in the series Drugs and the Pharmaceutical Sciences: a Series of Textbooks and Monographs (Dekker, NY). For example, solutions or suspensions used for parenteral application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use may include sterile aqueous solutions (water soluble) or dispersions and sterile powders for the extemporaneous preparation of sterile injectable solutions or dispersions. For intravenous administration, suitable carriers include physiological saline, bacteriostatic water, Cremophor EL^{™} (BASF, Parsippany, NJ) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent that delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, the preferred methods of preparation are vacuum drying and freeze-drying, which yield a powder of the active ingredient plus any additional desired ingredient from a previously sterile-filtered solution thereof.

In one embodiment, the therapeutic compounds are prepared with carriers that will protect the therapeutic compounds against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Such formulations can be prepared using standard techniques, or commercially available, e.g., from Alza Corporation and Nova Pharmaceuticals, Inc. Liposomal suspensions (including liposomes targeted to selected cells with monoclonal antibodies to cellular antigens) can also be used as pharmaceutically acceptable carriers. These can be prepared according to methods known to those skilled in the art, for example, as described in U.S. Patent No. 4,522,811.

The pharmaceutical compositions can be included in a kit, container, pack, or dispenser together with instructions for administration.

### Uses and Methods

In some embodiments of the present disclosure, there is provided use of the adeno-associated virus according to the present disclosure, the transgene delivery vector according to the present disclosure and/or the pharmaceutical composition according to the present disclosure in preparation of a reagent for delivering a transgene to a cell.

In other embodiments of the present disclosure, there is provided a method for delivering a transgene to a cell, the method comprising a step of bringing the cell into contact with the AAV according to the present disclosure, the transgene delivery vector according to the present disclosure and/or the pharmaceutical composition according to the present disclosure.

The AAV according to the present disclosure can be used to deliver a transgene such as a therapeutic transgene to a tissue, *e*.*g*., to the central nervous system (brain), heart, muscle, or dorsal root ganglion or spinal cord (peripheral nervous system). In some embodiments, the AAV according to the present disclosure can deliver the transgene such as a therapeutic transgene to specific brain regions, *e*.*g*., cortex, cerebellum, hippocampus, substantia nigra, or amygdala. In some embodiments, the AAV according to the present disclosure can deliver the transgene/therapeutic transgene to neurons, astrocytes and/or glial cells.

In some embodiments, the AAVs according to the present disclosure are used to deliver a nucleic acid sequence encoding a therapeutic agent to a subject who has a brain disease. In some specific embodiments, the AAVs according to the present disclosure are used to deliver a nucleic acid sequence encoding a therapeutic agent to a subject who has a brain tumor. Brain tumors include gliomas (*e.g.,* glioblastoma multiforme (GBM)), metastases (*e.g.,* from lung cancer, breast cancer, melanoma, or colon cancer), meningiomas, pituitary adenomas, and acoustic neuromas. Thus the AAV comprising the targeting peptide as described herein and encoding an immunotherapeutic agent can be systemically, *e*.*g*., intravenously, administered to a subject who has been diagnosed with a brain tumor.

In other specific embodiments, the AAVs according to the present disclosure are used to deliver a nucleic acid sequence encoding a therapeutic agent to a subject who has an additional brain disease, *e*.*g*., subjects having cerebrovascular diseases (exemplarily, cerebral arteriosclerosis, cerebral vascular stenosis, cerebral infarction, cerebral hemorrhage, arteriovenous malformation, aneurysm, etc.); brain infectious diseases (exemplarily, encephalitis and meningitis); neurodegenerative diseases (exemplarily, Alzheimer's disease and Parkinson's disease), but not limited thereto.

In some embodiments, the AAVs according to the present disclosure can also be co-administered with a chemotherapeutic agent. In some embodiments, the chemotherapeutic agent is a toxin or cytotoxic drug, including but not limited to temozolamide, lomustine, or a combination thereof. See, *e.g.,* Herrlinger et al., Lancet. 2019 Feb 16; 393(10172):678-688. The methods can also include administering radiation, surgical resection, or both.

In some specific embodiments of the present disclosure, there is provided use of the adeno-associated virus according to the present disclosure, the transgene delivery vector according to the present disclosure and/or the pharmaceutical composition according to the present disclosure in preparation of a medicament for treating a disease.

In other specific embodiments of the present disclosure, there is provided a method for treating a disease, comprising a step of administering, to a subject, the adeno-associated virus according to the present disclosure, the transgene delivery vector according to the present disclosure and/or the pharmaceutical composition according to the present disclosure.

In the present disclosure, the disease may be any disease that can be treated by an adeno-associated virus carrying a transgene. In some preferred embodiments, the disease may be a brain disease. In some specific embodiments, the brain disease includes brain tumors; cerebrovascular diseases (exemplarily, cerebral arteriosclerosis, cerebral vascular stenosis, cerebral infarction, cerebral hemorrhage, arteriovenous malformation, aneurysm, etc.); brain infectious diseases (exemplarily, encephalitis and meningitis); neurodegenerative diseases (exemplarily, Alzheimer's disease and Parkinson's disease), but is not limited thereto.

### Examples

The present disclosure will be described below in greater details with reference to the specific embodiments. The examples described below are merely intended to illustrate the present disclosure, rather than to limit its scope. The examples provided below may serve as guidance for further improvements by one of ordinary skill in the art and are not intended to limit the present disclosure in any way.

Unless otherwise specified, the experimental methods in the following examples are all conventional methods and are carried out according to the techniques or conditions described in the literatures in this field or according to the product instructions. All the materials, reagents and the like used in the following examples are commercially available, unless otherwise specified.

### Materials and Methods

In the following examples, the conventional three-plasmid co-transfection technique is used to prepare recombinant AAVs. The three plasmids include RC plasmid (containing the nucleotide sequence of capsid *Cap*), Helper plasmid (providing co-factors required for generating AAVs), and pAAV plasmid (providing the nucleotide sequence to be delivered, and generally containing the promoter, cDNA and the like required for protein translation). The AAV serotype is determined by the RC plasmid. For example, RC2 is used to package and prepare AAV2 serotype, RC6 is used to package and prepare AAV6 serotype, RC9 is used to package and prepare AAV9 serotype. The capsid modification is realized by genetically modifying the RC plasmid.

### 1. Construction of AAV Capsid Plasmids

Three capsid plasmids were used in the following examples: RC2, RC6, and RC9 (the nucleotide sequence of RC2 was set forth in SEQ ID NO: 1, the nucleotide sequence of RC6 was set forth in SEQ ID NO: 2, and the nucleotide sequence of RC9 was set forth in SEQ ID NO: 3). The nucleotide sequence (SEQ ID NO: 4) corresponding to the short peptide GRGDSP was inserted by the molecular biological technique at the site corresponding to amino acid 453 of the VP1 gene (SEQ ID NO: 5) in the RC2 plasmid to obtain a corresponding RC2.2 plasmid (SEQ ID NO: 6). Similarly, the nucleotide sequence (SEQ ID NO: 4) corresponding to the short peptide GRGDSP was inserted at the site corresponding to amino acid 588 of the VP1 gene (SEQ ID NO: 5) in the RC2 plasmid to obtain a corresponding RC2.3 plasmid (SEQ ID NO: 7). The nucleotide sequence (SEQ ID NO: 4) corresponding to the short peptide GRGDSP was inserted at the site corresponding to amino acid 454 of the VP1 gene (SEQ ID NO: 8) in the RC6 plasmid to obtain a corresponding RC6.2 plasmid (SEQ ID NO: 9). The nucleotide sequence (SEQ ID NO: 4) corresponding to the short peptide GRGDSP was inserted at the site corresponding to amino acid 588 of the VP1 gene (SEQ ID NO: 8) in the RC6 plasmid to obtain a corresponding RC6.3 plasmid (SEQ ID NO: 10). The nucleotide sequence (SEQ ID NO: 4) corresponding to the short peptide GRGDSP was inserted at the site corresponding to amino acid 455 of the VP1 gene (SEQ ID NO: 11) in the RC9 plasmid to obtain a corresponding RC9.2 plasmid (SEQ ID NO: 12). The nucleotide sequence (SEQ ID NO: 4) corresponding to the short peptide GRGDSP was inserted at the site corresponding to amino acid 588 of the VP1 gene (SEQ ID NO: 11) in the RC9 plasmid to obtain a corresponding RC9.3 plasmid (SEQ ID NO: 13). All the work of nucleotide synthesis and plasmid construction was finished by Nanjing GenScript Biotechnology Corporation. The related specific sequences were as shown below.
SEQ ID NO: 1 (nucleotide sequence of RC2 plasmid): 
GACAGGTTTCCCGACTGGAAAGCGGGCAGTGAGCGCAACGCAATTAATGTGA
GTTAGCTCACTCATTAGGCACCCCAGGCTTTACACTTTATGCTTCCGGCTCGTATGTT
GTGTGGAATTGTGAGCGGATAACAATTTCACACAGGAAACAGCTATGACCATGATTA
CGCCAAGCGCGCCGATATCGTTAACGCCCCGCGCCGGCCGCTCTAGAACTAGTGGA
TCCCCCGGAAGATCAGAAGTTCCTATTCCGAAGTTCCTATTCTCTAGAAAGTATAGG
AACTTCTGATCTGCGCAGCCGCCATGCCGGGGTTTTACGAGATTGTGATTAAGGTCC
CCAGCGACCTTGACGAGCATCTGCCCGGCATTTCTGACAGCTTTGTGAACTGGGTG
GCCGAGAAGGAATGGGAGTTGCCGCCAGATTCTGACATGGATCTGAATCTGATTGA
GCAGGCACCCCTGACCGTGGCCGAGAAGCTGCAGCGCGACTTTCTGACGGAATGG
CGCCGTGTGAGTAAGGCCCCGGAGGCCCTTTTCTTTGTGCAATTTGAGAAGGGAGA
GAGCTACTTCCACATGCACGTGCTCGTGGAAACCACCGGGGTGAAATCCATGGTTT
TGGGACGTTTCCTGAGTCAGATTCGCGAAAAACTGATTCAGAGAATTTACCGCGGG
ATCGAGCCGACTTTGCCAAACTGGTTCGCGGTCACAAAGACCAGAAATGGCGCCG
GAGGCGGGAACAAGGTGGTGGATGAGTGCTACATCCCCAATTACTTGCTCCCCAAA
ACCCAGCCTGAGCTCCAGTGGGCGTGGACTAATATGGAACAGTATTTAAGCGCCTG
TTTGAATCTCACGGAGCGTAAACGGTTGGTGGCGCAGCATCTGACGCACGTGTCGC
AGACGCAGGAGCAGAACAAAGAGAATCAGAATCCCAATTCTGATGCGCCGGTGAT
CAGATCAAAAACTTCAGCCAGGTACATGGAGCTGGTCGGGTGGCTCGTGGACAAG
GGGATTACCTCGGAGAAGCAGTGGATCCAGGAGGACCAGGCCTCATACATCTCCTT
CAATGCGGCCTCCAACTCGCGGTCCCAAATCAAGGCTGCCTTGGACAATGCGGGAA
AGATTATGAGCCTGACTAAAACCGCCCCCGACTACCTGGTGGGCCAGCAGCCCGTG
GAGGACATTTCCAGCAATCGGATTTATAAAATTTTGGAACTAAACGGGTACGATCCC
CAATATGCGGCTTCCGTCTTTCTGGGATGGGCCACGAAAAAGTTCGGCAAGAGGAA
CACCATCTGGCTGTTTGGGCCTGCAACTACCGGGAAGACCAACATCGCGGAGGCCA
TAGCCCACACTGTGCCCTTCTACGGGTGCGTAAACTGGACCAATGAGAACTTTCCCT
TCAACGACTGTGTCGACAAGATGGTGATCTGGTGGGAGGAGGGGAAGATGACCGC
CAAGGTCGTGGAGTCGGCCAAAGCCATTCTCGGAGGAAGCAAGGTGCGCGTGGAC
CAGAAATGCAAGTCCTCGGCCCAGATAGACCCGACTCCCGTGATCGTCACCTCCAA
CACCAACATGTGCGCCGTGATTGACGGGAACTCAACGACCTTCGAACACCAGCAG
CCGTTGCAAGACCGGATGTTCAAATTTGAACTCACCCGCCGTCTGGATCATGACTTT
GGGAAGGTCACCAAGCAGGAAGTCAAAGACTTTTTCCGGTGGGCAAAGGATCACG
TGGTTGAGGTGGAGCATGAATTCTACGTCAAAAAGGGTGGAGCCAAGAAAAGACC
CGCCCCCAGTGACGCAGATATAAGTGAGCCCAAACGGGTGCGCGAGTCAGTTGCGC
AGCCATCGACGTCAGACGCGGAAGCTTCGATCAACTACGCAGACAGGTACCAAAA
 
CAAATGTTCTCGTCACGTGGGCATGAATCTGATGCTGTTTCCCTGCAGACAATGCGA
GAGAATGAATCAGAATTCAAATATCTGCTTCACTCACGGACAGAAAGACTGTTTAG
AGTGCTTTCCCGTGTCAGAATCTCAACCCGTTTCTGTCGTCAAAAAGGCGTATCAGA
AACTGTGCTACATTCATCATATCATGGGAAAGGTGCCAGACGCTTGCACTGCCTGCG
ATCTGGTCAATGTGGATTTGGATGACTGCATCTTTGAACAATAAATGATTTAAATCAG
GTATGGCTGCCGATGGTTATCTTCCAGATTGGCTCGAGGACACTCTCTCTGAAGGAA
TAAGACAGTGGTGGAAGCTCAAACCTGGCCCACCACCACCAAAGCCCGCAGAGCG
GCATAAGGACGACAGCAGGGGTCTTGTGCTTCCTGGGTACAAGTACCTCGGACCCT
TCAACGGACTCGACAAGGGAGAGCCGGTCAACGAGGCAGACGCCGCGGCCCTCG
AGCACGACAAAGCCTACGACCGGCAGCTCGACAGCGGAGACAACCCGTACCTCAA
GTACAACCACGCCGACGCGGAGTTTCAGGAGCGCCTTAAAGAAGATACGTCTTTTG
GGGGCAACCTCGGACGAGCAGTCTTCCAGGCGAAAAAGAGGGTTCTTGAACCTCT
GGGCCTGGTTGAGGAACCTGTTAAGACGGCTCCGGGAAAAAAGAGGCCGGTAGAG
CACTCTCCTGTGGAGCCAGACTCCTCCTCGGGAACCGGAAAGGCGGGCCAGCAGC
CTGCAAGAAAAAGATTGAATTTTGGTCAGACTGGAGACGCAGACTCAGTACCTGAC
CCCCAGCCTCTCGGACAGCCACCAGCAGCCCCCTCTGGTCTGGGAACTAATACGAT
GGCTACAGGCAGTGGCGCACCAATGGCAGACAATAACGAGGGCGCCGACGGAGTG
GGTAATTCCTCGGGAAATTGGCATTGCGATTCCACATGGATGGGCGACAGAGTCATC
ACCACCAGCACCCGAACCTGGGCCCTGCCCACCTACAACAACCACCTCTACAAACA
AATTTCCAGCCAATCAGGAGCCTCGAACGACAATCACTACTTTGGCTACAGCACCC
CTTGGGGGTATTTTGACTTCAACAGATTCCACTGCCACTTTTCACCACGTGACTGGC
AAAGACTCATCAACAACAACTGGGGATTCCGACCCAAGAGACTCAACTTCAAGCT
CTTTAACATTCAAGTCAAAGAGGTCACGCAGAATGACGGTACGACGACGATTGCCA
ATAACCTTACCAGCACGGTTCAGGTGTTTACTGACTCGGAGTACCAGCTCCCGTACG
TCCTCGGCTCGGCGCATCAAGGATGCCTCCCGCCGTTCCCAGCAGACGTCTTCATG
GTGCCACAGTATGGATACCTCACCCTGAACAACGGGAGTCAGGCAGTAGGACGCTC
TTCATTTTACTGCCTGGAGTACTTTCCTTCTCAGATGCTGCGTACCGGAAACAACTTT
ACCTTCAGCTACACTTTTGAGGACGTTCCTTTCCACAGCAGCTACGCTCACAGCCA
GAGTCTGGACCGTCTCATGAATCCTCTCATCGACCAGTACCTGTATTACTTGAGCAG
AACAAACACTCCAAGTGGAACCACCACGCAGTCAAGGCTTCAGTTTTCTCAGGCC
GGAGCGAGTGACATTCGGGACCAGTCTAGGAACTGGCTTCCTGGACCCTGTTACCG
CCAGCAGCGAGTATCAAAGACATCTGCGGATAACAACAACAGTGAATACTCGTGGA
CTGGAGCTACCAAGTACCACCTCAATGGCAGAGACTCTCTGGTGAATCCGGGCCCG
GCCATGGCAAGCCACAAGGACGATGAAGAAAAGTTTTTTCCTCAGAGCGGGGTTCT
CATCTTTGGGAAGCAAGGCTCAGAGAAAACAAATGTGGACATTGAAAAGGTCATGA
 
TTACAGACGAAGAGGAAATCAGGACAACCAATCCCGTGGCTACGGAGCAGTATGGT
TCTGTATCTACCAACCTCCAGAGAGGCAACAGACAAGCAGCTACCGCAGATGTCAA
CACACAAGGCGTTCTTCCAGGCATGGTCTGGCAGGACAGAGATGTGTACCTTCAGG
GGCCCATCTGGGCAAAGATTCCACACACGGACGGACATTTTCACCCCTCTCCCCTCA
TGGGTGGATTCGGACTTAAACACCCTCCTCCACAGATTCTCATCAAGAACACCCCG
GTACCTGCGAATCCTTCGACCACCTTCAGTGCGGCAAAGTTTGCTTCCTTCATCACA
CAGTACTCCACGGGACAGGTCAGCGTGGAGATCGAGTGGGAGCTGCAGAAGGAAA
ACAGCAAACGCTGGAATCCCGAAATTCAGTACACTTCCAACTACAACAAGTCTGTT
AATGTGGACTTTACTGTGGACACTAATGGCGTGTATTCAGAGCCTCGCCCCATTGGC
ACCAGATACCTGACTCGTAATCTGTAATTGCTTGTTAATCAATAAACCGTTTAATTCG
TTTCAGTTGAACTTTGGTCTCTGCGTATTTCTTTCTTATCTAGTTTCCATGGCTACGTA
GATAAGTAGCATGGCGGGTTAATCATTAACTACAGCCCGGGCGTTTAAACAGCGGGC
GGAGGGGTGGAGTCGTGACGTGAATTACGTCATAGGGTTAGGGAGGTCCTGTATTA
GAGGTCACGTGAGTGTTTTGCGACATTTTGCGACACCATGTGGTCTCGCTGGGGGG
GGGGGCCCGAGTGAGCACGCAGGGTCTCCATTTTGAAGCGGGAGGTTTGAACGAG
CGCTGGCGCGCTCACTGGCCGTCGTTTTACAACGTCGTGACTGGGAAAACCCTGGC
GTTACCCAACTTAATCGCCTTGCAGCACATCCCCCTTTCGCCAGCTGGCGTAATAGC
GAAGAGGCCCGCACCGATCGCCCTTCCCAACAGTTGCGCAGCCTGAATGGCGAATG
GAAATTGTAAGCGTTAATATTTTGTTAAAATTCGCGTTAAATTTTTGTTAAATCAGCT
CATTTTTTTAACCAATAGGCCGAAATCGGCAAAATCCCTTATAAATCAAAAGAATAG
ACCGAGATAGGGTTGAGTGTTGTTCCAGTTTGGAACAAGAGTCCACTATTAAGAAC
GTGGACTCCAACGTCAAAGGGCGAAAAACCGTCTATCAGGGCGATGGCCCACTAC
GTGAACCATCACCCTAATCAAGTTTTTTGGGGTCGAGGTGCCGTAAAGCACTAAATC
GGAACCCTAAAGGGAGCCCCCGATTTAGAGCTTGACGGGGAAAGCCGGCGAACGT
GGCGAGAAAGGAAGGGAAGAAAGCGAAAGGAGCGGGCGCTAGGGCGCTGGCAAG
TGTAGCGGTCACGCTGCGCGTAACCACCACACCCGCCGCGCTTAATGCGCCGCTAC
AGGGCGCGTCAGGTGGCACTTTTCGGGGAAATGTGCGCGGAACCCCTATTTGTTTAT
TTTTCTAAATACATTCAAATATGTATCCGCTCATGAGACAATAACCCTGATAAATGCTT
CAATAATATTGAAAAAGGAAGAGTATGAGTATTCAACATTTCCGTGTCGCCCTTATTC
CCTTTTTTGCGGCATTTTGCCTTCCTGTTTTTGCTCACCCAGAAACGCTGGTGAAAG
TAAAAGATGCTGAAGATCAGTTGGGTGCACGAGTGGGTTACATCGAACTGGATCTC
AACAGCGGTAAGATCCTTGAGAGTTTTCGCCCCGAAGAACGTTTTCCAATGATGAG
CACTTTTAAAGTTCTGCTATGTGGCGCGGTATTATCCCGTATTGACGCCGGGCAAGA
GCAACTCGGTCGCCGCATACACTATTCTCAGAATGACTTGGTTGAGTACTCACCAGT
CACAGAAAAGCATCTTACGGATGGCATGACAGTAAGAGAATTATGCAGTGCTGCCAT
 
AACCATGAGTGATAACACTGCGGCCAACTTACTTCTGACAACGATCGGAGGACCGA
AGGAGCTAACCGCTTTTTTGCACAACATGGGGGATCATGTAACTCGCCTTGATCGTT
GGGAACCGGAGCTGAATGAAGCCATACCAAACGACGAGCGTGACACCACGATGCC
TGTAGCAATGGCAACAACGTTGCGCAAACTATTAACTGGCGAACTACTTACTCTAGC
TTCCCGGCAACAATTAATAGACTGGATGGAGGCGGATAAAGTTGCAGGACCACTTC
TGCGCTCGGCCCTTCCGGCTGGCTGGTTTATTGCTGATAAATCTGGAGCCGGTGAGC
GTGGGTCTCGCGGTATCATTGCAGCACTGGGGCCAGATGGTAAGCCCTCCCGTATCG
TAGTTATCTACACGACGGGGAGTCAGGCAACTATGGATGAACGAAATAGACAGATC
GCTGAGATAGGTGCCTCACTGATTAAGCATTGGTAACTGTCAGACCAAGTTTACTCA
TATATACTTTAGATTGATTTAAAACTTCATTTTTAATTTAAAAGGATCTAGGTGAAGAT
CCTTTTTGATAATCTCATGACCAAAATCCCTTAACGTGAGTTTTCGTTCCACTGAGCG
TCAGACCCCGTAGAAAAGATCAAAGGATCTTCTTGAGATCCTTTTTTTCTGCGCGTA
ATCTGCTGCTTGCAAACAAAAAAACCACCGCTACCAGCGGTGGTTTGTTTGCCGGA
TCAAGAGCTACCAACTCTTTTTCCGAAGGTAACTGGCTTCAGCAGAGCGCAGATAC
CAAATACTGTTCTTCTAGTGTAGCCGTAGTTAGGCCACCACTTCAAGAACTCTGTAG
CACCGCCTACATACCTCGCTCTGCTAATCCTGTTACCAGTGGCTGCTGCCAGTGGCG
ATAAGTCGTGTCTTACCGGGTTGGACTCAAGACGATAGTTACCGGATAAGGCGCAG
CGGTCGGGCTGAACGGGGGGTTCGTGCACACAGCCCAGCTTGGAGCGAACGACCT
ACACCGAACTGAGATACCTACAGCGTGAGCTATGAGAAAGCGCCACGCTTCCCGAA
GGGAGAAAGGCGGACAGGTATCCGGTAAGCGGCAGGGTCGGAACAGGAGAGCGC
ACGAGGGAGCTTCCAGGGGGAAACGCCTGGTATCTTTATAGTCCTGTCGGGTTTCG
CCACCTCTGACTTGAGCGTCGATTTTTGTGATGCTCGTCAGGGGGGCGGAGCCTATG
GAAAAACGCCAGCAACGCGGCCTTTTTACGGTTCCTGGCCTTTTGCTGGCCTTTTGC
TCACATGTTCTTTCCTGCGTTATCCCCTGATTCTGTGGATAACCGTATTACCGCCTTTG
AGTGAGCTGATACCGCTCGCCGCAGCCGAACGACCGAGCGCAGCGAGTCAGTGAG
CGAGGAAGCGGAAGAGCGCCCAATACGCAAACCGCCTCTCCCCGCGCGTTGGCCG
ATTCATTAATGCAGCTGGCAC
SEQ ID NO: 2 (nucleotide sequence of RC6 plasmid): 
AGGGTTGAGTGTTGTTCCAGTTTGGAACAAGAGTCCACTATTAAAGAACGTGG
ACTCCAACGTCAAAGGGCGAAAAACCGTCTATCAGGGCGATGGCCCACTACGTGA
ACCATCACCCTAATCAAGTTTTTTGGGGTCGAGGTGCCGTAAAGCACTAAATCGGA
ACCCTAAAGGGAGCCCCCGATTTAGAGCTTGACGGGGAAAGCCGGCGAACGTGGC
GAGAAAGGAAGGGAAGAAAGCGAAAGGAGCGGGCGCTAGGGCGCTGGCAAGTGT
AGCGGTCACGCTGCGCGTAACCACCACACCCGCCGCGCTTAATGCGCCGCTACAGG
GCGCGTCCATTCGCCATTCAGGCTGCGCAACTGTTGGGAAGGGCGATCGGTGCGGG
CCTCTTCGCTATTACGCCAGCTGGCGAAAGGGGGATGTGCTGCAAGGCGATTAAGT
TGGGTAACGCCAGGGTTTTCCCAGTCACGACGTTGTAAAACGACGGCCAGTGAATT
GTAATACGACTCACTATAGGGCGAATTCGAGCTCGGTACCCCTAGAGGTCCTGTATT
AGAGGTCACGTGAGTGTTTTGCGACATTTTGCGACACCATGTGGTCACGCTGGGTAT
TTAAGCCCGAGTGAGCACGCAGGGTCTCCATTTTGAAGCGGGAGGTTTGAACGCGC
AGCCACCACGCCGGGGTTTTACGAGATTGTGATTAAGGTCCCCAGCGACCTTGACG
AGCATCTGCCCGGCATTTCTGACAGCTTTGTGAACTGGGTGGCCGAGAAGGAATGG
GAGTTGCCGCCAGATTCTGACATGGATCTGAATCTGATTGAGCAGGCACCCCTGAC
CGTGGCCGAGAAGCTGCAGCGCGACTTTCTGACGGAATGGCGCCGTGTGAGTAAG
GCCCCGGAGGCCCTTTTCTTTGTGCAATTTGAGAAGGGAGAGAGCTACTTCCACAT
GCACGTGCTCGTGGAAACCACCGGGGTGAAATCCATGGTTTTGGGACGTTTCCTGA
GTCAGATTCGCGAAAAACTGATTCAGAGAATTTACCGCGGGATCGAGCCGACTTTG
CCAAACTGGTTCGCGGTCACAAAGACCAGAAATGGCGCCGGAGGCGGGAACAAG
GTGGTGGATGAGTGCTACATCCCCAATTACTTGCTCCCCAAAACCCAGCCTGAGCTC
CAGTGGGCGTGGACTAATATGGAACAGTATTTAAGCGCCTGTTTGAATCTCACGGAG
CGTAAACGGTTGGTGGCGCAGCATCTGACGCACGTGTCGCAGACGCAGGAGCAGA
ACAAAGAGAATCAGAATCCCAATTCTGATGCGCCGGTGATCAGATCAAAAACTTCA
GCCAGGTACATGGAGCTGGTCGGGTGGCTCGTGGACAAGGGGATTACCTCGGAGA
AGCAGTGGATCCAGGAGGACCAGGCCTCATACATCTCCTTCAATGCGGCCTCCAAC
TCGCGGTCCCAAATCAAGGCTGCCTTGGACAATGCGGGAAAGATTATGAGCCTGAC
TAAAACCGCCCCCGACTACCTGGTGGGCCAGCAGCCCGTGGAGGACATTTCCAGCA
ATCGGATTTATAAAATTTTGGAACTAAACGGGTACGATCCCCAATATGCGGCTTCCGT
CTTTCTGGGATGGGCCACGAAAAAGTTCGGCAAGAGGAACACCATCTGGCTGTTTG
GGCCTGCAACTACCGGGAAGACCAACATCGCGGAGGCCATAGCCCACACTGTGCCC
TTCTACGGGTGCGTAAACTGGACCAATGAGAACTTTCCCTTCAACGACTGTGTCGA
CAAGATGGTGATCTGGTGGGAGGAGGGCAAGATGACGGCCAAGGTCGTGGAGTCC
GCCAAGGCCATTCTCGGCGGCAGCAAGGTGCGCGTGGACCAAAAGTGCAAGTCGT
 
CCGCCCAGATCGATCCCACCCCCGTGATCGTCACCTCCAACACCAACATGTGCGCC
GTGATTGACGGGAACAGCACCACCTTCGAGCACCAGCAGCCGTTGCAGGACCGGA
TGTTCAAATTTGAACTCACCCGCCGTCTGGAGCATGACTTTGGCAAGGTGACAAAG
CAGGAAGTCAAAGAGTTCTTCCGCTGGGCGCAGGATCACGTGACCGAGGTGGCGC
ATGAGTTCTACGTCAGAAAGGGTGGAGCCAACAAGAGACCCGCCCCCGATGACGC
GGATAAAAGCGAGCCCAAGCGGGCCTGCCCCTCAGTCGCGGATCCATCGACGTCAG
ACGCGGAAGGAGCTCCGGTGGACTTTGCCGACAGGTACCAAAACAAATGTTCTCGT
CACGCGGGCATGCTTCAGATGCTGTTTCCCTGCAAAACATGCGAGAGAATGAATCA
GAATTTCAACATTTGCTTCACGCACGGGACCAGAGACTGTTCAGAATGTTTCCCCG
GCGTGTCAGAATCTCAACCGGTCGTCAGAAAGAGGACGTATCGGAAACTCTGTGCC
ATTCATCATCTGCTGGGGCGGGCTCCCGAGATTGCTTGCTCGGCCTGCGATCTGGTC
AACGTGGATCTGGATGACTGTGTTTCTGAGCAATAAATGACTTAAACCAGGTATGGC
TGCCGATGGTTATCTTCCAGATTGGCTCGAGGACAACCTCTCTGAGGGCATTCGCGA
GTGGTGGGACTTGAAACCTGGAGCCCCGAAACCCAAAGCCAACCAGCAAAAGCA
GGACGACGGCCGGGGTCTGGTGCTTCCTGGCTACAAGTACCTCGGACCCTTCAACG
GACTCGACAAGGGGGAGCCCGTCAACGCGGCGGATGCAGCGGCCCTCGAGCACGA
CAAGGCCTACGACCAGCAGCTCAAAGCGGGTGACAATCCGTACCTGCGGTATAACC
ACGCCGACGCCGAGTTTCAGGAGCGTCTGCAAGAAGATACGTCTTTTGGGGGCAAC
CTCGGGCGAGCAGTCTTCCAGGCCAAGAAGAGGGTTCTCGAACCTTTTGGTCTGGT
TGAGGAAGGTGCTAAGACGGCTCCTGGAAAGAAACGTCCGGTAGAGCAGTCGCCA
CAAGAGCCAGACTCCTCCTCGGGCATTGGCAAGACAGGCCAGCAGCCCGCTAAAA
AGAGACTCAATTTTGGTCAGACTGGCGACTCAGAGTCAGTCCCCGACCCACAACCT
CTCGGAGAACCTCCAGCAACCCCCGCTGCTGTGGGACCTACTACAATGGCTTCAGG
CGGTGGCGCACCAATGGCAGACAATAACGAAGGCGCCGACGGAGTGGGTAATGCC
TCAGGAAATTGGCATTGCGATTCCACATGGCTGGGCGACAGAGTCATCACCACCAG
CACCCGAACATGGGCCTTGCCCACCTATAACAACCACCTCTACAAGCAAATCTCCA
GTGCTTCAACGGGGGCCAGCAACGACAACCACTACTTCGGCTACAGCACCCCCTGG
GGGTATTTTGATTTCAACAGATTCCACTGCCATTTCTCACCACGTGACTGGCAGCGA
CTCATCAACAACAATTGGGGATTCCGGCCCAAGAGACTCAACTTCAAGCTCTTCAA
CATCCAAGTCAAGGAGGTCACGACGAATGATGGCGTCACGACCATCGCTAATAACC
TTACCAGCACGGTTCAAGTCTTCTCGGACTCGGAGTACCAGTTGCCGTACGTCCTCG
GCTCTGCGCACCAGGGCTGCCTCCCTCCGTTCCCGGCGGACGTGTTCATGATTCCGC
AGTACGGCTACCTAACGCTCAACAATGGCAGCCAGGCAGTGGGACGGTCATCCTTT
TACTGCCTGGAATATTTCCCATCGCAGATGCTGAGAACGGGCAATAACTTTACCTTC
AGCTACACCTTCGAGGACGTGCCTTTCCACAGCAGCTACGCGCACAGCCAGAGCCT
 
GGACCGGCTGATGAATCCTCTCATCGACCAGTACCTGTATTACCTGAACAGAACTCA
GAATCAGTCCGGAAGTGCCCAAAACAAGGACTTGCTGTTTAGCCGGGGGTCTCCAG
CTGGCATGTCTGTTCAGCCCAAAAACTGGCTACCTGGACCCTGTTACCGGCAGCAG
CGCGTTTCTAAAACAAAAACAGACAACAACAACAGCAACTTTACCTGGACTGGTG
CTTCAAAATATAACCTTAATGGGCGTGAATCTATAATCAACCCTGGCACTGCTATGGC
CTCACACAAAGACGACAAAGACAAGTTCTTTCCCATGAGCGGTGTCATGATTTTTG
GAAAGGAGAGCGCCGGAGCTTCAAACACTGCATTGGACAATGTCATGATCACAGA
CGAAGAGGAAATCAAAGCCACTAACCCCGTGGCCACCGAAAGATTTGGGACTGTG
GCAGTCAATCTCCAGAGCAGCAGCACAGACCCTGCGACCGGAGATGTGCATGTTAT
GGGAGCCTTACCTGGAATGGTGTGGCAAGACAGAGACGTATACCTGCAGGGTCCTA
TTTGGGCCAAAATTCCTCACACGGATGGACACTTTCACCCGTCTCCTCTCATGGGCG
GCTTTGGACTTAAGCACCCGCCTCCTCAGATCCTCATCAAAAACACGCCTGTTCCTG
CGAATCCTCCGGCAGAGTTTTCGGCTACAAAGTTTGCTTCATTCATCACCCAGTATT
CCACAGGACAAGTGAGCGTGGAGATTGAATGGGAGCTGCAGAAAGAAAACAGCA
AACGCTGGAATCCCGAAGTGCAGTATACATCTAACTATGCAAAATCTGCCAACGTTG
ATTTCACTGTGGACAACAATGGACTTTATACTGAGCCTCGCCCCATTGGCACCCGTT
ACCTCACCCGTCCCCTGTAATTGTCTGTTAATCAATAAACCGGTTGATTCGTTTCAGT
TGAACTTTGGTCTCGCGGCCGCTCGATAAGCTTTTGTTCCCTTTAGTGAGGGTTAAT
TTCGAGCTTGGCGTAATCATGGTCATAGCTGTTTCCTGTGTGAAATTGTTATCCGCTC
ACAATTCCACACAACATACGAGCCGGAAGCATAAAGTGTAAAGCCTGGGGTGCCTA
ATGAGTGAGCTAACTCACATTAATTGCGTTGCGCTCACTGCCCGCTTTCCAGTCGGG
AAACCTGTCGTGCCAGCTGCATTAATGAATCGGCCAACGCGCGGGGAGAGGCGGTT
TGCGTATTGGGCGCTCTTCCGCTTCCTCGCTCACTGACTCGCTGCGCTCGGTCGTTC
GGCTGCGGCGAGCGGTATCAGCTCACTCAAAGGCGGTAATACGGTTATCCACAGAA
TCAGGGGATAACGCAGGAAAGAACATGTGAGCAAAAGGCCAGCAAAAGGCCAGG
AACCGTAAAAAGGCCGCGTTGCTGGCGTTTTTCCATAGGCTCCGCCCCCCTGACGA
GCATCACAAAAATCGACGCTCAAGTCAGAGGTGGCGAAACCCGACAGGACTATAA
AGATACCAGGCGTTTCCCCCTGGAAGCTCCCTCGTGCGCTCTCCTGTTCCGACCCTG
CCGCTTACCGGATACCTGTCCGCCTTTCTCCCTTCGGGAAGCGTGGCGCTTTCTCAT
AGCTCACGCTGTAGGTATCTCAGTTCGGTGTAGGTCGTTCGCTCCAAGCTGGGCTGT
GTGCACGAACCCCCCGTTCAGCCCGACCGCTGCGCCTTATCCGGTAACTATCGTCTT
GAGTCCAACCCGGTAAGACACGACTTATCGCCACTGGCAGCAGCCACTGGTAACAG
GATTAGCAGAGCGAGGTATGTAGGCGGTGCTACAGAGTTCTTGAAGTGGTGGCCTA
ACTACGGCTACACTAGAAGAACAGTATTTGGTATCTGCGCTCTGCTGAAGCCAGTTA
CCTTCGGAAAAAGAGTTGGTAGCTCTTGATCCGGCAAACAAACCACCGCTGGTAGC
 
GGTGGTTTTTTTGTTTGCAAGCAGCAGATTACGCGCAGAAAAAAAGGATCTCAAGA
AGATCCTTTGATCTTTTCTACGGGGTCTGACGCTCAGTGGAACGAAAACTCACGTTA
AGGGATTTTGGTCATGAGATTATCAAAAAGGATCTTCACCTAGATCCTTTTAAATTAA
AAATGAAGTTTTAAATCAATCTAAAGTATATATGAGTAAACTTGGTCTGACAGTTACC
AATGCTTAATCAGTGAGGCACCTATCTCAGCGATCTGTCTATTTCGTTCATCCATAGT
TGCCTGACTCCCCGTCGTGTAGATAACTACGATACGGGAGGGCTTACCATCTGGCCC
CAGTGCTGCAATGATACCGCGAGACCCACGCTCACCGGCTCCAGATTTATCAGCAAT
AAACCAGCCAGCCGGAAGGGCCGAGCGCAGAAGTGGTCCTGCAACTTTATCCGCC
TCCATCCAGTCTATTAATTGTTGCCGGGAAGCTAGAGTAAGTAGTTCGCCAGTTAAT
AGTTTGCGCAACGTTGTTGCCATTGCTACAGGCATCGTGGTGTCACGCTCGTCGTTT
GGTATGGCTTCATTCAGCTCCGGTTCCCAACGATCAAGGCGAGTTACATGATCCCCC
ATGTTGTGCAAAAAAGCGGTTAGCTCCTTCGGTCCTCCGATCGTTGTCAGAAGTAA
GTTGGCCGCAGTGTTATCACTCATGGTTATGGCAGCACTGCATAATTCTCTTACTGTC
ATGCCATCCGTAAGATGCTTTTCTGTGACTGGTGAGTACTCAACCAAGTCATTCTGA
GAATAGTGTATGCGGCGACCGAGTTGCTCTTGCCCGGCGTCAATACGGGATAATACC
GCGCCACATAGCAGAACTTTAAAAGTGCTCATCATTGGAAAACGTTCTTCGGGGCG
AAAACTCTCAAGGATCTTACCGCTGTTGAGATCCAGTTCGATGTAACCCACTCGTGC
ACCCAACTGATCTTCAGCATCTTTTACTTTCACCAGCGTTTCTGGGTGAGCAAAAAC
AGGAAGGCAAAATGCCGCAAAAAAGGGAATAAGGGCGACACGGAAATGTTGAATA
CTCATACTCTTCCTTTTTCAATATTATTGAAGCATTTATCAGGGTTATTGTCTCATGAG
CGGATACATATTTGAATGTATTTAGAAAAATAAACAAATAGGGGTTCCGCGCACATTT
CCCCGAAAAGTGCCACCTGACGTCTAAGAAACCATTATTATCATGACATTAACCTAT
AAAAATAGGCGTATCACGAGGCCCTTTCGTCTCGCGCGTTTCGGTGATGACGGTGA
AAACCTCTGACACATGCAGCTCCCGGAGACGGTCACAGCTTGTCTGTAAGCGGATG
CCGGGAGCAGACAAGCCCGTCAGGGCGCGTCAGCGGGTGTTGGCGGGTGTCGGGG
CTGGCTTAACTATGCGGCATCAGAGCAGATTGTACTGAGAGTGCACCATATGCGGTG
TGAAATACCGCACAGATGCGTAAGGAGAAAATACCGCATCAGGAAATTGTAAGCGT
TAATATTTTGTTAAAATTCGCGTTAAATTTTTGTTAAATCAGCTCATTTTTTAACCAAT
AGGCCGAAATCGGCAAAATCCCTTATAAATCAAAAGAATAGACCGAGAT
SEQ ID NO: 3 (nucleotide sequence of RC9 plasmid): 
CTGTTGGGAAGGGCGATCGGTGCGGGCCTCTTCGCTATTACGCCAGCTGGCGA
AAGGGGGATGTGCTGCAAGGCGATTAAGTTGGGTAACGCCAGGGTTTTCCCAGTCA
CGACGTTGTAAAACGACGGCCAGTGAGCGCGCGTAATACGACTCACTATAGGGCGA
ATTGGGTACCGGGCCCCCCCTCGATCGAGGTCGACGGTATCGGGGGAGCTCGCAGG
GTCTCCATTTTGAAGCGGGAGGTTTGAACGCGCAGCCGCCATGCCGGGGTTTTACG
AGATTGTGATTAAGGTCCCCAGCGACCTTGACGAGCATCTGCCCGGCATTTCTGACA
GCTTTGTGAACTGGGTGGCCGAGAAGGAATGGGAGTTGCCGCCAGATTCTGACATG
GATCTGAATCTGATTGAGCAGGCACCCCTGACCGTGGCCGAGAAGCTGCAGCGCGA
CTTTCTGACGGAATGGCGCCGTGTGAGTAAGGCCCCGGAGGCTCTTTTCTTTGTGC
AATTTGAGAAGGGAGAGAGCTACTTCCACATGCACGTGCTCGTGGAAACCACCGG
GGTGAAATCCATGGTTTTGGGACGTTTCCTGAGTCAGATTCGCGAAAAACTGATTC
AGAGAATTTACCGCGGGATCGAGCCGACTTTGCCAAACTGGTTCGCGGTCACAAAG
ACCAGAAATGGCGCCGGAGGCGGGAACAAGGTGGTGGATGAGTGCTACATCCCCA
ATTACTTGCTCCCCAAAACCCAGCCTGAGCTCCAGTGGGCGTGGACTAATATGGAA
CAGTATTTAAGCGCCTGTTTGAATCTCACGGAGCGTAAACGGTTGGTGGCGCAGCAT
CTGACGCACGTGTCGCAGACGCAGGAGCAGAACAAAGAGAATCAGAATCCCAATT
CTGATGCGCCGGTGATCAGATCAAAAACTTCAGCCAGGTACATGGAGCTGGTCGGG
TGGCTCGTGGACAAGGGGATTACCTCGGAGAAGCAGTGGATCCAGGAGGACCAGG
CCTCATACATCTCCTTCAATGCGGCCTCCAACTCGCGGTCCCAAATCAAGGCTGCCT
TGGACAATGCGGGAAAGATTATGAGCCTGACTAAAACCGCCCCCGACTACCTGGTG
GGCCAGCAGCCCGTGGAGGACATTTCCAGCAATCGGATTTATAAAATTTTGGAACTA
AACGGGTACGATCCCCAATATGCGGCTTCCGTCTTTCTGGGATGGGCCACGAAAAA
GTTCGGCAAGAGGAACACCATCTGGCTGTTTGGGCCTGCAACTACCGGGAAGACC
AACATCGCGGAGGCCATAGCCCACACTGTGCCCTTCTACGGGTGCGTAAACTGGAC
CAATGAGAACTTTCCCTTCAACGACTGTGTCGACAAGATGGTGATCTGGTGGGAGG
AGGGGAAGATGACCGCCAAGGTCGTGGAGTCGGCCAAAGCCATTCTCGGAGGAAG
CAAGGTGCGCGTGGACCAGAAATGCAAGTCCTCGGCCCAGATAGACCCGACTCCC
GTGATCGTCACCTCCAACACCAACATGTGCGCCGTGATTGACGGGAACTCAACGAC
CTTCGAACACCAGCAGCCGTTGCAAGACCGGATGTTCAAATTTGAACTCACCCGCC
GTCTGGATCATGACTTTGGGAAGGTCACCAAGCAGGAAGTCAAAGACTTTTTCCGG
TGGGCAAAGGATCACGTGGTTGAGGTGGAGCATGAATTCTACGTCAAAAAGGGTG
GAGCCAAGAAAAGACCCGCCCCCAGTGACGCAGATATAAGTGAGCCCAAACGGGT
GCGCGAGTCAGTTGCGCAGCCATCGACGTCAGACGCGGAAGCTTCGATCAACTACG
CGGACAGGTACCAAAACAAATGTTCTCGTCACGTGGGCATGAATCTGATGCTGTTTC
 
CCTGCAGACAATGCGAGAGACTGAATCAGAATTCAAATATCTGCTTCACTCACGGT
GTCAAAGACTGTTTAGAGTGCTTTCCCGTGTCAGAATCTCAACCCGTTTCTGTCGTC
AAAAAGGCGTATCAGAAACTGTGCTACATTCATCACATCATGGGAAAGGTGCCAGA
CGCTTGCACTGCTTGCGACCTGGTCAATGTGGACTTGGATGACTGTGTTTCTGAACA
ATAAATGACTTAAACCAGGTATGGCTGCCGATGGTTATCTTCCAGATTGGCTCGAGG
ACAACCTTAGTGAAGGAATTCGCGAGTGGTGGGCTTTGAAACCTGGAGCCCCTCAA
CCCAAGGCAAATCAACAACATCAAGACAACGCTCGAGGTCTTGTGCTTCCGGGTTA
CAAATACCTTGGACCCGGCAACGGACTCGACAAGGGGGAGCCGGTCAACGCAGCA
GACGCGGCGGCCCTCGAGCACGACAAGGCCTACGACCAGCAGCTCAAGGCCGGAG
ACAACCCGTACCTCAAGTACAACCACGCCGACGCCGAGTTCCAGGAGCGGCTCAA
AGAAGATACGTCTTTTGGGGGCAACCTCGGGCGAGCAGTCTTCCAGGCCAAAAAG
AGGCTTCTTGAACCTCTTGGTCTGGTTGAGGAAGCGGCTAAGACGGCTCCTGGAAA
GAAGAGGCCTGTAGAGCAGTCTCCTCAGGAACCGGACTCCTCCGCGGGTATTGGCA
AATCGGGTGCACAGCCCGCTAAAAAGAGACTCAATTTCGGTCAGACTGGCGACAC
AGAGTCAGTCCCAGACCCTCAACCAATCGGAGAACCTCCCGCAGCCCCCTCAGGT
GTGGGATCTCTTACAATGGCTTCAGGTGGTGGCGCACCAGTGGCAGACAATAACGA
AGGTGCCGATGGAGTGGGTAGTTCCTCGGGAAATTGGCATTGCGATTCCCAATGGCT
GGGGGACAGAGTCATCACCACCAGCACCCGAACCTGGGCCCTGCCCACCTACAAC
AATCACCTCTACAAGCAAATCTCCAACAGCACATCTGGAGGATCTTCAAATGACAA
CGCCTACTTCGGCTACAGCACCCCCTGGGGGTATTTTGACTTCAACAGATTCCACTG
CCACTTCTCACCACGTGACTGGCAGCGACTCATCAACAACAACTGGGGATTCCGGC
CTAAGCGACTCAACTTCAAGCTCTTCAACATTCAGGTCAAAGAGGTTACGGACAAC
AATGGAGTCAAGACCATCGCCAATAACCTTACCAGCACGGTCCAGGTCTTCACGGA
CTCAGACTATCAGCTCCCGTACGTGCTCGGGTCGGCTCACGAGGGCTGCCTCCCGC
CGTTCCCAGCGGACGTTTTCATGATTCCTCAGTACGGGTATCTGACGCTTAATGATG
GAAGCCAGGCCGTGGGTCGTTCGTCCTTTTACTGCCTGGAATATTTCCCGTCGCAAA
TGCTAAGAACGGGTAACAACTTCCAGTTCAGCTACGAGTTTGAGAACGTACCTTTC
CATAGCAGCTACGCTCACAGCCAAAGCCTGGACCGACTAATGAATCCACTCATCGA
CCAATACTTGTACTATCTCTCAAAGACTATTAACGGTTCTGGACAGAATCAACAAAC
GCTAAAATTCAGTGTGGCCGGACCCAGCAACATGGCTGTCCAGGGAAGAAACTACA
TACCTGGACCCAGCTACCGACAACAACGTGTCTCAACCACTGTGACTCAAAACAAC
AACAGCGAATTTGCTTGGCCTGGAGCTTCTTCTTGGGCTCTCAATGGACGTAATAGC
TTGATGAATCCTGGACCTGCTATGGCCAGCCACAAAGAAGGAGAGGACCGTTTCTT
TCCTTTGTCTGGATCTTTAATTTTTGGCAAACAAGGAACTGGAAGAGACAACGTGG
ATGCGGACAAAGTCATGATAACCAACGAAGAAGAAATTAAAACTACTAACCCGGTA
 
GCAACGGAGTCCTATGGACAAGTGGCCACAAACCACCAGAGTGCCCAAGCACAGG
CGCAGACCGGCTGGGTTCAAAACCAAGGAATACTTCCGGGTATGGTTTGGCAGGAC
AGAGATGTGTACCTGCAAGGACCCATTTGGGCCAAAATTCCTCACACGGACGGCAA
CTTTCACCCTTCTCCGCTGATGGGAGGGTTTGGAATGAAGCACCCGCCTCCTCAGAT
CCTCATCAAAAACACACCTGTACCTGCGGATCCTCCAACGGCCTTCAACAAGGACA
AGCTGAACTCTTTCATCACCCAGTATTCTACTGGCCAAGTCAGCGTGGAGATCGAGT
GGGAGCTGCAGAAGGAAAACAGCAAGCGCTGGAACCCGGAGATCCAGTACACTTC
CAACTATTACAAGTCTAATAATGTTGAATTTGCTGTTAATACTGAAGGTGTATATAGT
GAACCCCGCCCCATTGGCACCAGATACCTGACTCGTAATCTGTAATTGCTTGTTAATC
AATAAACCGTTTAATTCGTTTCAGTTGAACTTTGGTCTCTGCGAAGGGCGAATTCGT
TTAAACCTGCAGGACTAGAGGTCCTGTATTAGAGGTCACGTGAGTGTTTTGCGACAT
TTTGCGACACCATGTGGTCACGCTGGGTATTTAAGCCCGAGTGAGCACGCAGGGTC
TCCATTTTGAAGCGGGAGGTTTGAACGCGCAGCCGCCAAGCCGAATTCTGCAGATA
TCCATCACACTGGCGGCCGCTCGACTAGAGCGGCCGCCACCGCGGTGGAGCTCCAG
CTTTTGTTCCCTTTAGTGAGGGTTAATTGCGCGCTTGGCGTAATCATGGTCATAGCTG
TTTCCTGTGTGAAATTGTTATCCGCTCACAATTCCACACAACATACGAGCCGGAAGC
ATAAAGTGTAAAGCCTGGGGTGCCTAATGAGTGAGCTAACTCACATTAATTGCGTTG
CGCTCACTGCCCGCTTTCCAGTCGGGAAACCTGTCGTGCCAGCTGCATTAATGAATC
GGCCAACGCGCGGGGAGAGGCGGTTTGCGTATTGGGCGCTCTTCCGCTTCCTCGCT
CACTGACTCGCTGCGCTCGGTCGTTCGGCTGCGGCGAGCGGTATCAGCTCACTCAA
AGGCGGTAATACGGTTATCCACAGAATCAGGGGATAACGCAGGAAAGAACATGTGA
GCAAAAGGCCAGCAAAAGGCCAGGAACCGTAAAAAGGCCGCGTTGCTGGCGTTTT
TCCATAGGCTCCGCCCCCCTGACGAGCATCACAAAAATCGACGCTCAAGTCAGAGG
TGGCGAAACCCGACAGGACTATAAAGATACCAGGCGTTTCCCCCTGGAAGCTCCCT
CGTGCGCTCTCCTGTTCCGACCCTGCCGCTTACCGGATACCTGTCCGCCTTTCTCCC
TTCGGGAAGCGTGGCGCTTTCTCATAGCTCACGCTGTAGGTATCTCAGTTCGGTGTA
GGTCGTTCGCTCCAAGCTGGGCTGTGTGCACGAACCCCCCGTTCAGCCCGACCGCT
GCGCCTTATCCGGTAACTATCGTCTTGAGTCCAACCCGGTAAGACACGACTTATCGC
CACTGGCAGCAGCCACTGGTAACAGGATTAGCAGAGCGAGGTATGTAGGCGGTGCT
ACAGAGTTCTTGAAGTGGTGGCCTAACTACGGCTACACTAGAAGAACAGTATTTGG
TATCTGCGCTCTGCTGAAGCCAGTTACCTTCGGAAAAAGAGTTGGTAGCTCTTGATC
CGGCAAACAAACCACCGCTGGTAGCGGTGGTTTTTTTGTTTGCAAGCAGCAGATTA
CGCGCAGAAAAAAAGGATCTCAAGAAGATCCTTTGATCTTTTCTACGGGGTCTGAC
GCTCAGTGGAACGAAAACTCACGTTAAGGGATTTTGGTCATGAGATTATCAAAAAG
GATCTTCACCTAGATCCTTTTAAATTAAAAATGAAGTTTTAAATCAATCTAAAGTATAT
 
ATGAGTAAACTTGGTCTGACAGTTACCAATGCTTAATCAGTGAGGCACCTATCTCAG
CGATCTGTCTATTTCGTTCATCCATAGTTGCCTGACTCCCCGTCGTGTAGATAACTAC
GATACGGGAGGGCTTACCATCTGGCCCCAGTGCTGCAATGATACCGCGAGACCCAC
GCTCACCGGCTCCAGATTTATCAGCAATAAACCAGCCAGCCGGAAGGGCCGAGCGC
AGAAGTGGTCCTGCAACTTTATCCGCCTCCATCCAGTCTATTAATTGTTGCCGGGAA
GCTAGAGTAAGTAGTTCGCCAGTTAATAGTTTGCGCAACGTTGTTGCCATTGCTACA
GGCATCGTGGTGTCACGCTCGTCGTTTGGTATGGCTTCATTCAGCTCCGGTTCCCAA
CGATCAAGGCGAGTTACATGATCCCCCATGTTGTGCAAAAAAGCGGTTAGCTCCTTC
GGTCCTCCGATCGTTGTCAGAAGTAAGTTGGCCGCAGTGTTATCACTCATGGTTATG
GCAGCACTGCATAATTCTCTTACTGTCATGCCATCCGTAAGATGCTTTTCTGTGACTG
GTGAGTACTCAACCAAGTCATTCTGAGAATAGTGTATGCGGCGACCGAGTTGCTCTT
GCCCGGCGTCAATACGGGATAATACCGCGCCACATAGCAGAACTTTAAAAGTGCTCA
TCATTGGAAAACGTTCTTCGGGGCGAAAACTCTCAAGGATCTTACCGCTGTTGAGA
TCCAGTTCGATGTAACCCACTCGTGCACCCAACTGATCTTCAGCATCTTTTACTTTCA
CCAGCGTTTCTGGGTGAGCAAAAACAGGAAGGCAAAATGCCGCAAAAAAGGGAAT
AAGGGCGACACGGAAATGTTGAATACTCATACTCTTCCTTTTTCAATATTATTGAAGC
ATTTATCAGGGTTATTGTCTCATGAGCGGATACATATTTGAATGTATTTAGAAAAATAA
ACAAATAGGGGTTCCGCGCACATTTCCCCGAAAAGTGCCACCTAAATTGTAAGCGTT
AATATTTTGTTAAAATTCGCGTTAAATTTTTGTTAAATCAGCTCATTTTTTAACCAATA
GGCCGAAATCGGCAAAATCCCTTATAAATCAAAAGAATAGACCGAGATAGGGTTGA
GTGTTGTTCCAGTTTGGAACAAGAGTCCACTATTAAAGAACGTGGACTCCAACGTC
AAAGGGCGAAAAACCGTCTATCAGGGCGATGGCCCACTACGTGAACCATCACCCTA
ATCAAGTTTTTTGGGGTCGAGGTGCCGTAAAGCACTAAATCGGAACCCTAAAGGGA
GCCCCCGATTTAGAGCTTGACGGGGAAAGCCGGCGAACGTGGCGAGAAAGGAAGG
GAAGAAAGCGAAAGGAGCGGGCGCTAGGGCGCTGGCAAGTGTAGCGGTCACGCT
GCGCGTAACCACCACACCCGCCGCGCTTAATGCGCCGCTACAGGGCGCGTCCCATT
CGCCATTCAGGCTGCGCAA
SEQ ID NO: 4 (nucleotide sequence corresponding to short peptide GRGDSP):
   GGTCGTGGCGACAGCCCG
SEQ ID NO: 5 (AAV2 VP1 gene sequence) AGTTTCAGGAGCGCCTTAAAGAAGATACGTCTTTTGGGGGCAACCTCGGACGA 
GCAGTCTTCCAGGCGAAAAAGAGGGTTCTTGAACCTCTGGGCCTGGTTGAGGAAC
CTGTTAAGACGGCTCCGGGAAAAAAGAGGCCGGTAGAGCACTCTCCTGTGGAGCC
AGACTCCTCCTCGGGAACCGGAAAGGCGGGCCAGCAGCCTGCAAGAAAAAGATTG
AATTTTGGTCAGACTGGAGACGCAGACTCAGTACCTGACCCCCAGCCTCTCGGACA
GCCACCAGCAGCCCCCTCTGGTCTGGGAACTAATACGATGGCTACAGGCAGTGGCG
CACCAATGGCAGACAATAACGAGGGCGCCGACGGAGTGGGTAATTCCTCGGGAAAT
TGGCATTGCGATTCCACATGGATGGGCGACAGAGTCATCACCACCAGCACCCGAAC
CTGGGCCCTGCCCACCTACAACAACCACCTCTACAAACAAATTTCCAGCCAATCAG
GAGCCTCGAACGACAATCACTACTTTGGCTACAGCACCCCTTGGGGGTATTTTGACT
TCAACAGATTCCACTGCCACTTTTCACCACGTGACTGGCAAAGACTCATCAACAAC
AACTGGGGATTCCGACCCAAGAGACTCAACTTCAAGCTCTTTAACATTCAAGTCAA
AGAGGTCACGCAGAATGACGGTACGACGACGATTGCCAATAACCTTACCAGCACGG
TTCAGGTGTTTACTGACTCGGAGTACCAGCTCCCGTACGTCCTCGGCTCGGCGCATC
AAGGATGCCTCCCGCCGTTCCCAGCAGACGTCTTCATGGTGCCACAGTATGGATACC
TCACCCTGAACAACGGGAGTCAGGCAGTAGGACGCTCTTCATTTTACTGCCTGGAG
TACTTTCCTTCTCAGATGCTGCGTACCGGAAACAACTTTACCTTCAGCTACACTTTT
GAGGACGTTCCTTTCCACAGCAGCTACGCTCACAGCCAGAGTCTGGACCGTCTCAT
GAATCCTCTCATCGACCAGTACCTGTATTACTTGAGCAGAACAAACACTCCAAGTGG
AACCACCACGCAGTCAAGGCTTCAGTTTTCTCAGGCCGGAGCGAGTGACATTCGGG
ACCAGTCTAGGAACTGGCTTCCTGGACCCTGTTACCGCCAGCAGCGAGTATCAAAG
ACATCTGCGGATAACAACAACAGTGAATACTCGTGGACTGGAGCTACCAAGTACCA
CCTCAATGGCAGAGACTCTCTGGTGAATCCGGGCCCGGCCATGGCAAGCCACAAGG
ACGATGAAGAAAAGTTTTTTCCTCAGAGCGGGGTTCTCATCTTTGGGAAGCAAGGC
TCAGAGAAAACAAATGTGGACATTGAAAAGGTCATGATTACAGACGAAGAGGAAA
TCAGGACAACCAATCCCGTGGCTACGGAGCAGTATGGTTCTGTATCTACCAACCTCC
AGAGAGGCAACAGACAAGCAGCTACCGCAGATGTCAACACACAAGGCGTTCTTCC
AGGCATGGTCTGGCAGGACAGAGATGTGTACCTTCAGGGGCCCATCTGGGCAAAGA
TTCCACACACGGACGGACATTTTCACCCCTCTCCCCTCATGGGTGGATTCGGACTTA
AACACCCTCCTCCACAGATTCTCATCAAGAACACCCCGGTACCTGCGAATCCTTCGA
CCACCTTCAGTGCGGCAAAGTTTGCTTCCTTCATCACACAGTACTCCACGGGACAG
GTCAGCGTGGAGATCGAGTGGGAGCTGCAGAAGGAAAACAGCAAACGCTGGAATC
CCGAAATTCAGTACACTTCCAACTACAACAAGTCTGTTAATGTGGACTTTACTGTGG
ACACTAATGGCGTGTATTCAGAGCCTCGCCCCATTGGCACCAGATACCTGACTCGTA
ATCTGTAA
SEQ ID NO: 6 (nucleotide sequence of RC2.2 plasmid): 
GACAGGTTTCCCGACTGGAAAGCGGGCAGTGAGCGCAACGCAATTAATGTGA
GTTAGCTCACTCATTAGGCACCCCAGGCTTTACACTTTATGCTTCCGGCTCGTATGTT
GTGTGGAATTGTGAGCGGATAACAATTTCACACAGGAAACAGCTATGACCATGATTA
CGCCAAGCGCGCCGATATCGTTAACGCCCCGCGCCGGCCGCTCTAGAACTAGTGGA
TCCCCCGGAAGATCAGAAGTTCCTATTCCGAAGTTCCTATTCTCTAGAAAGTATAGG
AACTTCTGATCTGCGCAGCCGCCATGCCGGGGTTTTACGAGATTGTGATTAAGGTCC
CCAGCGACCTTGACGAGCATCTGCCCGGCATTTCTGACAGCTTTGTGAACTGGGTG
GCCGAGAAGGAATGGGAGTTGCCGCCAGATTCTGACATGGATCTGAATCTGATTGA
GCAGGCACCCCTGACCGTGGCCGAGAAGCTGCAGCGCGACTTTCTGACGGAATGG
CGCCGTGTGAGTAAGGCCCCGGAGGCCCTTTTCTTTGTGCAATTTGAGAAGGGAGA
GAGCTACTTCCACATGCACGTGCTCGTGGAAACCACCGGGGTGAAATCCATGGTTT
TGGGACGTTTCCTGAGTCAGATTCGCGAAAAACTGATTCAGAGAATTTACCGCGGG
ATCGAGCCGACTTTGCCAAACTGGTTCGCGGTCACAAAGACCAGAAATGGCGCCG
GAGGCGGGAACAAGGTGGTGGATGAGTGCTACATCCCCAATTACTTGCTCCCCAAA
ACCCAGCCTGAGCTCCAGTGGGCGTGGACTAATATGGAACAGTATTTAAGCGCCTG
TTTGAATCTCACGGAGCGTAAACGGTTGGTGGCGCAGCATCTGACGCACGTGTCGC
AGACGCAGGAGCAGAACAAAGAGAATCAGAATCCCAATTCTGATGCGCCGGTGAT
CAGATCAAAAACTTCAGCCAGGTACATGGAGCTGGTCGGGTGGCTCGTGGACAAG
GGGATTACCTCGGAGAAGCAGTGGATCCAGGAGGACCAGGCCTCATACATCTCCTT
CAATGCGGCCTCCAACTCGCGGTCCCAAATCAAGGCTGCCTTGGACAATGCGGGAA
AGATTATGAGCCTGACTAAAACCGCCCCCGACTACCTGGTGGGCCAGCAGCCCGTG
GAGGACATTTCCAGCAATCGGATTTATAAAATTTTGGAACTAAACGGGTACGATCCC
CAATATGCGGCTTCCGTCTTTCTGGGATGGGCCACGAAAAAGTTCGGCAAGAGGAA
CACCATCTGGCTGTTTGGGCCTGCAACTACCGGGAAGACCAACATCGCGGAGGCCA
TAGCCCACACTGTGCCCTTCTACGGGTGCGTAAACTGGACCAATGAGAACTTTCCCT
TCAACGACTGTGTCGACAAGATGGTGATCTGGTGGGAGGAGGGGAAGATGACCGC
CAAGGTCGTGGAGTCGGCCAAAGCCATTCTCGGAGGAAGCAAGGTGCGCGTGGAC
CAGAAATGCAAGTCCTCGGCCCAGATAGACCCGACTCCCGTGATCGTCACCTCCAA
CACCAACATGTGCGCCGTGATTGACGGGAACTCAACGACCTTCGAACACCAGCAG
CCGTTGCAAGACCGGATGTTCAAATTTGAACTCACCCGCCGTCTGGATCATGACTTT
GGGAAGGTCACCAAGCAGGAAGTCAAAGACTTTTTCCGGTGGGCAAAGGATCACG
TGGTTGAGGTGGAGCATGAATTCTACGTCAAAAAGGGTGGAGCCAAGAAAAGACC
CGCCCCCAGTGACGCAGATATAAGTGAGCCCAAACGGGTGCGCGAGTCAGTTGCGC
AGCCATCGACGTCAGACGCGGAAGCTTCGATCAACTACGCAGACAGGTACCAAAA
 
CAAATGTTCTCGTCACGTGGGCATGAATCTGATGCTGTTTCCCTGCAGACAATGCGA
GAGAATGAATCAGAATTCAAATATCTGCTTCACTCACGGACAGAAAGACTGTTTAG
AGTGCTTTCCCGTGTCAGAATCTCAACCCGTTTCTGTCGTCAAAAAGGCGTATCAGA
AACTGTGCTACATTCATCATATCATGGGAAAGGTGCCAGACGCTTGCACTGCCTGCG
ATCTGGTCAATGTGGATTTGGATGACTGCATCTTTGAACAATAAATGATTTAAATCAG
GTATGGCTGCCGATGGTTATCTTCCAGATTGGCTCGAGGACACTCTCTCTGAAGGAA
TAAGACAGTGGTGGAAGCTCAAACCTGGCCCACCACCACCAAAGCCCGCAGAGCG
GCATAAGGACGACAGCAGGGGTCTTGTGCTTCCTGGGTACAAGTACCTCGGACCCT
TCAACGGACTCGACAAGGGAGAGCCGGTCAACGAGGCAGACGCCGCGGCCCTCG
AGCACGACAAAGCCTACGACCGGCAGCTCGACAGCGGAGACAACCCGTACCTCAA
GTACAACCACGCCGACGCGGAGTTTCAGGAGCGCCTTAAAGAAGATACGTCTTTTG
GGGGCAACCTCGGACGAGCAGTCTTCCAGGCGAAAAAGAGGGTTCTTGAACCTCT
GGGCCTGGTTGAGGAACCTGTTAAGACGGCTCCGGGAAAAAAGAGGCCGGTAGAG
CACTCTCCTGTGGAGCCAGACTCCTCCTCGGGAACCGGAAAGGCGGGCCAGCAGC
CTGCAAGAAAAAGATTGAATTTTGGTCAGACTGGAGACGCAGACTCAGTACCTGAC
CCCCAGCCTCTCGGACAGCCACCAGCAGCCCCCTCTGGTCTGGGAACTAATACGAT
GGCTACAGGCAGTGGCGCACCAATGGCAGACAATAACGAGGGCGCCGACGGAGTG
GGTAATTCCTCGGGAAATTGGCATTGCGATTCCACATGGATGGGCGACAGAGTCATC
ACCACCAGCACCCGAACCTGGGCCCTGCCCACCTACAACAACCACCTCTACAAACA
AATTTCCAGCCAATCAGGAGCCTCGAACGACAATCACTACTTTGGCTACAGCACCC
CTTGGGGGTATTTTGACTTCAACAGATTCCACTGCCACTTTTCACCACGTGACTGGC
AAAGACTCATCAACAACAACTGGGGATTCCGACCCAAGAGACTCAACTTCAAGCT
CTTTAACATTCAAGTCAAAGAGGTCACGCAGAATGACGGTACGACGACGATTGCCA
ATAACCTTACCAGCACGGTTCAGGTGTTTACTGACTCGGAGTACCAGCTCCCGTACG
TCCTCGGCTCGGCGCATCAAGGATGCCTCCCGCCGTTCCCAGCAGACGTCTTCATG
GTGCCACAGTATGGATACCTCACCCTGAACAACGGGAGTCAGGCAGTAGGACGCTC
TTCATTTTACTGCCTGGAGTACTTTCCTTCTCAGATGCTGCGTACCGGAAACAACTTT
ACCTTCAGCTACACTTTTGAGGACGTTCCTTTCCACAGCAGCTACGCTCACAGCCA
GAGTCTGGACCGTCTCATGAATCCTCTCATCGACCAGTACCTGTATTACTTGAGCAG
AACAAACACTCCAAGTGGAGGTCGTGGCGACAGCCCGACCACCACGCAGTCAAGG
CTTCAGTTTTCTCAGGCCGGAGCGAGTGACATTCGGGACCAGTCTAGGAACTGGCT
TCCTGGACCCTGTTACCGCCAGCAGCGAGTATCAAAGACATCTGCGGATAACAACA
ACAGTGAATACTCGTGGACTGGAGCTACCAAGTACCACCTCAATGGCAGAGACTCT
CTGGTGAATCCGGGCCCGGCCATGGCAAGCCACAAGGACGATGAAGAAAAGTTTT
TTCCTCAGAGCGGGGTTCTCATCTTTGGGAAGCAAGGCTCAGAGAAAACAAATGTG
 
GACATTGAAAAGGTCATGATTACAGACGAAGAGGAAATCAGGACAACCAATCCCGT
GGCTACGGAGCAGTATGGTTCTGTATCTACCAACCTCCAGAGAGGCAACAGACAAG
CAGCTACCGCAGATGTCAACACACAAGGCGTTCTTCCAGGCATGGTCTGGCAGGAC
AGAGATGTGTACCTTCAGGGGCCCATCTGGGCAAAGATTCCACACACGGACGGACA
TTTTCACCCCTCTCCCCTCATGGGTGGATTCGGACTTAAACACCCTCCTCCACAGAT
TCTCATCAAGAACACCCCGGTACCTGCGAATCCTTCGACCACCTTCAGTGCGGCAA
AGTTTGCTTCCTTCATCACACAGTACTCCACGGGACAGGTCAGCGTGGAGATCGAG
TGGGAGCTGCAGAAGGAAAACAGCAAACGCTGGAATCCCGAAATTCAGTACACTT
CCAACTACAACAAGTCTGTTAATGTGGACTTTACTGTGGACACTAATGGCGTGTATT
CAGAGCCTCGCCCCATTGGCACCAGATACCTGACTCGTAATCTGTAATTGCTTGTTA
ATCAATAAACCGTTTAATTCGTTTCAGTTGAACTTTGGTCTCTGCGTATTTCTTTCTTA
TCTAGTTTCCATGGCTACGTAGATAAGTAGCATGGCGGGTTAATCATTAACTACAGCC
CGGGCGTTTAAACAGCGGGCGGAGGGGTGGAGTCGTGACGTGAATTACGTCATAGG
GTTAGGGAGGTCCTGTATTAGAGGTCACGTGAGTGTTTTGCGACATTTTGCGACACC
ATGTGGTCTCGCTGGGGGGGGGGGCCCGAGTGAGCACGCAGGGTCTCCATTTTGAA
GCGGGAGGTTTGAACGAGCGCTGGCGCGCTCACTGGCCGTCGTTTTACAACGTCGT
GACTGGGAAAACCCTGGCGTTACCCAACTTAATCGCCTTGCAGCACATCCCCCTTTC
GCCAGCTGGCGTAATAGCGAAGAGGCCCGCACCGATCGCCCTTCCCAACAGTTGCG
CAGCCTGAATGGCGAATGGAAATTGTAAGCGTTAATATTTTGTTAAAATTCGCGTTA
AATTTTTGTTAAATCAGCTCATTTTTTTAACCAATAGGCCGAAATCGGCAAAATCCCT
TATAAATCAAAAGAATAGACCGAGATAGGGTTGAGTGTTGTTCCAGTTTGGAACAA
GAGTCCACTATTAAGAACGTGGACTCCAACGTCAAAGGGCGAAAAACCGTCTATCA
GGGCGATGGCCCACTACGTGAACCATCACCCTAATCAAGTTTTTTGGGGTCGAGGT
GCCGTAAAGCACTAAATCGGAACCCTAAAGGGAGCCCCCGATTTAGAGCTTGACGG
GGAAAGCCGGCGAACGTGGCGAGAAAGGAAGGGAAGAAAGCGAAAGGAGCGGG
CGCTAGGGCGCTGGCAAGTGTAGCGGTCACGCTGCGCGTAACCACCACACCCGCCG
CGCTTAATGCGCCGCTACAGGGCGCGTCAGGTGGCACTTTTCGGGGAAATGTGCGC
GGAACCCCTATTTGTTTATTTTTCTAAATACATTCAAATATGTATCCGCTCATGAGACA
ATAACCCTGATAAATGCTTCAATAATATTGAAAAAGGAAGAGTATGAGTATTCAACAT
TTCCGTGTCGCCCTTATTCCCTTTTTTGCGGCATTTTGCCTTCCTGTTTTTGCTCACCC
AGAAACGCTGGTGAAAGTAAAAGATGCTGAAGATCAGTTGGGTGCACGAGTGGGT
TACATCGAACTGGATCTCAACAGCGGTAAGATCCTTGAGAGTTTTCGCCCCGAAGA
ACGTTTTCCAATGATGAGCACTTTTAAAGTTCTGCTATGTGGCGCGGTATTATCCCGT
ATTGACGCCGGGCAAGAGCAACTCGGTCGCCGCATACACTATTCTCAGAATGACTT
GGTTGAGTACTCACCAGTCACAGAAAAGCATCTTACGGATGGCATGACAGTAAGAG
 
AATTATGCAGTGCTGCCATAACCATGAGTGATAACACTGCGGCCAACTTACTTCTGA
CAACGATCGGAGGACCGAAGGAGCTAACCGCTTTTTTGCACAACATGGGGGATCAT
GTAACTCGCCTTGATCGTTGGGAACCGGAGCTGAATGAAGCCATACCAAACGACGA
GCGTGACACCACGATGCCTGTAGCAATGGCAACAACGTTGCGCAAACTATTAACTG
GCGAACTACTTACTCTAGCTTCCCGGCAACAATTAATAGACTGGATGGAGGCGGATA
AAGTTGCAGGACCACTTCTGCGCTCGGCCCTTCCGGCTGGCTGGTTTATTGCTGATA
AATCTGGAGCCGGTGAGCGTGGGTCTCGCGGTATCATTGCAGCACTGGGGCCAGAT
GGTAAGCCCTCCCGTATCGTAGTTATCTACACGACGGGGAGTCAGGCAACTATGGAT
GAACGAAATAGACAGATCGCTGAGATAGGTGCCTCACTGATTAAGCATTGGTAACT
GTCAGACCAAGTTTACTCATATATACTTTAGATTGATTTAAAACTTCATTTTTAATTTA
AAAGGATCTAGGTGAAGATCCTTTTTGATAATCTCATGACCAAAATCCCTTAACGTG
AGTTTTCGTTCCACTGAGCGTCAGACCCCGTAGAAAAGATCAAAGGATCTTCTTGA
GATCCTTTTTTTCTGCGCGTAATCTGCTGCTTGCAAACAAAAAAACCACCGCTACCA
GCGGTGGTTTGTTTGCCGGATCAAGAGCTACCAACTCTTTTTCCGAAGGTAACTGG
CTTCAGCAGAGCGCAGATACCAAATACTGTTCTTCTAGTGTAGCCGTAGTTAGGCCA
CCACTTCAAGAACTCTGTAGCACCGCCTACATACCTCGCTCTGCTAATCCTGTTACC
AGTGGCTGCTGCCAGTGGCGATAAGTCGTGTCTTACCGGGTTGGACTCAAGACGAT
AGTTACCGGATAAGGCGCAGCGGTCGGGCTGAACGGGGGGTTCGTGCACACAGCC
CAGCTTGGAGCGAACGACCTACACCGAACTGAGATACCTACAGCGTGAGCTATGAG
AAAGCGCCACGCTTCCCGAAGGGAGAAAGGCGGACAGGTATCCGGTAAGCGGCAG
GGTCGGAACAGGAGAGCGCACGAGGGAGCTTCCAGGGGGAAACGCCTGGTATCTT
TATAGTCCTGTCGGGTTTCGCCACCTCTGACTTGAGCGTCGATTTTTGTGATGCTCGT
CAGGGGGGCGGAGCCTATGGAAAAACGCCAGCAACGCGGCCTTTTTACGGTTCCTG
GCCTTTTGCTGGCCTTTTGCTCACATGTTCTTTCCTGCGTTATCCCCTGATTCTGTGG
ATAACCGTATTACCGCCTTTGAGTGAGCTGATACCGCTCGCCGCAGCCGAACGACCG
AGCGCAGCGAGTCAGTGAGCGAGGAAGCGGAAGAGCGCCCAATACGCAAACCGC
CTCTCCCCGCGCGTTGGCCGATTCATTAATGCAGCTGGCAC
SEQ ID NO: 7 (nucleotide sequence of RC2.3 plasmid): 
GACAGGTTTCCCGACTGGAAAGCGGGCAGTGAGCGCAACGCAATTAATGTGA
GTTAGCTCACTCATTAGGCACCCCAGGCTTTACACTTTATGCTTCCGGCTCGTATGTT
GTGTGGAATTGTGAGCGGATAACAATTTCACACAGGAAACAGCTATGACCATGATTA
CGCCAAGCGCGCCGATATCGTTAACGCCCCGCGCCGGCCGCTCTAGAACTAGTGGA
TCCCCCGGAAGATCAGAAGTTCCTATTCCGAAGTTCCTATTCTCTAGAAAGTATAGG
AACTTCTGATCTGCGCAGCCGCCATGCCGGGGTTTTACGAGATTGTGATTAAGGTCC
CCAGCGACCTTGACGAGCATCTGCCCGGCATTTCTGACAGCTTTGTGAACTGGGTG
GCCGAGAAGGAATGGGAGTTGCCGCCAGATTCTGACATGGATCTGAATCTGATTGA
GCAGGCACCCCTGACCGTGGCCGAGAAGCTGCAGCGCGACTTTCTGACGGAATGG
CGCCGTGTGAGTAAGGCCCCGGAGGCCCTTTTCTTTGTGCAATTTGAGAAGGGAGA
GAGCTACTTCCACATGCACGTGCTCGTGGAAACCACCGGGGTGAAATCCATGGTTT
TGGGACGTTTCCTGAGTCAGATTCGCGAAAAACTGATTCAGAGAATTTACCGCGGG
ATCGAGCCGACTTTGCCAAACTGGTTCGCGGTCACAAAGACCAGAAATGGCGCCG
GAGGCGGGAACAAGGTGGTGGATGAGTGCTACATCCCCAATTACTTGCTCCCCAAA
ACCCAGCCTGAGCTCCAGTGGGCGTGGACTAATATGGAACAGTATTTAAGCGCCTG
TTTGAATCTCACGGAGCGTAAACGGTTGGTGGCGCAGCATCTGACGCACGTGTCGC
AGACGCAGGAGCAGAACAAAGAGAATCAGAATCCCAATTCTGATGCGCCGGTGAT
CAGATCAAAAACTTCAGCCAGGTACATGGAGCTGGTCGGGTGGCTCGTGGACAAG
GGGATTACCTCGGAGAAGCAGTGGATCCAGGAGGACCAGGCCTCATACATCTCCTT
CAATGCGGCCTCCAACTCGCGGTCCCAAATCAAGGCTGCCTTGGACAATGCGGGAA
AGATTATGAGCCTGACTAAAACCGCCCCCGACTACCTGGTGGGCCAGCAGCCCGTG
GAGGACATTTCCAGCAATCGGATTTATAAAATTTTGGAACTAAACGGGTACGATCCC
CAATATGCGGCTTCCGTCTTTCTGGGATGGGCCACGAAAAAGTTCGGCAAGAGGAA
CACCATCTGGCTGTTTGGGCCTGCAACTACCGGGAAGACCAACATCGCGGAGGCCA
TAGCCCACACTGTGCCCTTCTACGGGTGCGTAAACTGGACCAATGAGAACTTTCCCT
TCAACGACTGTGTCGACAAGATGGTGATCTGGTGGGAGGAGGGGAAGATGACCGC
CAAGGTCGTGGAGTCGGCCAAAGCCATTCTCGGAGGAAGCAAGGTGCGCGTGGAC
CAGAAATGCAAGTCCTCGGCCCAGATAGACCCGACTCCCGTGATCGTCACCTCCAA
CACCAACATGTGCGCCGTGATTGACGGGAACTCAACGACCTTCGAACACCAGCAG
CCGTTGCAAGACCGGATGTTCAAATTTGAACTCACCCGCCGTCTGGATCATGACTTT
GGGAAGGTCACCAAGCAGGAAGTCAAAGACTTTTTCCGGTGGGCAAAGGATCACG
TGGTTGAGGTGGAGCATGAATTCTACGTCAAAAAGGGTGGAGCCAAGAAAAGACC
CGCCCCCAGTGACGCAGATATAAGTGAGCCCAAACGGGTGCGCGAGTCAGTTGCGC
AGCCATCGACGTCAGACGCGGAAGCTTCGATCAACTACGCAGACAGGTACCAAAA
 
CAAATGTTCTCGTCACGTGGGCATGAATCTGATGCTGTTTCCCTGCAGACAATGCGA
GAGAATGAATCAGAATTCAAATATCTGCTTCACTCACGGACAGAAAGACTGTTTAG
AGTGCTTTCCCGTGTCAGAATCTCAACCCGTTTCTGTCGTCAAAAAGGCGTATCAGA
AACTGTGCTACATTCATCATATCATGGGAAAGGTGCCAGACGCTTGCACTGCCTGCG
ATCTGGTCAATGTGGATTTGGATGACTGCATCTTTGAACAATAAATGATTTAAATCAG
GTATGGCTGCCGATGGTTATCTTCCAGATTGGCTCGAGGACACTCTCTCTGAAGGAA
TAAGACAGTGGTGGAAGCTCAAACCTGGCCCACCACCACCAAAGCCCGCAGAGCG
GCATAAGGACGACAGCAGGGGTCTTGTGCTTCCTGGGTACAAGTACCTCGGACCCT
TCAACGGACTCGACAAGGGAGAGCCGGTCAACGAGGCAGACGCCGCGGCCCTCG
AGCACGACAAAGCCTACGACCGGCAGCTCGACAGCGGAGACAACCCGTACCTCAA
GTACAACCACGCCGACGCGGAGTTTCAGGAGCGCCTTAAAGAAGATACGTCTTTTG
GGGGCAACCTCGGACGAGCAGTCTTCCAGGCGAAAAAGAGGGTTCTTGAACCTCT
GGGCCTGGTTGAGGAACCTGTTAAGACGGCTCCGGGAAAAAAGAGGCCGGTAGAG
CACTCTCCTGTGGAGCCAGACTCCTCCTCGGGAACCGGAAAGGCGGGCCAGCAGC
CTGCAAGAAAAAGATTGAATTTTGGTCAGACTGGAGACGCAGACTCAGTACCTGAC
CCCCAGCCTCTCGGACAGCCACCAGCAGCCCCCTCTGGTCTGGGAACTAATACGAT
GGCTACAGGCAGTGGCGCACCAATGGCAGACAATAACGAGGGCGCCGACGGAGTG
GGTAATTCCTCGGGAAATTGGCATTGCGATTCCACATGGATGGGCGACAGAGTCATC
ACCACCAGCACCCGAACCTGGGCCCTGCCCACCTACAACAACCACCTCTACAAACA
AATTTCCAGCCAATCAGGAGCCTCGAACGACAATCACTACTTTGGCTACAGCACCC
CTTGGGGGTATTTTGACTTCAACAGATTCCACTGCCACTTTTCACCACGTGACTGGC
AAAGACTCATCAACAACAACTGGGGATTCCGACCCAAGAGACTCAACTTCAAGCT
CTTTAACATTCAAGTCAAAGAGGTCACGCAGAATGACGGTACGACGACGATTGCCA
ATAACCTTACCAGCACGGTTCAGGTGTTTACTGACTCGGAGTACCAGCTCCCGTACG
TCCTCGGCTCGGCGCATCAAGGATGCCTCCCGCCGTTCCCAGCAGACGTCTTCATG
GTGCCACAGTATGGATACCTCACCCTGAACAACGGGAGTCAGGCAGTAGGACGCTC
TTCATTTTACTGCCTGGAGTACTTTCCTTCTCAGATGCTGCGTACCGGAAACAACTTT
ACCTTCAGCTACACTTTTGAGGACGTTCCTTTCCACAGCAGCTACGCTCACAGCCA
GAGTCTGGACCGTCTCATGAATCCTCTCATCGACCAGTACCTGTATTACTTGAGCAG
AACAAACACTCCAAGTGGAACCACCACGCAGTCAAGGCTTCAGTTTTCTCAGGCC
GGAGCGAGTGACATTCGGGACCAGTCTAGGAACTGGCTTCCTGGACCCTGTTACCG
CCAGCAGCGAGTATCAAAGACATCTGCGGATAACAACAACAGTGAATACTCGTGGA
CTGGAGCTACCAAGTACCACCTCAATGGCAGAGACTCTCTGGTGAATCCGGGCCCG
GCCATGGCAAGCCACAAGGACGATGAAGAAAAGTTTTTTCCTCAGAGCGGGGTTCT
CATCTTTGGGAAGCAAGGCTCAGAGAAAACAAATGTGGACATTGAAAAGGTCATGA
 
TTACAGACGAAGAGGAAATCAGGACAACCAATCCCGTGGCTACGGAGCAGTATGGT
TCTGTATCTACCAACCTCCAGAGAGGCAACAGAGGTCGTGGCGACAGCCCGCAAG
CAGCTACCGCAGATGTCAACACACAAGGCGTTCTTCCAGGCATGGTCTGGCAGGAC
AGAGATGTGTACCTTCAGGGGCCCATCTGGGCAAAGATTCCACACACGGACGGACA
TTTTCACCCCTCTCCCCTCATGGGTGGATTCGGACTTAAACACCCTCCTCCACAGAT
TCTCATCAAGAACACCCCGGTACCTGCGAATCCTTCGACCACCTTCAGTGCGGCAA
AGTTTGCTTCCTTCATCACACAGTACTCCACGGGACAGGTCAGCGTGGAGATCGAG
TGGGAGCTGCAGAAGGAAAACAGCAAACGCTGGAATCCCGAAATTCAGTACACTT
CCAACTACAACAAGTCTGTTAATGTGGACTTTACTGTGGACACTAATGGCGTGTATT
CAGAGCCTCGCCCCATTGGCACCAGATACCTGACTCGTAATCTGTAATTGCTTGTTA
ATCAATAAACCGTTTAATTCGTTTCAGTTGAACTTTGGTCTCTGCGTATTTCTTTCTTA
TCTAGTTTCCATGGCTACGTAGATAAGTAGCATGGCGGGTTAATCATTAACTACAGCC
CGGGCGTTTAAACAGCGGGCGGAGGGGTGGAGTCGTGACGTGAATTACGTCATAGG
GTTAGGGAGGTCCTGTATTAGAGGTCACGTGAGTGTTTTGCGACATTTTGCGACACC
ATGTGGTCTCGCTGGGGGGGGGGGCCCGAGTGAGCACGCAGGGTCTCCATTTTGAA
GCGGGAGGTTTGAACGAGCGCTGGCGCGCTCACTGGCCGTCGTTTTACAACGTCGT
GACTGGGAAAACCCTGGCGTTACCCAACTTAATCGCCTTGCAGCACATCCCCCTTTC
GCCAGCTGGCGTAATAGCGAAGAGGCCCGCACCGATCGCCCTTCCCAACAGTTGCG
CAGCCTGAATGGCGAATGGAAATTGTAAGCGTTAATATTTTGTTAAAATTCGCGTTA
AATTTTTGTTAAATCAGCTCATTTTTTTAACCAATAGGCCGAAATCGGCAAAATCCCT
TATAAATCAAAAGAATAGACCGAGATAGGGTTGAGTGTTGTTCCAGTTTGGAACAA
GAGTCCACTATTAAGAACGTGGACTCCAACGTCAAAGGGCGAAAAACCGTCTATCA
GGGCGATGGCCCACTACGTGAACCATCACCCTAATCAAGTTTTTTGGGGTCGAGGT
GCCGTAAAGCACTAAATCGGAACCCTAAAGGGAGCCCCCGATTTAGAGCTTGACGG
GGAAAGCCGGCGAACGTGGCGAGAAAGGAAGGGAAGAAAGCGAAAGGAGCGGG
CGCTAGGGCGCTGGCAAGTGTAGCGGTCACGCTGCGCGTAACCACCACACCCGCCG
CGCTTAATGCGCCGCTACAGGGCGCGTCAGGTGGCACTTTTCGGGGAAATGTGCGC
GGAACCCCTATTTGTTTATTTTTCTAAATACATTCAAATATGTATCCGCTCATGAGACA
ATAACCCTGATAAATGCTTCAATAATATTGAAAAAGGAAGAGTATGAGTATTCAACAT
TTCCGTGTCGCCCTTATTCCCTTTTTTGCGGCATTTTGCCTTCCTGTTTTTGCTCACCC
AGAAACGCTGGTGAAAGTAAAAGATGCTGAAGATCAGTTGGGTGCACGAGTGGGT
TACATCGAACTGGATCTCAACAGCGGTAAGATCCTTGAGAGTTTTCGCCCCGAAGA
ACGTTTTCCAATGATGAGCACTTTTAAAGTTCTGCTATGTGGCGCGGTATTATCCCGT
ATTGACGCCGGGCAAGAGCAACTCGGTCGCCGCATACACTATTCTCAGAATGACTT
GGTTGAGTACTCACCAGTCACAGAAAAGCATCTTACGGATGGCATGACAGTAAGAG
 
AATTATGCAGTGCTGCCATAACCATGAGTGATAACACTGCGGCCAACTTACTTCTGA
CAACGATCGGAGGACCGAAGGAGCTAACCGCTTTTTTGCACAACATGGGGGATCAT
GTAACTCGCCTTGATCGTTGGGAACCGGAGCTGAATGAAGCCATACCAAACGACGA
GCGTGACACCACGATGCCTGTAGCAATGGCAACAACGTTGCGCAAACTATTAACTG
GCGAACTACTTACTCTAGCTTCCCGGCAACAATTAATAGACTGGATGGAGGCGGATA
AAGTTGCAGGACCACTTCTGCGCTCGGCCCTTCCGGCTGGCTGGTTTATTGCTGATA
AATCTGGAGCCGGTGAGCGTGGGTCTCGCGGTATCATTGCAGCACTGGGGCCAGAT
GGTAAGCCCTCCCGTATCGTAGTTATCTACACGACGGGGAGTCAGGCAACTATGGAT
GAACGAAATAGACAGATCGCTGAGATAGGTGCCTCACTGATTAAGCATTGGTAACT
GTCAGACCAAGTTTACTCATATATACTTTAGATTGATTTAAAACTTCATTTTTAATTTA
AAAGGATCTAGGTGAAGATCCTTTTTGATAATCTCATGACCAAAATCCCTTAACGTG
AGTTTTCGTTCCACTGAGCGTCAGACCCCGTAGAAAAGATCAAAGGATCTTCTTGA
GATCCTTTTTTTCTGCGCGTAATCTGCTGCTTGCAAACAAAAAAACCACCGCTACCA
GCGGTGGTTTGTTTGCCGGATCAAGAGCTACCAACTCTTTTTCCGAAGGTAACTGG
CTTCAGCAGAGCGCAGATACCAAATACTGTTCTTCTAGTGTAGCCGTAGTTAGGCCA
CCACTTCAAGAACTCTGTAGCACCGCCTACATACCTCGCTCTGCTAATCCTGTTACC
AGTGGCTGCTGCCAGTGGCGATAAGTCGTGTCTTACCGGGTTGGACTCAAGACGAT
AGTTACCGGATAAGGCGCAGCGGTCGGGCTGAACGGGGGGTTCGTGCACACAGCC
CAGCTTGGAGCGAACGACCTACACCGAACTGAGATACCTACAGCGTGAGCTATGAG
AAAGCGCCACGCTTCCCGAAGGGAGAAAGGCGGACAGGTATCCGGTAAGCGGCAG
GGTCGGAACAGGAGAGCGCACGAGGGAGCTTCCAGGGGGAAACGCCTGGTATCTT
TATAGTCCTGTCGGGTTTCGCCACCTCTGACTTGAGCGTCGATTTTTGTGATGCTCGT
CAGGGGGGCGGAGCCTATGGAAAAACGCCAGCAACGCGGCCTTTTTACGGTTCCTG
GCCTTTTGCTGGCCTTTTGCTCACATGTTCTTTCCTGCGTTATCCCCTGATTCTGTGG
ATAACCGTATTACCGCCTTTGAGTGAGCTGATACCGCTCGCCGCAGCCGAACGACCG
AGCGCAGCGAGTCAGTGAGCGAGGAAGCGGAAGAGCGCCCAATACGCAAACCGC
CTCTCCCCGCGCGTTGGCCGATTCATTAATGCAGCTGGCAC
SEQ ID NO: 8 (AAV6 VP1 gene sequence): 
ATGGCTGCCGATGGTTATCTTCCAGATTGGCTCGAGGACAACCTCTCTGAGGGC
ATTCGCGAGTGGTGGGACTTGAAACCTGGAGCCCCGAAACCCAAAGCCAACCAGC
AAAAGCAGGACGACGGCCGGGGTCTGGTGCTTCCTGGCTACAAGTACCTCGGACC
CTTCAACGGACTCGACAAGGGGGAGCCCGTCAACGCGGCGGATGCAGCGGCCCTC
GAGCACGACAAGGCCTACGACCAGCAGCTCAAAGCGGGTGACAATCCGTACCTGC
GGTATAACCACGCCGACGCCGAGTTTCAGGAGCGTCTGCAAGAAGATACGTCTTTT
GGGGGCAACCTCGGGCGAGCAGTCTTCCAGGCCAAGAAGAGGGTTCTCGAACCTC
TTGGTCTGGTTGAGGAAGGTGCTAAGACGGCTCCTGGAAAGAAACGTCCGGTAGA
GCAGTCGCCACAAGAGCCAGACTCCTCCTCGGGCATTGGCAAGACAGGCCAGCAG
CCCGCTAAAAAGAGACTCAATTTTGGTCAGACTGGCGACTCAGAGTCAGTCCCCGA
CCCACAACCTCTCGGAGAACCTCCAGCAACCCCCGCTGCTGTGGGACCTACTACAA
TGGCTTCAGGCGGTGGCGCACCAATGGCAGACAATAACGAAGGCGCCGACGGAGT
GGGTAATGCCTCAGGAAATTGGCATTGCGATTCCACATGGCTGGGCGACAGAGTCA
TCACCACCAGCACCCGAACATGGGCCTTGCCCACCTATAACAACCACCTCTACAAG
CAAATCTCCAGTGCTTCAACGGGGGCCAGCAACGACAACCACTACTTCGGCTACAG
CACCCCCTGGGGGTATTTTGATTTCAACAGATTCCACTGCCATTTCTCACCACGTGA
CTGGCAGCGACTCATCAACAACAATTGGGGATTCCGGCCCAAGAGACTCAACTTCA
AGCTCTTCAACATCCAAGTCAAGGAGGTCACGACGAATGATGGCGTCACGACCATC
GCTAATAACCTTACCAGCACGGTTCAAGTCTTCTCGGACTCGGAGTACCAGTTGCCG
TACGTCCTCGGCTCTGCGCACCAGGGCTGCCTCCCTCCGTTCCCGGCGGACGTGTT
CATGATTCCGCAGTACGGCTACCTAACGCTCAACAATGGCAGCCAGGCAGTGGGAC
GGTCATCCTTTTACTGCCTGGAATATTTCCCATCGCAGATGCTGAGAACGGGCAATA
ACTTTACCTTCAGCTACACCTTCGAGGACGTGCCTTTCCACAGCAGCTACGCGCAC
AGCCAGAGCCTGGACCGGCTGATGAATCCTCTCATCGACCAGTACCTGTATTACCTG
AACAGAACTCAGAATCAGTCCGGAAGTGCCCAAAACAAGGACTTGCTGTTTAGCC
GGGGGTCTCCAGCTGGCATGTCTGTTCAGCCCAAAAACTGGCTACCTGGACCCTGT
TACCGGCAGCAGCGCGTTTCTAAAACAAAAACAGACAACAACAACAGCAACTTTA
CCTGGACTGGTGCTTCAAAATATAACCTTAATGGGCGTGAATCTATAATCAACCCTG
GCACTGCTATGGCCTCACACAAAGACGACAAAGACAAGTTCTTTCCCATGAGCGGT
GTCATGATTTTTGGAAAGGAGAGCGCCGGAGCTTCAAACACTGCATTGGACAATGT
CATGATCACAGACGAAGAGGAAATCAAAGCCACTAACCCCGTGGCCACCGAAAGA
TTTGGGACTGTGGCAGTCAATCTCCAGAGCAGCAGCACAGACCCTGCGACCGGAG
ATGTGCATGTTATGGGAGCCTTACCTGGAATGGTGTGGCAAGACAGAGACGTATACC
TGCAGGGTCCTATTTGGGCCAAAATTCCTCACACGGATGGACACTTTCACCCGTCTC
 
CTCTCATGGGCGGCTTTGGACTTAAGCACCCGCCTCCTCAGATCCTCATCAAAAACA
CGCCTGTTCCTGCGAATCCTCCGGCAGAGTTTTCGGCTACAAAGTTTGCTTCATTCA
TCACCCAGTATTCCACAGGACAAGTGAGCGTGGAGATTGAATGGGAGCTGCAGAA
AGAAAACAGCAAACGCTGGAATCCCGAAGTGCAGTATACATCTAACTATGCAAAAT
CTGCCAACGTTGATTTCACTGTGGACAACAATGGACTTTATACTGAGCCTCGCCCCA
TTGGCACCCGTTACCTCACCCGTCCCCTGTAA
SEQ ID NO: 9 (nucleotide sequence of RC6.2 plasmid): 
AGGGTTGAGTGTTGTTCCAGTTTGGAACAAGAGTCCACTATTAAAGAACGTGG
ACTCCAACGTCAAAGGGCGAAAAACCGTCTATCAGGGCGATGGCCCACTACGTGA
ACCATCACCCTAATCAAGTTTTTTGGGGTCGAGGTGCCGTAAAGCACTAAATCGGA
ACCCTAAAGGGAGCCCCCGATTTAGAGCTTGACGGGGAAAGCCGGCGAACGTGGC
GAGAAAGGAAGGGAAGAAAGCGAAAGGAGCGGGCGCTAGGGCGCTGGCAAGTGT
AGCGGTCACGCTGCGCGTAACCACCACACCCGCCGCGCTTAATGCGCCGCTACAGG
GCGCGTCCATTCGCCATTCAGGCTGCGCAACTGTTGGGAAGGGCGATCGGTGCGGG
CCTCTTCGCTATTACGCCAGCTGGCGAAAGGGGGATGTGCTGCAAGGCGATTAAGT
TGGGTAACGCCAGGGTTTTCCCAGTCACGACGTTGTAAAACGACGGCCAGTGAATT
GTAATACGACTCACTATAGGGCGAATTCGAGCTCGGTACCCCTAGAGGTCCTGTATT
AGAGGTCACGTGAGTGTTTTGCGACATTTTGCGACACCATGTGGTCACGCTGGGTAT
TTAAGCCCGAGTGAGCACGCAGGGTCTCCATTTTGAAGCGGGAGGTTTGAACGCGC
AGCCACCACGCCGGGGTTTTACGAGATTGTGATTAAGGTCCCCAGCGACCTTGACG
AGCATCTGCCCGGCATTTCTGACAGCTTTGTGAACTGGGTGGCCGAGAAGGAATGG
GAGTTGCCGCCAGATTCTGACATGGATCTGAATCTGATTGAGCAGGCACCCCTGAC
CGTGGCCGAGAAGCTGCAGCGCGACTTTCTGACGGAATGGCGCCGTGTGAGTAAG
GCCCCGGAGGCCCTTTTCTTTGTGCAATTTGAGAAGGGAGAGAGCTACTTCCACAT
GCACGTGCTCGTGGAAACCACCGGGGTGAAATCCATGGTTTTGGGACGTTTCCTGA
GTCAGATTCGCGAAAAACTGATTCAGAGAATTTACCGCGGGATCGAGCCGACTTTG
CCAAACTGGTTCGCGGTCACAAAGACCAGAAATGGCGCCGGAGGCGGGAACAAG
GTGGTGGATGAGTGCTACATCCCCAATTACTTGCTCCCCAAAACCCAGCCTGAGCTC
CAGTGGGCGTGGACTAATATGGAACAGTATTTAAGCGCCTGTTTGAATCTCACGGAG
CGTAAACGGTTGGTGGCGCAGCATCTGACGCACGTGTCGCAGACGCAGGAGCAGA
ACAAAGAGAATCAGAATCCCAATTCTGATGCGCCGGTGATCAGATCAAAAACTTCA
GCCAGGTACATGGAGCTGGTCGGGTGGCTCGTGGACAAGGGGATTACCTCGGAGA
AGCAGTGGATCCAGGAGGACCAGGCCTCATACATCTCCTTCAATGCGGCCTCCAAC
TCGCGGTCCCAAATCAAGGCTGCCTTGGACAATGCGGGAAAGATTATGAGCCTGAC
TAAAACCGCCCCCGACTACCTGGTGGGCCAGCAGCCCGTGGAGGACATTTCCAGCA
ATCGGATTTATAAAATTTTGGAACTAAACGGGTACGATCCCCAATATGCGGCTTCCGT
CTTTCTGGGATGGGCCACGAAAAAGTTCGGCAAGAGGAACACCATCTGGCTGTTTG
GGCCTGCAACTACCGGGAAGACCAACATCGCGGAGGCCATAGCCCACACTGTGCCC
TTCTACGGGTGCGTAAACTGGACCAATGAGAACTTTCCCTTCAACGACTGTGTCGA
CAAGATGGTGATCTGGTGGGAGGAGGGCAAGATGACGGCCAAGGTCGTGGAGTCC
GCCAAGGCCATTCTCGGCGGCAGCAAGGTGCGCGTGGACCAAAAGTGCAAGTCGT
 
CCGCCCAGATCGATCCCACCCCCGTGATCGTCACCTCCAACACCAACATGTGCGCC
GTGATTGACGGGAACAGCACCACCTTCGAGCACCAGCAGCCGTTGCAGGACCGGA
TGTTCAAATTTGAACTCACCCGCCGTCTGGAGCATGACTTTGGCAAGGTGACAAAG
CAGGAAGTCAAAGAGTTCTTCCGCTGGGCGCAGGATCACGTGACCGAGGTGGCGC
ATGAGTTCTACGTCAGAAAGGGTGGAGCCAACAAGAGACCCGCCCCCGATGACGC
GGATAAAAGCGAGCCCAAGCGGGCCTGCCCCTCAGTCGCGGATCCATCGACGTCAG
ACGCGGAAGGAGCTCCGGTGGACTTTGCCGACAGGTACCAAAACAAATGTTCTCGT
CACGCGGGCATGCTTCAGATGCTGTTTCCCTGCAAAACATGCGAGAGAATGAATCA
GAATTTCAACATTTGCTTCACGCACGGGACCAGAGACTGTTCAGAATGTTTCCCCG
GCGTGTCAGAATCTCAACCGGTCGTCAGAAAGAGGACGTATCGGAAACTCTGTGCC
ATTCATCATCTGCTGGGGCGGGCTCCCGAGATTGCTTGCTCGGCCTGCGATCTGGTC
AACGTGGATCTGGATGACTGTGTTTCTGAGCAATAAATGACTTAAACCAGGTATGGC
TGCCGATGGTTATCTTCCAGATTGGCTCGAGGACAACCTCTCTGAGGGCATTCGCGA
GTGGTGGGACTTGAAACCTGGAGCCCCGAAACCCAAAGCCAACCAGCAAAAGCA
GGACGACGGCCGGGGTCTGGTGCTTCCTGGCTACAAGTACCTCGGACCCTTCAACG
GACTCGACAAGGGGGAGCCCGTCAACGCGGCGGATGCAGCGGCCCTCGAGCACGA
CAAGGCCTACGACCAGCAGCTCAAAGCGGGTGACAATCCGTACCTGCGGTATAACC
ACGCCGACGCCGAGTTTCAGGAGCGTCTGCAAGAAGATACGTCTTTTGGGGGCAAC
CTCGGGCGAGCAGTCTTCCAGGCCAAGAAGAGGGTTCTCGAACCTTTTGGTCTGGT
TGAGGAAGGTGCTAAGACGGCTCCTGGAAAGAAACGTCCGGTAGAGCAGTCGCCA
CAAGAGCCAGACTCCTCCTCGGGCATTGGCAAGACAGGCCAGCAGCCCGCTAAAA
AGAGACTCAATTTTGGTCAGACTGGCGACTCAGAGTCAGTCCCCGACCCACAACCT
CTCGGAGAACCTCCAGCAACCCCCGCTGCTGTGGGACCTACTACAATGGCTTCAGG
CGGTGGCGCACCAATGGCAGACAATAACGAAGGCGCCGACGGAGTGGGTAATGCC
TCAGGAAATTGGCATTGCGATTCCACATGGCTGGGCGACAGAGTCATCACCACCAG
CACCCGAACATGGGCCTTGCCCACCTATAACAACCACCTCTACAAGCAAATCTCCA
GTGCTTCAACGGGGGCCAGCAACGACAACCACTACTTCGGCTACAGCACCCCCTGG
GGGTATTTTGATTTCAACAGATTCCACTGCCATTTCTCACCACGTGACTGGCAGCGA
CTCATCAACAACAATTGGGGATTCCGGCCCAAGAGACTCAACTTCAAGCTCTTCAA
CATCCAAGTCAAGGAGGTCACGACGAATGATGGCGTCACGACCATCGCTAATAACC
TTACCAGCACGGTTCAAGTCTTCTCGGACTCGGAGTACCAGTTGCCGTACGTCCTCG
GCTCTGCGCACCAGGGCTGCCTCCCTCCGTTCCCGGCGGACGTGTTCATGATTCCGC
AGTACGGCTACCTAACGCTCAACAATGGCAGCCAGGCAGTGGGACGGTCATCCTTT
TACTGCCTGGAATATTTCCCATCGCAGATGCTGAGAACGGGCAATAACTTTACCTTC
AGCTACACCTTCGAGGACGTGCCTTTCCACAGCAGCTACGCGCACAGCCAGAGCCT
 
GGACCGGCTGATGAATCCTCTCATCGACCAGTACCTGTATTACCTGAACAGAACTCA
GAATCAGTCCGGAGGTCGTGGCGACAGCCCGAGTGCCCAAAACAAGGACTTGCTG
TTTAGCCGGGGGTCTCCAGCTGGCATGTCTGTTCAGCCCAAAAACTGGCTACCTGG
ACCCTGTTACCGGCAGCAGCGCGTTTCTAAAACAAAAACAGACAACAACAACAGC
AACTTTACCTGGACTGGTGCTTCAAAATATAACCTTAATGGGCGTGAATCTATAATCA
ACCCTGGCACTGCTATGGCCTCACACAAAGACGACAAAGACAAGTTCTTTCCCATG
AGCGGTGTCATGATTTTTGGAAAGGAGAGCGCCGGAGCTTCAAACACTGCATTGGA
CAATGTCATGATCACAGACGAAGAGGAAATCAAAGCCACTAACCCCGTGGCCACCG
AAAGATTTGGGACTGTGGCAGTCAATCTCCAGAGCAGCAGCACAGACCCTGCGAC
CGGAGATGTGCATGTTATGGGAGCCTTACCTGGAATGGTGTGGCAAGACAGAGACG
TATACCTGCAGGGTCCTATTTGGGCCAAAATTCCTCACACGGATGGACACTTTCACC
CGTCTCCTCTCATGGGCGGCTTTGGACTTAAGCACCCGCCTCCTCAGATCCTCATCA
AAAACACGCCTGTTCCTGCGAATCCTCCGGCAGAGTTTTCGGCTACAAAGTTTGCT
TCATTCATCACCCAGTATTCCACAGGACAAGTGAGCGTGGAGATTGAATGGGAGCT
GCAGAAAGAAAACAGCAAACGCTGGAATCCCGAAGTGCAGTATACATCTAACTATG
CAAAATCTGCCAACGTTGATTTCACTGTGGACAACAATGGACTTTATACTGAGCCTC
GCCCCATTGGCACCCGTTACCTCACCCGTCCCCTGTAATTGTCTGTTAATCAATAAAC
CGGTTGATTCGTTTCAGTTGAACTTTGGTCTCGCGGCCGCTCGATAAGCTTTTGTTC
CCTTTAGTGAGGGTTAATTTCGAGCTTGGCGTAATCATGGTCATAGCTGTTTCCTGTG
TGAAATTGTTATCCGCTCACAATTCCACACAACATACGAGCCGGAAGCATAAAGTGT
AAAGCCTGGGGTGCCTAATGAGTGAGCTAACTCACATTAATTGCGTTGCGCTCACTG
CCCGCTTTCCAGTCGGGAAACCTGTCGTGCCAGCTGCATTAATGAATCGGCCAACG
CGCGGGGAGAGGCGGTTTGCGTATTGGGCGCTCTTCCGCTTCCTCGCTCACTGACT
CGCTGCGCTCGGTCGTTCGGCTGCGGCGAGCGGTATCAGCTCACTCAAAGGCGGTA
ATACGGTTATCCACAGAATCAGGGGATAACGCAGGAAAGAACATGTGAGCAAAAGG
CCAGCAAAAGGCCAGGAACCGTAAAAAGGCCGCGTTGCTGGCGTTTTTCCATAGGC
TCCGCCCCCCTGACGAGCATCACAAAAATCGACGCTCAAGTCAGAGGTGGCGAAA
CCCGACAGGACTATAAAGATACCAGGCGTTTCCCCCTGGAAGCTCCCTCGTGCGCT
CTCCTGTTCCGACCCTGCCGCTTACCGGATACCTGTCCGCCTTTCTCCCTTCGGGAA
GCGTGGCGCTTTCTCATAGCTCACGCTGTAGGTATCTCAGTTCGGTGTAGGTCGTTC
GCTCCAAGCTGGGCTGTGTGCACGAACCCCCCGTTCAGCCCGACCGCTGCGCCTTA
TCCGGTAACTATCGTCTTGAGTCCAACCCGGTAAGACACGACTTATCGCCACTGGCA
GCAGCCACTGGTAACAGGATTAGCAGAGCGAGGTATGTAGGCGGTGCTACAGAGTT
CTTGAAGTGGTGGCCTAACTACGGCTACACTAGAAGAACAGTATTTGGTATCTGCGC
TCTGCTGAAGCCAGTTACCTTCGGAAAAAGAGTTGGTAGCTCTTGATCCGGCAAAC
 
AAACCACCGCTGGTAGCGGTGGTTTTTTTGTTTGCAAGCAGCAGATTACGCGCAGA
AAAAAAGGATCTCAAGAAGATCCTTTGATCTTTTCTACGGGGTCTGACGCTCAGTG
GAACGAAAACTCACGTTAAGGGATTTTGGTCATGAGATTATCAAAAAGGATCTTCA
CCTAGATCCTTTTAAATTAAAAATGAAGTTTTAAATCAATCTAAAGTATATATGAGTA
AACTTGGTCTGACAGTTACCAATGCTTAATCAGTGAGGCACCTATCTCAGCGATCTG
TCTATTTCGTTCATCCATAGTTGCCTGACTCCCCGTCGTGTAGATAACTACGATACGG
GAGGGCTTACCATCTGGCCCCAGTGCTGCAATGATACCGCGAGACCCACGCTCACC
GGCTCCAGATTTATCAGCAATAAACCAGCCAGCCGGAAGGGCCGAGCGCAGAAGT
GGTCCTGCAACTTTATCCGCCTCCATCCAGTCTATTAATTGTTGCCGGGAAGCTAGA
GTAAGTAGTTCGCCAGTTAATAGTTTGCGCAACGTTGTTGCCATTGCTACAGGCATC
GTGGTGTCACGCTCGTCGTTTGGTATGGCTTCATTCAGCTCCGGTTCCCAACGATCA
AGGCGAGTTACATGATCCCCCATGTTGTGCAAAAAAGCGGTTAGCTCCTTCGGTCCT
CCGATCGTTGTCAGAAGTAAGTTGGCCGCAGTGTTATCACTCATGGTTATGGCAGCA
CTGCATAATTCTCTTACTGTCATGCCATCCGTAAGATGCTTTTCTGTGACTGGTGAGT
ACTCAACCAAGTCATTCTGAGAATAGTGTATGCGGCGACCGAGTTGCTCTTGCCCG
GCGTCAATACGGGATAATACCGCGCCACATAGCAGAACTTTAAAAGTGCTCATCATT
GGAAAACGTTCTTCGGGGCGAAAACTCTCAAGGATCTTACCGCTGTTGAGATCCAG
TTCGATGTAACCCACTCGTGCACCCAACTGATCTTCAGCATCTTTTACTTTCACCAG
CGTTTCTGGGTGAGCAAAAACAGGAAGGCAAAATGCCGCAAAAAAGGGAATAAGG
GCGACACGGAAATGTTGAATACTCATACTCTTCCTTTTTCAATATTATTGAAGCATTTA
TCAGGGTTATTGTCTCATGAGCGGATACATATTTGAATGTATTTAGAAAAATAAACAA
ATAGGGGTTCCGCGCACATTTCCCCGAAAAGTGCCACCTGACGTCTAAGAAACCAT
TATTATCATGACATTAACCTATAAAAATAGGCGTATCACGAGGCCCTTTCGTCTCGCG
CGTTTCGGTGATGACGGTGAAAACCTCTGACACATGCAGCTCCCGGAGACGGTCAC
AGCTTGTCTGTAAGCGGATGCCGGGAGCAGACAAGCCCGTCAGGGCGCGTCAGCG
GGTGTTGGCGGGTGTCGGGGCTGGCTTAACTATGCGGCATCAGAGCAGATTGTACT
GAGAGTGCACCATATGCGGTGTGAAATACCGCACAGATGCGTAAGGAGAAAATACC
GCATCAGGAAATTGTAAGCGTTAATATTTTGTTAAAATTCGCGTTAAATTTTTGTTAA
ATCAGCTCATTTTTTAACCAATAGGCCGAAATCGGCAAAATCCCTTATAAATCAAAA
GAATAGACCGAGAT
SEQ ID NO: 10 (nucleotide sequence of RC6.3 plasmid): 
AGGGTTGAGTGTTGTTCCAGTTTGGAACAAGAGTCCACTATTAAAGAACGTGG
ACTCCAACGTCAAAGGGCGAAAAACCGTCTATCAGGGCGATGGCCCACTACGTGA
ACCATCACCCTAATCAAGTTTTTTGGGGTCGAGGTGCCGTAAAGCACTAAATCGGA
ACCCTAAAGGGAGCCCCCGATTTAGAGCTTGACGGGGAAAGCCGGCGAACGTGGC
GAGAAAGGAAGGGAAGAAAGCGAAAGGAGCGGGCGCTAGGGCGCTGGCAAGTGT
AGCGGTCACGCTGCGCGTAACCACCACACCCGCCGCGCTTAATGCGCCGCTACAGG
GCGCGTCCATTCGCCATTCAGGCTGCGCAACTGTTGGGAAGGGCGATCGGTGCGGG
CCTCTTCGCTATTACGCCAGCTGGCGAAAGGGGGATGTGCTGCAAGGCGATTAAGT
TGGGTAACGCCAGGGTTTTCCCAGTCACGACGTTGTAAAACGACGGCCAGTGAATT
GTAATACGACTCACTATAGGGCGAATTCGAGCTCGGTACCCCTAGAGGTCCTGTATT
AGAGGTCACGTGAGTGTTTTGCGACATTTTGCGACACCATGTGGTCACGCTGGGTAT
TTAAGCCCGAGTGAGCACGCAGGGTCTCCATTTTGAAGCGGGAGGTTTGAACGCGC
AGCCACCACGCCGGGGTTTTACGAGATTGTGATTAAGGTCCCCAGCGACCTTGACG
AGCATCTGCCCGGCATTTCTGACAGCTTTGTGAACTGGGTGGCCGAGAAGGAATGG
GAGTTGCCGCCAGATTCTGACATGGATCTGAATCTGATTGAGCAGGCACCCCTGAC
CGTGGCCGAGAAGCTGCAGCGCGACTTTCTGACGGAATGGCGCCGTGTGAGTAAG
GCCCCGGAGGCCCTTTTCTTTGTGCAATTTGAGAAGGGAGAGAGCTACTTCCACAT
GCACGTGCTCGTGGAAACCACCGGGGTGAAATCCATGGTTTTGGGACGTTTCCTGA
GTCAGATTCGCGAAAAACTGATTCAGAGAATTTACCGCGGGATCGAGCCGACTTTG
CCAAACTGGTTCGCGGTCACAAAGACCAGAAATGGCGCCGGAGGCGGGAACAAG
GTGGTGGATGAGTGCTACATCCCCAATTACTTGCTCCCCAAAACCCAGCCTGAGCTC
CAGTGGGCGTGGACTAATATGGAACAGTATTTAAGCGCCTGTTTGAATCTCACGGAG
CGTAAACGGTTGGTGGCGCAGCATCTGACGCACGTGTCGCAGACGCAGGAGCAGA
ACAAAGAGAATCAGAATCCCAATTCTGATGCGCCGGTGATCAGATCAAAAACTTCA
GCCAGGTACATGGAGCTGGTCGGGTGGCTCGTGGACAAGGGGATTACCTCGGAGA
AGCAGTGGATCCAGGAGGACCAGGCCTCATACATCTCCTTCAATGCGGCCTCCAAC
TCGCGGTCCCAAATCAAGGCTGCCTTGGACAATGCGGGAAAGATTATGAGCCTGAC
TAAAACCGCCCCCGACTACCTGGTGGGCCAGCAGCCCGTGGAGGACATTTCCAGCA
ATCGGATTTATAAAATTTTGGAACTAAACGGGTACGATCCCCAATATGCGGCTTCCGT
CTTTCTGGGATGGGCCACGAAAAAGTTCGGCAAGAGGAACACCATCTGGCTGTTTG
GGCCTGCAACTACCGGGAAGACCAACATCGCGGAGGCCATAGCCCACACTGTGCCC
TTCTACGGGTGCGTAAACTGGACCAATGAGAACTTTCCCTTCAACGACTGTGTCGA
CAAGATGGTGATCTGGTGGGAGGAGGGCAAGATGACGGCCAAGGTCGTGGAGTCC
GCCAAGGCCATTCTCGGCGGCAGCAAGGTGCGCGTGGACCAAAAGTGCAAGTCGT
 
CCGCCCAGATCGATCCCACCCCCGTGATCGTCACCTCCAACACCAACATGTGCGCC
GTGATTGACGGGAACAGCACCACCTTCGAGCACCAGCAGCCGTTGCAGGACCGGA
TGTTCAAATTTGAACTCACCCGCCGTCTGGAGCATGACTTTGGCAAGGTGACAAAG
CAGGAAGTCAAAGAGTTCTTCCGCTGGGCGCAGGATCACGTGACCGAGGTGGCGC
ATGAGTTCTACGTCAGAAAGGGTGGAGCCAACAAGAGACCCGCCCCCGATGACGC
GGATAAAAGCGAGCCCAAGCGGGCCTGCCCCTCAGTCGCGGATCCATCGACGTCAG
ACGCGGAAGGAGCTCCGGTGGACTTTGCCGACAGGTACCAAAACAAATGTTCTCGT
CACGCGGGCATGCTTCAGATGCTGTTTCCCTGCAAAACATGCGAGAGAATGAATCA
GAATTTCAACATTTGCTTCACGCACGGGACCAGAGACTGTTCAGAATGTTTCCCCG
GCGTGTCAGAATCTCAACCGGTCGTCAGAAAGAGGACGTATCGGAAACTCTGTGCC
ATTCATCATCTGCTGGGGCGGGCTCCCGAGATTGCTTGCTCGGCCTGCGATCTGGTC
AACGTGGATCTGGATGACTGTGTTTCTGAGCAATAAATGACTTAAACCAGGTATGGC
TGCCGATGGTTATCTTCCAGATTGGCTCGAGGACAACCTCTCTGAGGGCATTCGCGA
GTGGTGGGACTTGAAACCTGGAGCCCCGAAACCCAAAGCCAACCAGCAAAAGCA
GGACGACGGCCGGGGTCTGGTGCTTCCTGGCTACAAGTACCTCGGACCCTTCAACG
GACTCGACAAGGGGGAGCCCGTCAACGCGGCGGATGCAGCGGCCCTCGAGCACGA
CAAGGCCTACGACCAGCAGCTCAAAGCGGGTGACAATCCGTACCTGCGGTATAACC
ACGCCGACGCCGAGTTTCAGGAGCGTCTGCAAGAAGATACGTCTTTTGGGGGCAAC
CTCGGGCGAGCAGTCTTCCAGGCCAAGAAGAGGGTTCTCGAACCTTTTGGTCTGGT
TGAGGAAGGTGCTAAGACGGCTCCTGGAAAGAAACGTCCGGTAGAGCAGTCGCCA
CAAGAGCCAGACTCCTCCTCGGGCATTGGCAAGACAGGCCAGCAGCCCGCTAAAA
AGAGACTCAATTTTGGTCAGACTGGCGACTCAGAGTCAGTCCCCGACCCACAACCT
CTCGGAGAACCTCCAGCAACCCCCGCTGCTGTGGGACCTACTACAATGGCTTCAGG
CGGTGGCGCACCAATGGCAGACAATAACGAAGGCGCCGACGGAGTGGGTAATGCC
TCAGGAAATTGGCATTGCGATTCCACATGGCTGGGCGACAGAGTCATCACCACCAG
CACCCGAACATGGGCCTTGCCCACCTATAACAACCACCTCTACAAGCAAATCTCCA
GTGCTTCAACGGGGGCCAGCAACGACAACCACTACTTCGGCTACAGCACCCCCTGG
GGGTATTTTGATTTCAACAGATTCCACTGCCATTTCTCACCACGTGACTGGCAGCGA
CTCATCAACAACAATTGGGGATTCCGGCCCAAGAGACTCAACTTCAAGCTCTTCAA
CATCCAAGTCAAGGAGGTCACGACGAATGATGGCGTCACGACCATCGCTAATAACC
TTACCAGCACGGTTCAAGTCTTCTCGGACTCGGAGTACCAGTTGCCGTACGTCCTCG
GCTCTGCGCACCAGGGCTGCCTCCCTCCGTTCCCGGCGGACGTGTTCATGATTCCGC
AGTACGGCTACCTAACGCTCAACAATGGCAGCCAGGCAGTGGGACGGTCATCCTTT
TACTGCCTGGAATATTTCCCATCGCAGATGCTGAGAACGGGCAATAACTTTACCTTC
AGCTACACCTTCGAGGACGTGCCTTTCCACAGCAGCTACGCGCACAGCCAGAGCCT
 
GGACCGGCTGATGAATCCTCTCATCGACCAGTACCTGTATTACCTGAACAGAACTCA
GAATCAGTCCGGAAGTGCCCAAAACAAGGACTTGCTGTTTAGCCGGGGGTCTCCAG
CTGGCATGTCTGTTCAGCCCAAAAACTGGCTACCTGGACCCTGTTACCGGCAGCAG
CGCGTTTCTAAAACAAAAACAGACAACAACAACAGCAACTTTACCTGGACTGGTG
CTTCAAAATATAACCTTAATGGGCGTGAATCTATAATCAACCCTGGCACTGCTATGGC
CTCACACAAAGACGACAAAGACAAGTTCTTTCCCATGAGCGGTGTCATGATTTTTG
GAAAGGAGAGCGCCGGAGCTTCAAACACTGCATTGGACAATGTCATGATCACAGA
CGAAGAGGAAATCAAAGCCACTAACCCCGTGGCCACCGAAAGATTTGGGACTGTG
GCAGTCAATCTCCAGAGCAGCAGCGGTCGTGGCGACAGCCCGACAGACCCTGCGA
CCGGAGATGTGCATGTTATGGGAGCCTTACCTGGAATGGTGTGGCAAGACAGAGAC
GTATACCTGCAGGGTCCTATTTGGGCCAAAATTCCTCACACGGATGGACACTTTCAC
CCGTCTCCTCTCATGGGCGGCTTTGGACTTAAGCACCCGCCTCCTCAGATCCTCATC
AAAAACACGCCTGTTCCTGCGAATCCTCCGGCAGAGTTTTCGGCTACAAAGTTTGC
TTCATTCATCACCCAGTATTCCACAGGACAAGTGAGCGTGGAGATTGAATGGGAGC
TGCAGAAAGAAAACAGCAAACGCTGGAATCCCGAAGTGCAGTATACATCTAACTAT
GCAAAATCTGCCAACGTTGATTTCACTGTGGACAACAATGGACTTTATACTGAGCCT
CGCCCCATTGGCACCCGTTACCTCACCCGTCCCCTGTAATTGTCTGTTAATCAATAAA
CCGGTTGATTCGTTTCAGTTGAACTTTGGTCTCGCGGCCGCTCGATAAGCTTTTGTT
CCCTTTAGTGAGGGTTAATTTCGAGCTTGGCGTAATCATGGTCATAGCTGTTTCCTGT
GTGAAATTGTTATCCGCTCACAATTCCACACAACATACGAGCCGGAAGCATAAAGTG
TAAAGCCTGGGGTGCCTAATGAGTGAGCTAACTCACATTAATTGCGTTGCGCTCACT
GCCCGCTTTCCAGTCGGGAAACCTGTCGTGCCAGCTGCATTAATGAATCGGCCAAC
GCGCGGGGAGAGGCGGTTTGCGTATTGGGCGCTCTTCCGCTTCCTCGCTCACTGAC
TCGCTGCGCTCGGTCGTTCGGCTGCGGCGAGCGGTATCAGCTCACTCAAAGGCGGT
AATACGGTTATCCACAGAATCAGGGGATAACGCAGGAAAGAACATGTGAGCAAAAG
GCCAGCAAAAGGCCAGGAACCGTAAAAAGGCCGCGTTGCTGGCGTTTTTCCATAG
GCTCCGCCCCCCTGACGAGCATCACAAAAATCGACGCTCAAGTCAGAGGTGGCGA
AACCCGACAGGACTATAAAGATACCAGGCGTTTCCCCCTGGAAGCTCCCTCGTGCG
CTCTCCTGTTCCGACCCTGCCGCTTACCGGATACCTGTCCGCCTTTCTCCCTTCGGG
AAGCGTGGCGCTTTCTCATAGCTCACGCTGTAGGTATCTCAGTTCGGTGTAGGTCGT
TCGCTCCAAGCTGGGCTGTGTGCACGAACCCCCCGTTCAGCCCGACCGCTGCGCCT
TATCCGGTAACTATCGTCTTGAGTCCAACCCGGTAAGACACGACTTATCGCCACTGG
CAGCAGCCACTGGTAACAGGATTAGCAGAGCGAGGTATGTAGGCGGTGCTACAGAG
TTCTTGAAGTGGTGGCCTAACTACGGCTACACTAGAAGAACAGTATTTGGTATCTGC
GCTCTGCTGAAGCCAGTTACCTTCGGAAAAAGAGTTGGTAGCTCTTGATCCGGCAA
 
ACAAACCACCGCTGGTAGCGGTGGTTTTTTTGTTTGCAAGCAGCAGATTACGCGCA
GAAAAAAAGGATCTCAAGAAGATCCTTTGATCTTTTCTACGGGGTCTGACGCTCAG
TGGAACGAAAACTCACGTTAAGGGATTTTGGTCATGAGATTATCAAAAAGGATCTTC
ACCTAGATCCTTTTAAATTAAAAATGAAGTTTTAAATCAATCTAAAGTATATATGAGT
AAACTTGGTCTGACAGTTACCAATGCTTAATCAGTGAGGCACCTATCTCAGCGATCT
GTCTATTTCGTTCATCCATAGTTGCCTGACTCCCCGTCGTGTAGATAACTACGATACG
GGAGGGCTTACCATCTGGCCCCAGTGCTGCAATGATACCGCGAGACCCACGCTCAC
CGGCTCCAGATTTATCAGCAATAAACCAGCCAGCCGGAAGGGCCGAGCGCAGAAG
TGGTCCTGCAACTTTATCCGCCTCCATCCAGTCTATTAATTGTTGCCGGGAAGCTAGA
GTAAGTAGTTCGCCAGTTAATAGTTTGCGCAACGTTGTTGCCATTGCTACAGGCATC
GTGGTGTCACGCTCGTCGTTTGGTATGGCTTCATTCAGCTCCGGTTCCCAACGATCA
AGGCGAGTTACATGATCCCCCATGTTGTGCAAAAAAGCGGTTAGCTCCTTCGGTCCT
CCGATCGTTGTCAGAAGTAAGTTGGCCGCAGTGTTATCACTCATGGTTATGGCAGCA
CTGCATAATTCTCTTACTGTCATGCCATCCGTAAGATGCTTTTCTGTGACTGGTGAGT
ACTCAACCAAGTCATTCTGAGAATAGTGTATGCGGCGACCGAGTTGCTCTTGCCCG
GCGTCAATACGGGATAATACCGCGCCACATAGCAGAACTTTAAAAGTGCTCATCATT
GGAAAACGTTCTTCGGGGCGAAAACTCTCAAGGATCTTACCGCTGTTGAGATCCAG
TTCGATGTAACCCACTCGTGCACCCAACTGATCTTCAGCATCTTTTACTTTCACCAG
CGTTTCTGGGTGAGCAAAAACAGGAAGGCAAAATGCCGCAAAAAAGGGAATAAGG
GCGACACGGAAATGTTGAATACTCATACTCTTCCTTTTTCAATATTATTGAAGCATTTA
TCAGGGTTATTGTCTCATGAGCGGATACATATTTGAATGTATTTAGAAAAATAAACAA
ATAGGGGTTCCGCGCACATTTCCCCGAAAAGTGCCACCTGACGTCTAAGAAACCAT
TATTATCATGACATTAACCTATAAAAATAGGCGTATCACGAGGCCCTTTCGTCTCGCG
CGTTTCGGTGATGACGGTGAAAACCTCTGACACATGCAGCTCCCGGAGACGGTCAC
AGCTTGTCTGTAAGCGGATGCCGGGAGCAGACAAGCCCGTCAGGGCGCGTCAGCG
GGTGTTGGCGGGTGTCGGGGCTGGCTTAACTATGCGGCATCAGAGCAGATTGTACT
GAGAGTGCACCATATGCGGTGTGAAATACCGCACAGATGCGTAAGGAGAAAATACC
GCATCAGGAAATTGTAAGCGTTAATATTTTGTTAAAATTCGCGTTAAATTTTTGTTAA
ATCAGCTCATTTTTTAACCAATAGGCCGAAATCGGCAAAATCCCTTATAAATCAAAA
GAATAGACCGAGAT
SEQ ID NO: 11 (AAV9 VP1 gene sequence): 
ATGGCTGCCGATGGTTATCTTCCAGATTGGCTCGAGGACAACCTTAGTGAAGGA
ATTCGCGAGTGGTGGGCTTTGAAACCTGGAGCCCCTCAACCCAAGGCAAATCAACA
ACATCAAGACAACGCTCGAGGTCTTGTGCTTCCGGGTTACAAATACCTTGGACCCG
GCAACGGACTCGACAAGGGGGAGCCGGTCAACGCAGCAGACGCGGCGGCCCTCG
AGCACGACAAGGCCTACGACCAGCAGCTCAAGGCCGGAGACAACCCGTACCTCAA
GTACAACCACGCCGACGCCGAGTTCCAGGAGCGGCTCAAAGAAGATACGTCTTTTG
GGGGCAACCTCGGGCGAGCAGTCTTCCAGGCCAAAAAGAGGCTTCTTGAACCTCT
TGGTCTGGTTGAGGAAGCGGCTAAGACGGCTCCTGGAAAGAAGAGGCCTGTAGAG
CAGTCTCCTCAGGAACCGGACTCCTCCGCGGGTATTGGCAAATCGGGTGCACAGCC
CGCTAAAAAGAGACTCAATTTCGGTCAGACTGGCGACACAGAGTCAGTCCCAGAC
CCTCAACCAATCGGAGAACCTCCCGCAGCCCCCTCAGGTGTGGGATCTCTTACAAT
GGCTTCAGGTGGTGGCGCACCAGTGGCAGACAATAACGAAGGTGCCGATGGAGTG
GGTAGTTCCTCGGGAAATTGGCATTGCGATTCCCAATGGCTGGGGGACAGAGTCAT
CACCACCAGCACCCGAACCTGGGCCCTGCCCACCTACAACAATCACCTCTACAAGC
AAATCTCCAACAGCACATCTGGAGGATCTTCAAATGACAACGCCTACTTCGGCTAC
AGCACCCCCTGGGGGTATTTTGACTTCAACAGATTCCACTGCCACTTCTCACCACGT
GACTGGCAGCGACTCATCAACAACAACTGGGGATTCCGGCCTAAGCGACTCAACTT
CAAGCTCTTCAACATTCAGGTCAAAGAGGTTACGGACAACAATGGAGTCAAGACCA
TCGCCAATAACCTTACCAGCACGGTCCAGGTCTTCACGGACTCAGACTATCAGCTCC
CGTACGTGCTCGGGTCGGCTCACGAGGGCTGCCTCCCGCCGTTCCCAGCGGACGTT
TTCATGATTCCTCAGTACGGGTATCTGACGCTTAATGATGGAAGCCAGGCCGTGGGT
CGTTCGTCCTTTTACTGCCTGGAATATTTCCCGTCGCAAATGCTAAGAACGGGTAAC
AACTTCCAGTTCAGCTACGAGTTTGAGAACGTACCTTTCCATAGCAGCTACGCTCAC
AGCCAAAGCCTGGACCGACTAATGAATCCACTCATCGACCAATACTTGTACTATCTC
TCAAAGACTATTAACGGTTCTGGACAGAATCAACAAACGCTAAAATTCAGTGTGGC
CGGACCCAGCAACATGGCTGTCCAGGGAAGAAACTACATACCTGGACCCAGCTACC
GACAACAACGTGTCTCAACCACTGTGACTCAAAACAACAACAGCGAATTTGCTTG
GCCTGGAGCTTCTTCTTGGGCTCTCAATGGACGTAATAGCTTGATGAATCCTGGACC
TGCTATGGCCAGCCACAAAGAAGGAGAGGACCGTTTCTTTCCTTTGTCTGGATCTTT
AATTTTTGGCAAACAAGGAACTGGAAGAGACAACGTGGATGCGGACAAAGTCATG
ATAACCAACGAAGAAGAAATTAAAACTACTAACCCGGTAGCAACGGAGTCCTATGG
ACAAGTGGCCACAAACCACCAGAGTGCCCAAGCACAGGCGCAGACCGGCTGGGTT
CAAAACCAAGGAATACTTCCGGGTATGGTTTGGCAGGACAGAGATGTGTACCTGCA
AGGACCCATTTGGGCCAAAATTCCTCACACGGACGGCAACTTTCACCCTTCTCCGC
 
TGATGGGAGGGTTTGGAATGAAGCACCCGCCTCCTCAGATCCTCATCAAAAACACA
CCTGTACCTGCGGATCCTCCAACGGCCTTCAACAAGGACAAGCTGAACTCTTTCAT
CACCCAGTATTCTACTGGCCAAGTCAGCGTGGAGATCGAGTGGGAGCTGCAGAAGG
AAAACAGCAAGCGCTGGAACCCGGAGATCCAGTACACTTCCAACTATTACAAGTCT
AATAATGTTGAATTTGCTGTTAATACTGAAGGTGTATATAGTGAACCCCGCCCCATTG
GCACCAGATACCTGACTCGTAATCTGTAA
SEQ ID NO: 12 (nucleotide sequence of RC9.2 plasmid): 
TAGGGTTGAGTGTTGTTCCAGTTTGGAACAAGAGTCCACTATTAAAGAACGTG
GACTCCAACGTCAAAGGGCGAAAAACCGTCTATCAGGGCGATGGCCCACTACGTG
AACCATCACCCTAATCAAGTTTTTTGGGGTCGAGGTGCCGTAAAGCACTAAATCGG
AACCCTAAAGGGAGCCCCCGATTTAGAGCTTGACGGGGAAAGCCGGCGAACGTGG
CGAGAAAGGAAGGGAAGAAAGCGAAAGGAGCGGGCGCTAGGGCGCTGGCAAGTG
TAGCGGTCACGCTGCGCGTAACCACCACACCCGCCGCGCTTAATGCGCCGCTACAG
GGCGCGTCCcATTCGCCATTCAGGCTGCGCAACTGTTGGGAAGGGCGATCGGTGCG
GGCCTCTTCGCTATTACGCCAGCTGGCGAAAGGGGGATGTGCTGCAAGGCGATTAA
GTTGGGTAACGCCAGGGTTTTCCCAGTCACGACGTTGTAAAACGACGGCCAGTGAG
CGCGCGTAATACGACTCACTATAGGGCGAATTGGGTACCGGGCCCCCCCTCGATCGA
GGTCGACGGTATCGGGGGAGCTCGCAGGGTCTCCATTTTGAAGCGGGAGGTTTGAA
CGCGCAGCCGCCATGCCGGGGTTTTACGAGATTGTGATTAAGGTCCCCAGCGACCT
TGACGAGCATCTGCCCGGCATTTCTGACAGCTTTGTGAACTGGGTGGCCGAGAAGG
AATGGGAGTTGCCGCCAGATTCTGACATGGATCTGAATCTGATTGAGCAGGCACCC
CTGACCGTGGCCGAGAAGCTGCAGCGCGACTTTCTGACGGAATGGCGCCGTGTGA
GTAAGGCCCCGGAGGCTCTTTTCTTTGTGCAATTTGAGAAGGGAGAGAGCTACTTC
CACATGCACGTGCTCGTGGAAACCACCGGGGTGAAATCCATGGTTTTGGGACGTTT
CCTGAGTCAGATTCGCGAAAAACTGATTCAGAGAATTTACCGCGGGATCGAGCCGA
CTTTGCCAAACTGGTTCGCGGTCACAAAGACCAGAAATGGCGCCGGAGGCGGGAA
CAAGGTGGTGGATGAGTGCTACATCCCCAATTACTTGCTCCCCAAAACCCAGCCTG
AGCTCCAGTGGGCGTGGACTAATATGGAACAGTATTTAAGCGCCTGTTTGAATCTCA
CGGAGCGTAAACGGTTGGTGGCGCAGCATCTGACGCACGTGTCGCAGACGCAGGA
GCAGAACAAAGAGAATCAGAATCCCAATTCTGATGCGCCGGTGATCAGATCAAAAA
CTTCAGCCAGGTACATGGAGCTGGTCGGGTGGCTCGTGGACAAGGGGATTACCTCG
GAGAAGCAGTGGATCCAGGAGGACCAGGCCTCATACATCTCCTTCAATGCGGCCTC
CAACTCGCGGTCCCAAATCAAGGCTGCCTTGGACAATGCGGGAAAGATTATGAGCC
TGACTAAAACCGCCCCCGACTACCTGGTGGGCCAGCAGCCCGTGGAGGACATTTCC
AGCAATCGGATTTATAAAATTTTGGAACTAAACGGGTACGATCCCCAATATGCGGCT
TCCGTCTTTCTGGGATGGGCCACGAAAAAGTTCGGCAAGAGGAACACCATCTGGCT
GTTTGGGCCTGCAACTACCGGGAAGACCAACATCGCGGAGGCCATAGCCCACACTG
TGCCCTTCTACGGGTGCGTAAACTGGACCAATGAGAACTTTCCCTTCAACGACTGT
GTCGACAAGATGGTGATCTGGTGGGAGGAGGGGAAGATGACCGCCAAGGTCGTGG
AGTCGGCCAAAGCCATTCTCGGAGGAAGCAAGGTGCGCGTGGACCAGAAATGCAA
GTCCTCGGCCCAGATAGACCCGACTCCCGTGATCGTCACCTCCAACACCAACATGT
 
GCGCCGTGATTGACGGGAACTCAACGACCTTCGAACACCAGCAGCCGTTGCAAGA
CCGGATGTTCAAATTTGAACTCACCCGCCGTCTGGATCATGACTTTGGGAAGGTCAC
CAAGCAGGAAGTCAAAGACTTTTTCCGGTGGGCAAAGGATCACGTGGTTGAGGTG
GAGCATGAATTCTACGTCAAAAAGGGTGGAGCCAAGAAAAGACCCGCCCCCAGTG
ACGCAGATATAAGTGAGCCCAAACGGGTGCGCGAGTCAGTTGCGCAGCCATCGACG
TCAGACGCGGAAGCTTCGATCAACTACGCAGACAGGTACCAAAACAAATGTTCTCG
TCACGTGGGCATGAATCTGATGCTGTTTCCCTGCAGACAATGCGAGAGAATGAATC
AGAATTCAAATATCTGCTTCACTCACGGACAGAAAGACTGTTTAGAGTGCTTTCCCG
TGTCAGAATCTCAACCCGTTTCTGTCGTCAAAAAGGCGTATCAGAAACTGTGCTACA
TTCATCATATCATGGGAAAGGTGCCAGACGCTTGCACTGCCTGCGATCTGGTCAATG
TGGATTTGGATGACTGCATCTTTGAACAATAAATGATTTAAATCAGGTATGGCTGCCG
ATGGTTATCTTCCAGATTGGCTCGAGGACAACCTTAGTGAAGGAATTCGCGAGTGGT
GGGCTTTGAAACCTGGAGCCCCTCAACCCAAGGCAAATCAACAACATCAAGACAA
CGCTCGAGGTCTTGTGCTTCCGGGTTACAAATACCTTGGACCCGGCAACGGACTCG
ACAAGGGGGAGCCGGTCAACGCAGCAGACGCGGCGGCCCTCGAGCACGACAAGG
CCTACGACCAGCAGCTCAAGGCCGGAGACAACCCGTACCTCAAGTACAACCACGC
CGACGCCGAGTTCCAGGAGCGGCTCAAAGAAGATACGTCTTTTGGGGGCAACCTC
GGGCGAGCAGTCTTCCAGGCCAAAAAGAGGCTTCTTGAACCTCTTGGTCTGGTTGA
GGAAGCGGCTAAGACGGCTCCTGGAAAGAAGAGGCCTGTAGAGCAGTCTCCTCAG
GAACCGGACTCCTCCGCGGGTATTGGCAAATCGGGTGCACAGCCCGCTAAAAAGA
GACTCAATTTCGGTCAGACTGGCGACACAGAGTCAGTCCCAGACCCTCAACCAATC
GGAGAACCTCCCGCAGCCCCCTCAGGTGTGGGATCTCTTACAATGGCTTCAGGTGG
TGGCGCACCAGTGGCAGACAATAACGAAGGTGCCGATGGAGTGGGTAGTTCCTCG
GGAAATTGGCATTGCGATTCCCAATGGCTGGGGGACAGAGTCATCACCACCAGCAC
CCGAACCTGGGCCCTGCCCACCTACAACAATCACCTCTACAAGCAAATCTCCAACA
GCACATCTGGAGGATCTTCAAATGACAACGCCTACTTCGGCTACAGCACCCCCTGG
GGGTATTTTGACTTCAACAGATTCCACTGCCACTTCTCACCACGTGACTGGCAGCGA
CTCATCAACAACAACTGGGGATTCCGGCCTAAGCGACTCAACTTCAAGCTCTTCAA
CATTCAGGTCAAAGAGGTTACGGACAACAATGGAGTCAAGACCATCGCCAATAACC
TTACCAGCACGGTCCAGGTCTTCACGGACTCAGACTATCAGCTCCCGTACGTGCTCG
GGTCGGCTCACGAGGGCTGCCTCCCGCCGTTCCCAGCGGACGTTTTCATGATTCCTC
AGTACGGGTATCTGACGCTTAATGATGGAAGCCAGGCCGTGGGTCGTTCGTCCTTTT
ACTGCCTGGAATATTTCCCGTCGCAAATGCTAAGAACGGGTAACAACTTCCAGTTCA
GCTACGAGTTTGAGAACGTACCTTTCCATAGCAGCTACGCTCACAGCCAAAGCCTG
GACCGACTAATGAATCCACTCATCGACCAATACTTGTACTATCTCTCAAAGACTATTA
 
ACGGTTCTGGAGGTCGTGGCGACAGCCCGCAGAATCAACAAACGCTAAAATTCAGT
GTGGCCGGACCCAGCAACATGGCTGTCCAGGGAAGAAACTACATACCTGGACCCA
GCTACCGACAACAACGTGTCTCAACCACTGTGACTCAAAACAACAACAGCGAATTT
GCTTGGCCTGGAGCTTCTTCTTGGGCTCTCAATGGACGTAATAGCTTGATGAATCCT
GGACCTGCTATGGCCAGCCACAAAGAAGGAGAGGACCGTTTCTTTCCTTTGTCTGG
ATCTTTAATTTTTGGCAAACAAGGAACTGGAAGAGACAACGTGGATGCGGACAAAG
TCATGATAACCAACGAAGAAGAAATTAAAACTACTAACCCGGTAGCAACGGAGTCC
TATGGACAAGTGGCCACAAACCACCAGAGTGCCCAAGCACAGGCGCAGACCGGCT
GGGTTCAAAACCAAGGAATACTTCCGGGTATGGTTTGGCAGGACAGAGATGTGTAC
CTGCAAGGACCCATTTGGGCCAAAATTCCTCACACGGACGGCAACTTTCACCCTTC
TCCGCTGATGGGAGGGTTTGGAATGAAGCACCCGCCTCCTCAGATCCTCATCAAAA
ACACACCTGTACCTGCGGATCCTCCAACGGCCTTCAACAAGGACAAGCTGAACTCT
TTCATCACCCAGTATTCTACTGGCCAAGTCAGCGTGGAGATCGAGTGGGAGCTGCA
GAAGGAAAACAGCAAGCGCTGGAACCCGGAGATCCAGTACACTTCCAACTATTACA
AGTCTAATAATGTTGAATTTGCTGTTAATACTGAAGGTGTATATAGTGAACCCCGCCC
CATTGGCACCAGATACCTGACTCGTAATCTGTAATTGCTTGTTAATCAATAAACCGTT
TAATTCGTTTCAGTTGAACTTTGGTCTCTGCGAAGGGCGAATTCGTTTAAACCTGCA
GGACTAGAGGTCCTGTATTAGAGGTCACGTGAGTGTTTTGCGACATTTTGCGACACC
ATGTGGTCACGCTGGGTATTTAAGCCCGAGTGAGCACGCAGGGTCTCCATTTTGAA
GCGGGAGGTTTGAACGCGCAGCCGCCAAGCCGAATTCTGCAGATATCCATCACACT
GGCGGCCGCTCGACTAGAGCGGCCGCCACCGCGGTGGAGCTCCAGCTTTTGTTCCC
TTTAGTGAGGGTTAATTGCGCGCTTGGCGTAATCATGGTCATAGCTGTTTCCTGTGTG
AAATTGTTATCCGCTCACAATTCCACACAACATACGAGCCGGAAGCATAAAGTGTAA
AGCCTGGGGTGCCTAATGAGTGAGCTAACTCACATTAATTGCGTTGCGCTCACTGCC
CGCTTTCCAGTCGGGAAACCTGTCGTGCCAGCTGCATTAATGAATCGGCCAACGCG
CGGGGAGAGGCGGTTTGCGTATTGGGCGCTCTTCCGCTTCCTCGCTCACTGACTCG
CTGCGCTCGGTCGTTCGGCTGCGGCGAGCGGTATCAGCTCACTCAAAGGCGGTAAT
ACGGTTATCCACAGAATCAGGGGATAACGCAGGAAAGAACATGTGAGCAAAAGGC
CAGCAAAAGGCCAGGAACCGTAAAAAGGCCGCGTTGCTGGCGTTTTTCCATAGGCT
CCGCCCCCCTGACGAGCATCACAAAAATCGACGCTCAAGTCAGAGGTGGCGAAAC
CCGACAGGACTATAAAGATACCAGGCGTTTCCCCCTGGAAGCTCCCTCGTGCGCTCT
CCTGTTCCGACCCTGCCGCTTACCGGATACCTGTCCGCCTTTCTCCCTTCGGGAAGC
GTGGCGCTTTCTCATAGCTCACGCTGTAGGTATCTCAGTTCGGTGTAGGTCGTTCGC
TCCAAGCTGGGCTGTGTGCACGAACCCCCCGTTCAGCCCGACCGCTGCGCCTTATC
CGGTAACTATCGTCTTGAGTCCAACCCGGTAAGACACGACTTATCGCCACTGGCAG
 
CAGCCACTGGTAACAGGATTAGCAGAGCGAGGTATGTAGGCGGTGCTACAGAGTTC
TTGAAGTGGTGGCCTAACTACGGCTACACTAGAAGAACAGTATTTGGTATCTGCGCT
CTGCTGAAGCCAGTTACCTTCGGAAAAAGAGTTGGTAGCTCTTGATCCGGCAAACA
AACCACCGCTGGTAGCGGTGGTTTTTTTGTTTGCAAGCAGCAGATTACGCGCAGAA
AAAAAGGATCTCAAGAAGATCCTTTGATCTTTTCTACGGGGTCTGACGCTCAGTGG
AACGAAAACTCACGTTAAGGGATTTTGGTCATGAGATTATCAAAAAGGATCTTCACC
TAGATCCTTTTAAATTAAAAATGAAGTTTTAAATCAATCTAAAGTATATATGAGTAAA
CTTGGTCTGACAGTTACCAATGCTTAATCAGTGAGGCACCTATCTCAGCGATCTGTC
TATTTCGTTCATCCATAGTTGCCTGACTCCCCGTCGTGTAGATAACTACGATACGGGA
GGGCTTACCATCTGGCCCCAGTGCTGCAATGATACCGCGAGACCCACGCTCACCGG
CTCCAGATTTATCAGCAATAAACCAGCCAGCCGGAAGGGCCGAGCGCAGAAGTGGT
CCTGCAACTTTATCCGCCTCCATCCAGTCTATTAATTGTTGCCGGGAAGCTAGAGTAA
GTAGTTCGCCAGTTAATAGTTTGCGCAACGTTGTTGCCATTGCTACAGGCATCGTGG
TGTCACGCTCGTCGTTTGGTATGGCTTCATTCAGCTCCGGTTCCCAACGATCAAGGC
GAGTTACATGATCCCCCATGTTGTGCAAAAAAGCGGTTAGCTCCTTCGGTCCTCCGA
TCGTTGTCAGAAGTAAGTTGGCCGCAGTGTTATCACTCATGGTTATGGCAGCACTGC
ATAATTCTCTTACTGTCATGCCATCCGTAAGATGCTTTTCTGTGACTGGTGAGTACTC
AACCAAGTCATTCTGAGAATAGTGTATGCGGCGACCGAGTTGCTCTTGCCCGGCGT
CAATACGGGATAATACCGCGCCACATAGCAGAACTTTAAAAGTGCTCATCATTGGAA
AACGTTCTTCGGGGCGAAAACTCTCAAGGATCTTACCGCTGTTGAGATCCAGTTCG
ATGTAACCCACTCGTGCACCCAACTGATCTTCAGCATCTTTTACTTTCACCAGCGTTT
CTGGGTGAGCAAAAACAGGAAGGCAAAATGCCGCAAAAAAGGGAATAAGGGCGA
CACGGAAATGTTGAATACTCATACTCTTCCTTTTTCAATATTATTGAAGCATTTATCAG
GGTTATTGTCTCATGAGCGGATACATATTTGAATGTATTTAGAAAAATAAACAAATAG
GGGTTCCGCGCACATTTCCCCGAAAAGTGCCACCTAAATTGTAAGCGTTAATATTTT
GTTAAAATTCGCGTTAAATTTTTGTTAAATCAGCTCATTTTTTAACCAATAGGCCGAA
ATCGGCAAAATCCCTTATAAATCAAAAGAATAGACCGAGA
SEQ ID NO: 13 (nucleotide sequence of RC9.3 plasmid):

CTGTTGGGAAGGGCGATCGGTGCGGGCCTCTTCGCTATTACGCCAGCTGGCGA
AAGGGGGATGTGCTGCAAGGCGATTAAGTTGGGTAACGCCAGGGTTTTCCCAGTCA
CGACGTTGTAAAACGACGGCCAGTGAGCGCGCGTAATACGACTCACTATAGGGCGA
ATTGGGTACCGGGCCCCCCCTCGATCGAGGTCGACGGTATCGGGGGAGCTCGCAGG
GTCTCCATTTTGAAGCGGGAGGTTTGAACGCGCAGCCGCCATGCCGGGGTTTTACG
AGATTGTGATTAAGGTCCCCAGCGACCTTGACGAGCATCTGCCCGGCATTTCTGACA
GCTTTGTGAACTGGGTGGCCGAGAAGGAATGGGAGTTGCCGCCAGATTCTGACATG
GATCTGAATCTGATTGAGCAGGCACCCCTGACCGTGGCCGAGAAGCTGCAGCGCGA
CTTTCTGACGGAATGGCGCCGTGTGAGTAAGGCCCCGGAGGCTCTTTTCTTTGTGC
AATTTGAGAAGGGAGAGAGCTACTTCCACATGCACGTGCTCGTGGAAACCACCGG
GGTGAAATCCATGGTTTTGGGACGTTTCCTGAGTCAGATTCGCGAAAAACTGATTC
AGAGAATTTACCGCGGGATCGAGCCGACTTTGCCAAACTGGTTCGCGGTCACAAAG
ACCAGAAATGGCGCCGGAGGCGGGAACAAGGTGGTGGATGAGTGCTACATCCCCA
ATTACTTGCTCCCCAAAACCCAGCCTGAGCTCCAGTGGGCGTGGACTAATATGGAA
CAGTATTTAAGCGCCTGTTTGAATCTCACGGAGCGTAAACGGTTGGTGGCGCAGCAT
CTGACGCACGTGTCGCAGACGCAGGAGCAGAACAAAGAGAATCAGAATCCCAATT
CTGATGCGCCGGTGATCAGATCAAAAACTTCAGCCAGGTACATGGAGCTGGTCGGG
TGGCTCGTGGACAAGGGGATTACCTCGGAGAAGCAGTGGATCCAGGAGGACCAGG
CCTCATACATCTCCTTCAATGCGGCCTCCAACTCGCGGTCCCAAATCAAGGCTGCCT
TGGACAATGCGGGAAAGATTATGAGCCTGACTAAAACCGCCCCCGACTACCTGGTG
GGCCAGCAGCCCGTGGAGGACATTTCCAGCAATCGGATTTATAAAATTTTGGAACTA
AACGGGTACGATCCCCAATATGCGGCTTCCGTCTTTCTGGGATGGGCCACGAAAAA
GTTCGGCAAGAGGAACACCATCTGGCTGTTTGGGCCTGCAACTACCGGGAAGACC
AACATCGCGGAGGCCATAGCCCACACTGTGCCCTTCTACGGGTGCGTAAACTGGAC
CAATGAGAACTTTCCCTTCAACGACTGTGTCGACAAGATGGTGATCTGGTGGGAGG
AGGGGAAGATGACCGCCAAGGTCGTGGAGTCGGCCAAAGCCATTCTCGGAGGAAG
CAAGGTGCGCGTGGACCAGAAATGCAAGTCCTCGGCCCAGATAGACCCGACTCCC
GTGATCGTCACCTCCAACACCAACATGTGCGCCGTGATTGACGGGAACTCAACGAC
CTTCGAACACCAGCAGCCGTTGCAAGACCGGATGTTCAAATTTGAACTCACCCGCC
GTCTGGATCATGACTTTGGGAAGGTCACCAAGCAGGAAGTCAAAGACTTTTTCCGG
TGGGCAAAGGATCACGTGGTTGAGGTGGAGCATGAATTCTACGTCAAAAAGGGTG
GAGCCAAGAAAAGACCCGCCCCCAGTGACGCAGATATAAGTGAGCCCAAACGGGT
GCGCGAGTCAGTTGCGCAGCCATCGACGTCAGACGCGGAAGCTTCGATCAACTACG
CGGACAGGTACCAAAACAAATGTTCTCGTCACGTGGGCATGAATCTGATGCTGTTTC

CCTGCAGACAATGCGAGAGACTGAATCAGAATTCAAATATCTGCTTCACTCACGGT
GTCAAAGACTGTTTAGAGTGCTTTCCCGTGTCAGAATCTCAACCCGTTTCTGTCGTC
AAAAAGGCGTATCAGAAACTGTGCTACATTCATCACATCATGGGAAAGGTGCCAGA
CGCTTGCACTGCTTGCGACCTGGTCAATGTGGACTTGGATGACTGTGTTTCTGAACA
ATAAATGACTTAAACCAGGTATGGCTGCCGATGGTTATCTTCCAGATTGGCTCGAGG
ACAACCTTAGTGAAGGAATTCGCGAGTGGTGGGCTTTGAAACCTGGAGCCCCTCAA
CCCAAGGCAAATCAACAACATCAAGACAACGCTCGAGGTCTTGTGCTTCCGGGTTA
CAAATACCTTGGACCCGGCAACGGACTCGACAAGGGGGAGCCGGTCAACGCAGCA
GACGCGGCGGCCCTCGAGCACGACAAGGCCTACGACCAGCAGCTCAAGGCCGGAG
ACAACCCGTACCTCAAGTACAACCACGCCGACGCCGAGTTCCAGGAGCGGCTCAA
AGAAGATACGTCTTTTGGGGGCAACCTCGGGCGAGCAGTCTTCCAGGCCAAAAAG
AGGCTTCTTGAACCTCTTGGTCTGGTTGAGGAAGCGGCTAAGACGGCTCCTGGAAA
GAAGAGGCCTGTAGAGCAGTCTCCTCAGGAACCGGACTCCTCCGCGGGTATTGGCA
AATCGGGTGCACAGCCCGCTAAAAAGAGACTCAATTTCGGTCAGACTGGCGACAC
AGAGTCAGTCCCAGACCCTCAACCAATCGGAGAACCTCCCGCAGCCCCCTCAGGT
GTGGGATCTCTTACAATGGCTTCAGGTGGTGGCGCACCAGTGGCAGACAATAACGA
AGGTGCCGATGGAGTGGGTAGTTCCTCGGGAAATTGGCATTGCGATTCCCAATGGCT
GGGGGACAGAGTCATCACCACCAGCACCCGAACCTGGGCCCTGCCCACCTACAAC
AATCACCTCTACAAGCAAATCTCCAACAGCACATCTGGAGGATCTTCAAATGACAA
CGCCTACTTCGGCTACAGCACCCCCTGGGGGTATTTTGACTTCAACAGATTCCACTG
CCACTTCTCACCACGTGACTGGCAGCGACTCATCAACAACAACTGGGGATTCCGGC
CTAAGCGACTCAACTTCAAGCTCTTCAACATTCAGGTCAAAGAGGTTACGGACAAC
AATGGAGTCAAGACCATCGCCAATAACCTTACCAGCACGGTCCAGGTCTTCACGGA
CTCAGACTATCAGCTCCCGTACGTGCTCGGGTCGGCTCACGAGGGCTGCCTCCCGC
CGTTCCCAGCGGACGTTTTCATGATTCCTCAGTACGGGTATCTGACGCTTAATGATG
GAAGCCAGGCCGTGGGTCGTTCGTCCTTTTACTGCCTGGAATATTTCCCGTCGCAAA
TGCTAAGAACGGGTAACAACTTCCAGTTCAGCTACGAGTTTGAGAACGTACCTTTC
CATAGCAGCTACGCTCACAGCCAAAGCCTGGACCGACTAATGAATCCACTCATCGA
CCAATACTTGTACTATCTCTCAAAGACTATTAACGGTTCTGGACAGAATCAACAAAC
GCTAAAATTCAGTGTGGCCGGACCCAGCAACATGGCTGTCCAGGGAAGAAACTACA
TACCTGGACCCAGCTACCGACAACAACGTGTCTCAACCACTGTGACTCAAAACAAC
AACAGCGAATTTGCTTGGCCTGGAGCTTCTTCTTGGGCTCTCAATGGACGTAATAGC
TTGATGAATCCTGGACCTGCTATGGCCAGCCACAAAGAAGGAGAGGACCGTTTCTT
TCCTTTGTCTGGATCTTTAATTTTTGGCAAACAAGGAACTGGAAGAGACAACGTGG
ATGCGGACAAAGTCATGATAACCAACGAAGAAGAAATTAAAACTACTAACCCGGTA

GCAACGGAGTCCTATGGACAAGTGGCCACAAACCACCAGAGTGCCCAAGGTCGTG
GCGACAGCCCGGCACAGGCGCAGACCGGCTGGGTTCAAAACCAAGGAATACTTCC
GGGTATGGTTTGGCAGGACAGAGATGTGTACCTGCAAGGACCCATTTGGGCCAAAA
TTCCTCACACGGACGGCAACTTTCACCCTTCTCCGCTGATGGGAGGGTTTGGAATG
AAGCACCCGCCTCCTCAGATCCTCATCAAAAACACACCTGTACCTGCGGATCCTCC
AACGGCCTTCAACAAGGACAAGCTGAACTCTTTCATCACCCAGTATTCTACTGGCC
AAGTCAGCGTGGAGATCGAGTGGGAGCTGCAGAAGGAAAACAGCAAGCGCTGGA
ACCCGGAGATCCAGTACACTTCCAACTATTACAAGTCTAATAATGTTGAATTTGCTGT
TAATACTGAAGGTGTATATAGTGAACCCCGCCCCATTGGCACCAGATACCTGACTCG
TAATCTGTAATTGCTTGTTAATCAATAAACCGTTTAATTCGTTTCAGTTGAACTTTGG
TCTCTGCGAAGGGCGAATTCGTTTAAACCTGCAGGACTAGAGGTCCTGTATTAGAG
GTCACGTGAGTGTTTTGCGACATTTTGCGACACCATGTGGTCACGCTGGGTATTTAA
GCCCGAGTGAGCACGCAGGGTCTCCATTTTGAAGCGGGAGGTTTGAACGCGCAGC
CGCCAAGCCGAATTCTGCAGATATCCATCACACTGGCGGCCGCTCGACTAGAGCGG
CCGCCACCGCGGTGGAGCTCCAGCTTTTGTTCCCTTTAGTGAGGGTTAATTGCGCGC
TTGGCGTAATCATGGTCATAGCTGTTTCCTGTGTGAAATTGTTATCCGCTCACAATTC
CACACAACATACGAGCCGGAAGCATAAAGTGTAAAGCCTGGGGTGCCTAATGAGTG
AGCTAACTCACATTAATTGCGTTGCGCTCACTGCCCGCTTTCCAGTCGGGAAACCTG
TCGTGCCAGCTGCATTAATGAATCGGCCAACGCGCGGGGAGAGGCGGTTTGCGTAT
TGGGCGCTCTTCCGCTTCCTCGCTCACTGACTCGCTGCGCTCGGTCGTTCGGCTGCG
GCGAGCGGTATCAGCTCACTCAAAGGCGGTAATACGGTTATCCACAGAATCAGGGG
ATAACGCAGGAAAGAACATGTGAGCAAAAGGCCAGCAAAAGGCCAGGAACCGTA
AAAAGGCCGCGTTGCTGGCGTTTTTCCATAGGCTCCGCCCCCCTGACGAGCATCAC
AAAAATCGACGCTCAAGTCAGAGGTGGCGAAACCCGACAGGACTATAAAGATACC
AGGCGTTTCCCCCTGGAAGCTCCCTCGTGCGCTCTCCTGTTCCGACCCTGCCGCTTA
CCGGATACCTGTCCGCCTTTCTCCCTTCGGGAAGCGTGGCGCTTTCTCATAGCTCAC
GCTGTAGGTATCTCAGTTCGGTGTAGGTCGTTCGCTCCAAGCTGGGCTGTGTGCACG
AACCCCCCGTTCAGCCCGACCGCTGCGCCTTATCCGGTAACTATCGTCTTGAGTCCA
ACCCGGTAAGACACGACTTATCGCCACTGGCAGCAGCCACTGGTAACAGGATTAGC
AGAGCGAGGTATGTAGGCGGTGCTACAGAGTTCTTGAAGTGGTGGCCTAACTACGG
CTACACTAGAAGAACAGTATTTGGTATCTGCGCTCTGCTGAAGCCAGTTACCTTCGG
AAAAAGAGTTGGTAGCTCTTGATCCGGCAAACAAACCACCGCTGGTAGCGGTGGTT
TTTTTGTTTGCAAGCAGCAGATTACGCGCAGAAAAAAAGGATCTCAAGAAGATCCT
TTGATCTTTTCTACGGGGTCTGACGCTCAGTGGAACGAAAACTCACGTTAAGGGAT
TTTGGTCATGAGATTATCAAAAAGGATCTTCACCTAGATCCTTTTAAATTAAAAATGA

AGTTTTAAATCAATCTAAAGTATATATGAGTAAACTTGGTCTGACAGTTACCAATGCT
TAATCAGTGAGGCACCTATCTCAGCGATCTGTCTATTTCGTTCATCCATAGTTGCCTG
ACTCCCCGTCGTGTAGATAACTACGATACGGGAGGGCTTACCATCTGGCCCCAGTGC
TGCAATGATACCGCGAGACCCACGCTCACCGGCTCCAGATTTATCAGCAATAAACCA
GCCAGCCGGAAGGGCCGAGCGCAGAAGTGGTCCTGCAACTTTATCCGCCTCCATCC
AGTCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTATGG
CTTCATTCAGCTCCGGTTCCCAACGATCAAGGCGAGTTACATGATCCCCCATGTTGT
GCAAAAAAGCGGTTAGCTCCTTCGGTCCTCCGATCGTTGTCAGAAGTAAGTTGGCC
GCAGTGTTATCACTCATGGTTATGGCAGCACTGCATAATTCTCTTACTGTCATGCCAT
CCGTAAGATGCTTTTCTGTGACTGGTGAGTACTCAACCAAGTCATTCTGAGAATAGT
GTATGCGGCGACCGAGTTGCTCTTGCCCGGCGTCAATACGGGATAATACCGCGCCAC
ATAGCAGAACTTTAAAAGTGCTCATCATTGGAAAACGTTCTTCGGGGCGAAAACTC
TCAAGGATCTTACCGCTGTTGAGATCCAGTTCGATGTAACCCACTCGTGCACCCAAC
TGATCTTCAGCATCTTTTACTTTCACCAGCGTTTCTGGGTGAGCAAAAACAGGAAG
GCAAAATGCCGCAAAAAAGGGAATAAGGGCGACACGGAAATGTTGAATACTCATAC
TCTTCCTTTTTCAATATTATTGAAGCATTTATCAGGGTTATTGTCTCATGAGCGGATAC
ATATTTGAATGTATTTAGAAAAATAAACAAATAGGGGTTCCGCGCACATTTCCCCGA
AAAGTGCCACCTAAATTGTAAGCGTTAATATTTTGTTAAAATTCGCGTTAAATTTTTG
TTAAATCAGCTCATTTTTTAACCAATAGGCCGAAATCGGCAAAATCCCTTATAAATCA
AAAGAATAGACCGAGATAGGGTTGAGTGTTGTTCCAGTTTGGAACAAGAGTCCACT
ATTAAAGAACGTGGACTCCAACGTCAAAGGGCGAAAAACCGTCTATCAGGGCGAT
GGCCCACTACGTGAACCATCACCCTAATCAAGTTTTTTGGGGTCGAGGTGCCGTAA
AGCACTAAATCGGAACCCTAAAGGGAGCCCCCGATTTAGAGCTTGACGGGGAAAG
CCGGCGAACGTGGCGAGAAAGGAAGGGAAGAAAGCGAAAGGAGCGGGCGCTAGG
GCGCTGGCAAGTGTAGCGGTCACGCTGCGCGTAACCACCACACCCGCCGCGCTTAA
TGCGCCGCTACAGGGCGCGTCCCATTCGCCATTCAGGCTGCGCAA

### 2. AAV Packaging and Titer Determination

AAV packaging was carried out by the conventional three-plasmid co-transfection technique. The RC plasmid, Helper plasmid, and pAAV-CAG-RFP plasmid were chemically transfected into HEK293 cells. AAV particles were isolated from the HEK293T cells and purified by the iodixanol density gradient method. The AAV titer was determined by the common qPCR technique. All the AAVs were inserted with the CAG universal promoter and used to express the nucleus-targeted red fluorescent protein (RFP). Both the AAV packaging and titer determination were completed by PackGene Biotech in Guangzhou, China. PackGene Biotech provided the reagents and equipment other than RC plasmids.

### 3. Assay on AAV Infection Efficiency in In Vitro Cells

To verify the infection efficiencies of various AAVs in the *in vitro* cultured cells, the tested cells (HEK293 cells and U251 human glioma cells) were seeded into a 24-well cell culture plate at a density of 100,000 cells per well. Subsequently, 10⁹ vg (Viral Genome; equivalent to Genome Copy) of AAVs to be tested were added to each well, and 6 replicate wells were set for each type of AAV. After 4-day incubation, the cells were fixed with 4% paraformaldehyde for 15 min. 0.05% DAPI was then used to stain the nuclei. Finally, all the cells were photographed with a fluorescence microscope and subjected to quantitative analysis.

### 4. Intracerebroventricular Injection in Mice and AAV Infection Efficiency Assay

All the *in vivo* experiments in mice were commissioned to Wuhan Servicebio. Female C57BL6 mice aged 6 to 8 weeks were securely positioned on a stereotaxic frame under deep anesthesia. After the lateral ventricles of mice were well positioned (coordinates: relative to the bregma: anteroposterior -0.11 to -0.35 mm, medial/lateral 0.90 to 0.10 mm, depth 2.2 mm), 5 µl of AAVs were slowly injected via a microsyringe into the unilateral lateral ventricle (8.3 × 10⁹ vg per mouse).

After localized injection, the mice were fed for 4 weeks, and then perfused transcardially with pre-chilled PBS and sampled. For the brain tissues and part of liver tissues, the fresh tissues were quickly frozen, sliced into cryosections, and photographed with a fluorescence microscope. The remaining liver, skeletal muscle, heart, kidney, spleen, pancreas, and dorsal ganglion (DRG) tissues were quickly frozen, and then DNA and RNA of AAVs were extracted therefrom for quantitative assay by qPCR.

### Example 1: Harvest of Modified AAVs

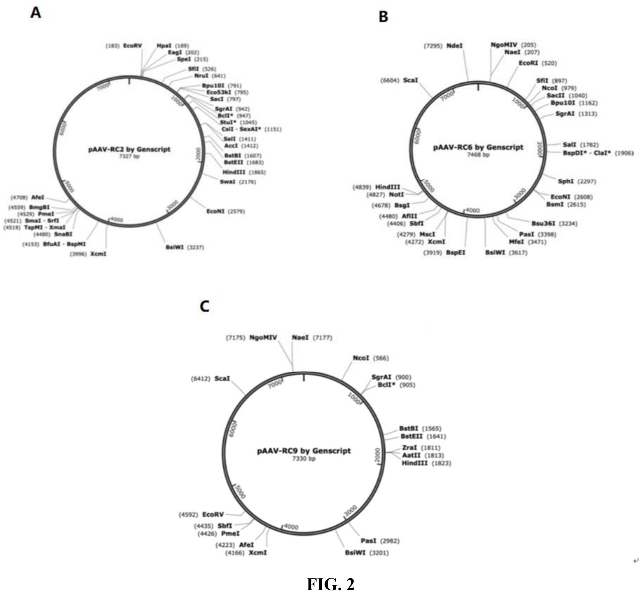

As shown in FIG. 1, this Example was designed to modify the molecules of capsid plasmids RC2, RC6, and RC9 (FIG. 2) by inserting the short peptide GRGDSP at sites 453 and 588 in wild-type AAV2 (also designated as AAV2.1) to harvest modified AAV2.2 and AAV2.3, respectively; inserting the short peptide GRGDSP at sites 454 and 588 in wild-type AAV6 (also designated as AAV6.1) to harvest modified AAV6.2 and AAV6.3, respectively; and inserting the short peptide GRGDSP at sites 453 and 588 in wild-type AAV9 (also designated as AAV9.1) to harvest modified AAV9.2 and AAV9.3, respectively. All of the six modified AAVs could be successfully packaged, and the resulting virus titers were roughly comparable to those of the wild types (Table 1). This Example indicated good compatibility between the short peptide GRGDSP newly inserted at the designed site and the original AAV capsid.

**Table 1:**

| **AAV** | **Original AAV** | **RC Plasmid** | **Targeting Peptide Sequence** | **Amino Acid Before Insertion Site** | **Virus Titer (vg/ml)** |
|---|---|---|---|---|---|
| AAV2.1 (wild-type AAV2) | Wild-type AAV2 | RC2 | - | - | 1.64E+12 |
| AAV2.2 | Wild-type AAV2 | RC2.2 | GRGDSP | G453 | 2.66E+12 |
| AAV2.3 | Wild-type AAV2 | RC2.3 | GRGDSP | R588 | 3.16E+12 |
| AAV6.1 (wild-type AAV6) | Wild-type AAV6 | RC6 | - | - | 2.56E+12 |
| AAV6.2 | Wild-type AAV6 | RC6.2 | GRGDSP | G454 | 2.77E+12 |
| AAV6.3 | Wild-type AAV6 | RC6.3 | GRGDSP | S588 | 2.37E+12 |
| AAV9.1 (wild-type AAV9) | Wild-type AAV9 | RC9 | - | - | 2.16E+13 |
| AAV9.2 | Wild-type AAV9 | RC9.2 | GRGDSP | G455 | 2.51E+12 |
| AAV9.3 | Wild-type AAV9 | RC9.3 | GRGDSP | Q588 | 2.51E+13 |

In the table, "-" denoted no insertion of the targeting peptide.

### Example 2: Enhanced Cell Infection Efficiencies of Modified AAVs

To detect the cell infection efficiencies of the modified AAVs, the *in vitro* cultured tool cells - HEK293 cells were used firstly in this Example. As shown in FIG. 3 to FIG. 5, most of AAVs could infect the HEK293 cells well. Many DAPI-positive HEK293 cells expressed the AAV-mediated red fluorescence concurrently. Compared with wild-type AAV2 (AAV2.1) (FIG. 3), the infection efficiency of AAV2.2 was comparable thereto, while the infection efficiency of AAV2.3 was decreased. Compared with wild-type AAV6 (AAV6.1) (FIG. 4), both the infection efficiencies of AAV6.2 and AAV6.3 were somewhat enhanced. Compared with wild-type AAV9 (AAV9.1) (FIG. 5), both the infection efficiencies of AAV9.2 and AAV9.3 were somewhat enhanced as well. The positive percentages of infection with the AAVs were as shown in FIG. 6 (analytical method of significant difference: conducting a pairwise comparison between modified and wild-type AAVs by ANOVA + Dunnett post hoc, **** *P <* 0.0001).

Furthermore, this Example also evaluated the infection efficiencies of all AAVs in the *in vitro* cultured U251 human glioma cells. As shown in FIG. 7 to FIG. 9, the AAVs exhibited divergent infection efficiencies in the U251 tumor cells. Compared with wild-type AAV2 (AAV2.1) (FIG. 7), the infection efficiency of AAV2.2 was comparable thereto, while the infection efficiency of AAV2.3 was decreased. Compared with wild-type AAV6 (AAV6.1) (FIG. 8), AAV6.2 showed an enhanced infection efficiency and a significant difference, while AAV6.3 showed a decreased infection efficiency. Compared with wild-type AAV9 (AAV9.1) (FIG. 9), AAV9.2 showed a significantly enhanced infection efficiency, while AAV9.3 showed a slightly decreased infection efficiency but was still in the usable range. In comparison, wild-type AAV2 and AAV6 appeared to have greater affinity for U251 tumor cells than wild-type AAV9. The positive percentages of infection with the AAVs were as shown in FIG. 10 (analytical method of significant difference: conducting a pairwise comparison between modified and wild-type AAVs by ANOVA + Dunnett post hoc, **** *P <* 0.0001).

In conclusion, insertion of the short peptide GRGDSP at the site 453/454/455 in AAV2/6/9 could enhance its cell infection efficiency, and AAV9.3 was still in the usable range despite a slight decrease in the infection efficiency.

### Example 3: Higher Targeting Ability of Modified AAVs to Brain

To study the targeting ability of each AAV to brain tissue, in this Example, the AAV (AAV-CAG-RFP) carrying the red fluorescent protein gene was directly injected stereotaxically into the lateral ventricle of an adult mouse brain, and then the expression of AAV in the brain tissue was observed. It would found in previous studies that AAVs injected into the lateral ventricle would "leak" into the systemic blood circulation system in many cases and thus infected the other tissues throughout the body and incurred an off-target risk. Therefore, a major research priority of this Example was to observe whether the expression of the modified AAVs would be reduced in other tissues than the brain tissue. As shown in FIG. 11 and FIG. 12, the AAV-mediated red fluorescence, after injected into the lateral ventricle, was mainly expressed in the brain tissue near the lateral ventricle. The overall expression efficiency of the wild-type AAV2 (AAV2.1) was lower and the expression efficiency of the modified AAV2.2 was slightly improved, but AAV2.3 was less expressed in brain tissue. The fluorescent protein expression in the wild-type AAV6 (AAV6.1) was higher than that in AAV2, while the expression efficiencies of AAV6.2 and AAV6.3 were comparable to that of AAV6. The wild-type AAV9 (AAV9.1) showed the highest overall expression efficiency, while the expression efficiency of the modified AAV9.2 was significantly reduced, which led to a decrease in its *in vivo* application value. The AAV9.3 also showed a slight decrease in the expression efficiency, but it had no significant difference from the wild-type AAV9, and was still in the usable range. The analytical method of significant difference shown in FIG. 12 was as follows: conducting a pairwise comparison between modified and wild-type AAVs by ANOVA + Dunnett post hoc, * *P* < 0.05.

Consistent with prior studies, the histological data from this Example showed that a significant portion of AAV2 and AAV9 injected into the lateral ventricles leaked into the periphery, resulting in high expression of AAV in the liver (FIG. 13 and FIG. 14). The expression of wild-type AAV9 (AAV9.1) in the liver was especially high. The AAV2.2, AAV6.2, and AAV9.2 obtained by inserting GRGDSP at the sites 453/454/455 showed a decreased expression level of AAV in the liver. In another aspect, the expression of AAV2.3 modified at site 588 in the liver was higher than that of the corresponding wild-type AAV2, the expression of AAV6.3 modified at site 588 in the liver was comparable to the expression level of the corresponding wild-type AAV6 in the liver, whereas the expression of AAV9.3 in the liver was lower than that of wild-type AAV9. The analytical method of significant difference shown in FIG. 14 was as follows: conducting a pairwise comparison between modified and wild-type AAVs by ANOVA + Dunnett post hoc. Analytical method of significant difference was as follows: conducting a pairwise comparison between modified and wild-type AAVs by ANOVA + Dunnett post hoc, * *P* < 0.05, *** *P* < 0.001, **** *P* < 0.0001.

In this Example, the distribution of expression of AAVs in various main peripheral tissues was further systematically detected by the qPCR technique (FIG. 15 and FIG. 16). Similar to the histological data described above, this Example showed once again at the DNA and RNA levels that the modified AAVs obtained by inserting GRGDSP at the sites 453/454/455 could effectively reduce "leakage" of AAVs injected into the lateral ventricle, as compared to the wild type. The analytical method of significant difference shown in FIGS. 15 and 16 was as follows: conducting a pairwise comparison between modified and wild-type AAVs by ANOVA + Dunnett post hoc. Analytical method of significant difference was as follows: conducting a pairwise comparison between modified and wild-type AAVs by ANOVA + Dunnett post hoc, * *P <* 0.05, ** *P* < 0.01, *** *P* < 0.001.

In summary, insertion of the short peptide GRGDSP at the site 453 or 454 in AAV2 or AAV6 could enhance the affinity of the corresponding AAV for the brain tissue, effectively reduce the "off-target" infection to the peripheral tissues, and significantly improve the targeting ability to the brain tissue. When the short peptide GRGDSP was inserted at the site 588 in AAV9 (AAV9.3), its expression in the brain tissue was slightly lower than that in wild-type AAV9, but it did not have a significant difference from wild-type AAV9. Since the overall expression efficiency of the wild-type AAV9 in brain tissue was the highest among the wild types, AAV9.3 was still within the usable range. Besides, since AAV9.3 was less expressed in the liver than wild-type AAV9 and could reduce the "leakage" of wild-type AAV9, AAV9.3 could also serve as an ideal vector.

### Example 4: In Vitro Assay on Variations in Abilities of AAV Capsids to Mediate Protein Expression before and after Modification by qPCR (transcriptional level) and Western Blot (protein level)

The above experimental results of Examples 2 and 3 showed that modified AAV6.2 and AAV9.2 had better abilities to mediate the expression of the protein of interest in the *in vitro* cell line (*e.g.* by the qualitative experiment for counting RFP-positive cells) and better hepatic tropism than those of their respective wild types. Therefore, this Example 4 selected AAV6.2 and AAV9.2 as examples for further studies, and conducted relative quantification by qPCR and WB to compare the abilities to infect the target cell and express the protein of interest between AAV6.1 and AAV6.2 and between AAV9.1 and AAV9.2.

### Experimental Method:

**AAV packaging and titer determination:** AAV packaging was carried out by the conventional three-plasmid co-transfection technique. The RC plasmid (the same as in Example 1, namely, RC6, RC6.2, RC9, and RC9.2 in Example 1), Helper plasmid, and pAAV-CAG-EGFP plasmid were chemically transfected into HEK293 cells. AAV particles were isolated from the HEK293T cells and purified by the iodixanol density gradient method. The AAV titer was determined by the common qPCR technique. All the AAVs were inserted with the CAG universal promoter and used to express the enhanced green fluorescent protein (EGFP). Both the AAV packaging and titer determination were completed by Shandong Vigene Biosciences Co., Ltd. Vigene Biosciences provided the regents and equipment other than the RC plasmids.

The titers of packaged viruses were as follows:

| AAV | Virus Titer **(vg/ml)** |
|---|---|
| AAV6-CAG-EGFP | 6.15E+13 |
| AAV6.2-CAG-EGFP | 2.08E+13 |
| AAV9-CAG-EGFP | 5.71E+13 |
| AAV9.2-CAG-EGFP | 5.46E+13 |

### Cell Recovery

HEK293 cells (donated by NovoCodex Biopharmaceuticals Co., Ltd.) and BT474 cells (donated by NovoCodex Biopharmaceuticals Co., Ltd.) were taken from the liquid nitrogen tank and recovered in a culture dish and cultured until the third passage under appropriate conditions.

### Cell Seeding and Virus Infection

1) cells in logarithmic growth phase were collected and seeded in a 24-well plate at a density of 1 × 10⁵ cells per well; and in a 96-well plate at a density of 10,000 cells per well;
2) when the cell confluency reached 70% to 80%, the cells were infected with viruses, and the culture medium was replaced with 1 ml of medium per well at 2 h to 4 h before infection; 4 × 10¹⁰ vg of viruses (N=3) were added to each well of the 6-well plate, and 10¹⁰ vg of viruses (N=3) were added to each well of the 24-well plate.

### RNA Extraction

RNA was extracted from cells using the RNA Kit from Yeasen Biotechnology (Shanghai) Co., Ltd., and immediately subjected to the concentration assay and reverse transcription.

### RNA Reverse Transcription

RNA was reverse transcribed into cDNA using the reverse transcription kit from TransGen Biotech Co., Ltd.

### qPCR

1. Primer design and synthesis: primers were designed by primer 5 and synthesized by GenScript.
2. The primer sequences were as shown below. After acquired, the primers were added with DEPC water according to the instructions, vortexed thoroughly, and preserved at -20°C.
   hActin-F: CATGTACGTTGCTATCCAGGC (SEQ ID NO: 24)
   hActin-R: CTCCTTAATGTCACGCACGAT (SEQ ID NO: 25)
   EGFP-F: GACCACTACCAGCAGAACAC (SEQ ID NO: 26)
   EGFP-R: GAACTCCAGCAGGACCATG (SEQ ID NO: 27)

### qPCR system and conditions:

The experiment was conducted using the qPCR kit from TransGen Biotech Co., Ltd.

### Configuration of system:

| | |
|---|---|
| 2 ×Green qPCR SuperMix | 10 µl |
| Forward primer (10 mM) | 0.4µl |
| Reverse primer (10 mM) | 0.4 µl |
| template cDNA | 1 µl |
| ddH₂O | 8.2 µl |

After the samples were loaded, the plate was sealed with a cover and briefly centrifuged to mix the system uniformly and remove air bubbles. The samples were detected on a real-time quantitative PCR instrument, and the reaction conditions were set as follows: 94°C -- 30 sec; 94°C -- 5 s; 60°C -- 30 s; Melt curve-cycles: 40. The data were analyzed by the delta - delta Ct method.

### Western Blot (WB)

### (1) Preparation of cell protein samples

a. Cell lysate was formulated. 100 mM PMSF was added to the RIPA cell lysate at a volume ratio of RIPA: PMSF=100: 1 (Shanghai Beyotime Biotechnology Co., Ltd.), and stored temporarily on ice. b. Cell samples at the corresponding time point were washed three times with PBS buffer pre-chilled at 4°C. c. 200 µl of RIPA lysate were added to each well of a 6-well plate. The culture plate was gently shaken and placed in an ice box. The ice box was placed on a shaker and reacted for 10 min, so that the cells were fully lysed. d. The cell lysate was gently pipetted up and down with a pipette gun until there were no visible agglomerated cell blocks. The cell lysate was transferred into a 1.5-mL centrifuge tube, and centrifuged at 4°C, 12,000 rpm for 10 min. 150 µl (the remaining for BCA) of supernatant were carefully aspirated into a new pre-labeled 1.5-mL centrifuge tube. 50 µl of 4×SDS protein loading buffer were added. The samples were heated in a metal bath at 100°C for 10 min, and stored at -20°C for a long time.

### (2) Protein concentration assay

a. 20 µl of the above protein sample were aspirated carefully into a 96-well plate. b. 0, 2, 4, 6, 8, 12, 16, and 20 µl of 5 mg/mL BSA protein standards were loaded to a 96-well plate respectively, and PBS buffer was added to a final volume of 20 µl. c. The working solution (BCA reagent: Cu reagent=50: 1, 200 µl/well) (Shanghai Beyotime Biotechnology Co., Ltd.) was added. d. The system was reacted in a 37°C incubator protected from light for 30 min. The absorbance at 562 nm was detected by a microplate reader. Standard curves were plotted and the concentrations of the corresponding protein samples were calculated.

### (3) SDS-PAGE Western blot assay

a. Electrophoresis conditions: constant voltage 70V - 50 min, constant voltage 110V - 70 min, determination of whether the electrophoresis was finished by observing the bromophenol blue at the front of the gel plate. b. Membrane transfer: 1× transfer buffer pre-chilled in a refrigerator was removed to soak the transfer device. The PVDF membrane was activated with anhydrous methanol for 30 s, and then the transfer sandwich was assembled in the direction of black side - metal grid - three-layer filter paper - gel - PVDF membrane - three-layer filter paper - metal grid - white side in a wet state. The cassette was put in the transfer tank in the (black-to-black, red-to-red) orientation, and the transfer buffer was added to the Blotting marker line. The membrane was transferred at 4°C, 200 mA for 2 h. c. After the transfer membrane was blocked, the separation gel was examined to see if there were excessive residual markers. If there were no excessive residues, the PVDF membrane was fully immersed in a plastic box containing 5% skim milk. The plastic box was placed on a shaker running at a low speed, and incubated at room temperature for 1 to 2 h. d. Upon completion of antibody incubation and blocking, the PVDF membrane was washed simply with Ultrapure water. The membrane was cut according to the size of the corresponding protein. The corresponding membrane was placed into the compartment of the antibody incubation box, and the corresponding primary antibody diluted at 1:1000 was added. The antibody incubation box was placed on a shaker running at a low speed, and incubated at 4°C for 16 to 20 h. After incubation of the primary antibody, the primary antibody was recovered, rinsed with 1× TBST, and fast incubated on a shaker at room temperature for 10 min (repeated three times). The secondary antibody (1: 10000) was added. The antibody incubation box was placed on a shaker running at a low speed, and incubated at room temperature for 1 h, and the solution was discarded. The antibody was rinsed with 1× TBST, and fast incubated on a shaker at room temperature for 10 min (repeated three times). e. Developing: the membrane was added with an ECL solution (formulated at a ratio of solution A: solution B=1: 1) and exposed. The image data was preserved and the gray scale was analyzed using image J.

### Experimental results and analysis:

RNAs and proteins were extracted by lysing the cell samples in the 24- and 6-well plates. The mRNA and protein levels of EGFP were detected by means of relative quantification by qPCR and western blot (β-actin as the internal reference) and the results were as shown in FIG. 17 to FIG. 20B. As shown in FIG. 17, the results showed that in the HEK293 cells, all the capsids mediated the EGFP expression, and the EGFP levels were significantly increased as compared to the NC group (blank control group - without infection with AAV). After the short peptide was inserted into the capsids, their infection abilities were significantly improved as compared to the corresponding wild types (AAV6.2 showed a 36.5-fold increase over AAV6, and AAV9.2 showed a 6.44-fold increase over AAV9), and AAV6.2 was significantly better than AAV9.2 (by 20.8 folds).

As shown in FIG. 18A and FIG. 18B, the WB experimental results were consistent with the qPCR results. In the HEK293 cells, the abilities of modified AAVs to mediate the EGFP expression were significantly improved as compared to those of the corresponding wild types, and AAV6.2 was better than AAV9.2.

As shown in FIG. 19, in the BT474 cells, modified AAV6.2 had a strong ability to infect BT474 cells (AAV6.2 showed a 4.4-fold increase over AAV6), while the abilities of AAV9 and AAV9.2 to infect BT474 cells were lower and they did not show a significant difference before and after modification.

As shown in FIG. 20A and FIG. 20B, the WB experimental results were consistent with the qPCR results. The abilities of modified AAV6.2 to mediate the EGFP expression were significantly improved as compared to those of the corresponding wild types, and it was not found that AAV9 and AAV9.2 mediated EGFP to express in BT474 cells.

Therefore, the qPCR and WB experimental results indicated that AAV6.2 had a stronger infection ability as compared to the corresponding wild type, and the ability of AAV6.2 to mediate the EGFP expression was better than that of AAV9.2 (in HEK293 and BT474 cells).

As to the results in this Example, it should be noted that as substantiated in Examples 2 and 3, the modified AAV9.2 could enhance the affinity of the corresponding AAV for the brain tissue, while effectively reducing the "off-target" infection in peripheral tissues, and significantly improving the targeting ability to the brain tissue, so its lower ability to infect human embryonic kidney cell HEK293T and human breast ductal carcinoma cell BT474 cells did not mean its inability to serve as a carrier for targeting the brain tissue.

### Example 5: In Vitro Verification of Relationship between AAV6- and AAV6.2-Mediated Protein Expression Abilities and Infection Time

Since the results in Example 4 showed that the ability of modified AAV6.2 to mediate expression of the protein of interest in the *in vitro* cell line was significantly better than that of the corresponding wild type (*e*.*g*. experiments on relative quantification of the mRNA level and protein level), AAV6.2 was selected in this Example 5 as an example for further research.

This Example was used designed to investigate the ability and advantages of a novel capsid obtained by modification to mediate the expression of the protein of interest. Brain metastases often occurred in, for example, gastric cancer, breast cancer, colon cancer, prostate cancer, and melanoma, so the corresponding cell lines and brain-derived tumor cell lines were selected for experiment. In this experiment, AAV6 and AAV6.2 at the same titer were used to infect cell lines of different sources respectively, and the fluorescence intensity of EGFP was detected by a microplate reader to quantify the abilities of AAVs to mediate the expression of the protein of interest.

### Experimental Method

Different cell lines (as shown in Table 2, all of which were purchased from Wuhan Pricella Biotechnology Co., Ltd.) were used for cell seeding in a 96-well plate, and the number of cells per well was determined by the cell characteristics while seeding. For cells with the doubling time of about 24 h, 5000 to 10000 cells should be seeded; and for cells with the doubling time of about 84 h or longer, 20000 to 30000 cells should be seeded.

After the confluency reached 40% to 50%, viruses (AAV6-CAG-EGFP and AAV6.2-CAG-EGFP prepared in Example 4) were added.

According to the corresponding time points, the medium in the corresponding group was replaced with a virus-free complete medium (n=6), and the culture was terminated at 72 h after infection. The EGFP fluorescence intensity was detected by a microplate reader. The time points of infection were at 0 h, 4 h, 6 h, 8 h, 12 h, 24 h, 36 h, 48 h, 60 h, and 72 h.

### Experimental results and analysis:

**Table 2:**

| Cell Names | Chinese Cell Names | Culture Conditions | AAV6 72h Mean | AAV6.2 72h Mean | AAV6.2/AAV6(72h) |
|---|---|---|---|---|---|
| NCI-H196 | Human small cell lung cancer cells | RPMI-1640 + 10% FBS + 1% P/S | 6.6 | 480.3 | 72.8 |
| SK-N-SH | Human neuroblastoma cells | MEM (containing NEAA) + 10% FBS + 1% P/S | 3.2 | 19.6 | 6.13 |
| T98G | Human glioma cells | MEM (containing NEAA) + 10% FBS + 1% P/S | 6.1 | 47.8 | 7.84 |
| A172 | Human glioblastoma cells | DMEM + 10% FBS + 1% P/S | 5.228 | 10.92 | 2.08 |
| U-87 MG | Human brain astroblastoma cells | MEM (containing NEAA) + 10% FBS + 1% P/S | 20.7 | 358.5 | 17.3 |
| SK-BR-3 | Human breast cancer cells | McCoy's 5A + 10% FBS + 1% P/S | 8.3 | 24.5 | 2.95 |
| PC-3 | Human prostate cancer cells | Ham's F-12K + 10% FBS + 1% P/S | 6.2 | 46 | 7.42 |
| A-375 | Human malignant melanoma cells | DMEM + 10% FBS + 1% P/S | 91 | 1068 | 11.74 |
| HCT116 | Human colon cancer cells | McCoy's 5A + 10% FBS + 1% P/S | 32.5 | 73 | 2.25 |

The experimental results were as shown in above Table 2 and FIGS. 21A to 21I. The experimental results showed that both AAV6 and AAV6.2 could infect small cell lung cancer, neuroblastoma, glioma, glioblastoma, breast cancer, prostate cancer, malignant melanoma, and colon cancer cells, and the abilities of the modified capsids to mediate the expression of protein of interest were significantly improved.

### References

1. Lawler, S E et al. "Genetic strategies for brain tumor therapy." Cancer gene therapy vol. 13,3 (2006): 225-33. doi:10.1038/sj.cgt.7700886
2. Vance, Melisa, Mitchell, Angela, Samulski, Richard. "AAV Biology, Infectivity and Therapeutic Use from Bench to Clinic". Gene Therapy - Principles and Challenges, edited by Doaa Hashad, IntechOpen, 2015. 10.5772/61988.
3. Santiago-Ortiz, Jorge L, and David V Schaffer. "Adeno-associated virus (AAV) vectors in cancer gene therapy." Journal of controlled release: official journal of the Controlled Release Society vol. 240 (2016): 287-301. doi:10.1016/j.jconrel.2016.01.001
4. Biining, Hildegard, and Arun Srivastava. "Capsid Modifications for Targeting and Improving the Efficacy of AAV Vectors." Molecular therapy. Methods & clinical development vol. 12 248-265. 26 Jan. 2019, doi:10.1016/j.omtm.2019.01.008
5. Davidson, B L et al. "Recombinant adeno-associated virus type 2, 4, and 5 vectors: transduction of variant cell types and regions in the mammalian central nervous system." Proceedings of the National Academy of Sciences of the United States of America vol. 97,7 (2000): 3428-32. doi:10.1073/pnas.97.7.3428
6. Dirren, Elisabeth et al. "Intracerebroventricular injection of adeno-associated virus 6 and 9 vectors for cell type-specific transgene expression in the spinal cord." Human gene therapy vol. 25,2 (2014): 109-20. do1:10.1089/hum.2013.021
7. Girod, A et al. "Genetic capsid modifications allow efficient re-targeting of adeno-associated virus type 2." Nature medicine vol. 5,9 (1999): 1052-6. doi:10.1038/12491
8. Shi, Wenfang, and Jeffrey S Bartlett. "RGD inclusion in VP3 provides adeno-associated virus type 2 (AAV2)-based vectors with a heparan sulfate-independent cell entry mechanism." Molecular therapy: the journal of the American Society of Gene Therapy vol. 7,4 (2003): 515-25. doi:10.1016/s1525-0016(03)00042-x
9. Boucas, Jorge et al. "Engineering adeno-associated virus serotype 2-based targeting vectors using a new insertion site-position 453-and single point mutations." The journal of gene medicine vol. 11,12 (2009): 1103-13. doi:10.1002/jgm.1392

## Claims

1. An adeno-associated virus capsid protein, comprising a targeting peptide, wherein the targeting peptide comprises one or more of the following sequences:
(i) an amino acid sequence as set forth in SEQ ID NO: 23;
(ii) an amino acid sequence having at least 80%, 82%, 85%, 87%, 90%, 92%, 95%, 96%, 97%, 98% or 99% identity to the amino acid sequence as set forth in SEQ ID NO: 23, and having or partially having a function of the amino acid sequence as set forth in SEQ ID NO: 23;
(iii) an amino acid sequence obtained by adding, substituting, deleting or inserting one or more amino acid residues in the amino acid sequence as set forth in SEQ ID NO: 23, and having or partially having a function of the amino acid sequence as set forth in SEQ ID NO: 23; or
(iv) an amino acid sequence encoded by a nucleotide sequence that hybridizes with a nucleotide sequence encoding the amino acid sequence as set forth in SEQ ID NO: 23 under stringency conditions, and the amino acid sequence having or partially having a function of the amino acid sequence as set forth in SEQ ID NO: 23, wherein the stringency conditions are moderate stringency conditions, moderate-high stringency conditions, high stringency conditions, or very high stringency conditions.

2. The adeno-associated virus capsid protein according to claim 1, wherein the adeno-associated virus is selected from one or more of AAV1, AAV2, AAV3, AAV4, AAV5, AAV6, AAV7, AAV8, AAV9, rh.10, rh.39, rh.43, and rh.74;
preferably, the adeno-associated virus is selected from one or more of AAV2, AAV6, and AAV9.

3. The adeno-associated virus capsid protein according to claim 1 or 2, wherein
AAV2 comprises AAV2 VP1, AAV2 VP2 and/or AAV2 VP3; and/or
AAV6 comprises AAV6 VP1, AAV6 VP2 and/or AAV6 VP3; and/or
AAV9 comprises AAV9 VP1, AAV9 VP2 and/or AAV9 VP3.

4. The adeno-associated virus capsid protein according to claim 3, wherein
the targeting peptide is inserted at a position corresponding to amino acid 453 and/or a position corresponding to amino acid 588 in AAV2 VP1, AAV2 VP2 and/or AAV2 VP3; and/or
the targeting peptide is inserted at a position corresponding to amino acid 454 and/or a position corresponding to amino acid 588 in AAV6 VP1, AAV6 VP2 and/or AAV6 VP3; and/or
the targeting peptide is inserted at a position corresponding to amino acid 455 and/or a position corresponding to amino acid 588 in AAV9 VP1, AAV9 VP2 and/or AAV9 VP3.

5. The adeno-associated virus capsid protein according to claim 3, wherein the targeting peptide is inserted at a position corresponding to amino acid 453 in AAV2 VP1, AAV2 VP2 and/or AAV2 VP3; and/or
the targeting peptide is inserted at a position corresponding to amino acid 454 in AAV6 VP1, AAV6 VP2 and/or AAV6 VP3; and/or
the targeting peptide is inserted at a position corresponding to amino acid 588 in AAV9 VP1, AAV9 VP2 and/or AAV9 VP3.

6. A polynucleotide encoding the adeno-associated virus capsid protein according to any one of claims 1 to 5.

7. An adeno-associated virus, comprising the adeno-associated virus capsid protein according to any one of claims 1 to 5.

8. The adeno-associated virus according to claim 7, further comprising a transgene; optionally, the transgene being a therapeutic transgene, a prophylactic transgene or a diagnostic transgene.

9. A transgene delivery vector, comprising the adeno-associated virus according to claim 7 or 8.

10. A pharmaceutical composition, comprising:
(a) the adeno-associated virus according to claim 7 or 8, or the transgene delivery vector according to claim 9; and optionally
(b) a pharmaceutically acceptable carrier.

11. Use of the adeno-associated virus according to claim 7 or 8, the transgene delivery vector according to claim 9, or the pharmaceutical composition according to claim 10 in preparation of a reagent for delivering a transgene to a cell.

12. The use according to claim 11, wherein the cell is derived from a subject;
preferably, the subject is a mammalian subject;
more preferably, the subject is a human.

13. The use according to claim 11 or 12, wherein the cell is derived from a brain tissue.

14. The use according to claim 12 or 13, wherein the subject suffers from a brain disease.

15. The use according to any one of claims 11 to 14, wherein the adeno-associated virus, the transgene delivery vector, or the pharmaceutical composition is administered to a subject via one or more of intravenous, intraarterial, subcutaneous, intraperitoneal, intramuscular, intracerebrospinal fluid, intraparenchymal, or intratumoral administration;
preferably, the adeno-associated virus, the transgene delivery vector, or the pharmaceutical composition is administered to a subject via one or more of intravenous, arterial, intracerebrospinal fluid, intraparenchymal, or intratumoral administration.

16. Use of the adeno-associated virus according to claim 7 or 8, the transgene delivery vector according to claim 9, or the pharmaceutical composition according to claim 10 in preparation of a medicament for treating a disease;
preferably, the disease comprises a brain disease.
